# EUROPEAN PATENT SPECIFICATION

(11) **EP 2 057 141 B1**
(45) Date of publication and mention of the grant of the patent: **26.10.2011**
(21) Application number: 07789659.5
(22) Date of filing: 13.08.2007
(51) Int. Cl.: C07D 401/04, C07D 401/14, C07D 403/04, C07D 403/12, C07D 405/14, C07D 413/14, C07D 417/14, C07D 487/04, A61K 31/435, A61K 31/505, A61K 31/55

(54) **PYRIMIDONE COMPOUNDS AS GSK-3 INHIBITORS**
PYRIMIDONVERBINDUNGEN ALS GSK-3-INHIBITOREN
COMPOSÉS DE PYRIMIDONE EN TANT QU'INHIBITEURS DE GSK-3

(30) Priority: 23.08.2006 US 823267 P
(43) Date of publication of application: 13.05.2009
(73) Proprietor: Pfizer Products Inc., Groton, CT 06340 (US)
(72) Inventor: LEFKER, Bruce, Allen, Groton, CT 06340 (US); BRODNEY, Michael, Aaron, Groton, CT 06340 (US); SAKYA, Subas, Man, Groton, CT 06340 (US); HAY, Bruce, Allan, East Lyme, CT 06333 (US); WESSEL, Matthew, David, Groton, CT 06340 (US); CONN, Edward, Lee, Groton, CT 06340 (US)
(74) Representative: Motion, Keith Robert
(86) International application number: PCT/IB2007/002390
(87) International publication number: WO 2008/023239

(56) References cited:
- EP-A- 1 136 482
- EP-A- 1 136 483
- EP-A- 1 136 489
- EP-A- 1 136 491
- EP-A- 1 136 493
- WO-A-03/027080
- WO-A-03/037888
- WO-A-2004/055007
- WO-A-2004/085408
- WO-A-2006/028290
- WO-A-2006/036015

## Description

This application claims priority to U.S. Provisional Application No. 60/823,267, filed August 23, 2006.

### Field of the Invention

The invention relates to pyrimidone derivatives having activity as GSK-3 inhibitors. The invention further relates to pharmaceutical compositions comprising such derivatives, and uses thereof in treating certain disorders.

### Background of Invention

Protein kinases regulate the signaling of extracellular events in the cytoplasm and the nucleus, and take part in practically many events relating to the life and death of cells, including mitosis, differentiation and apoptosis. Inhibitors of certain kinases may have utility in the treatment of diseases when the kinase is not misregulated, but is nonetheless essential for maintenance of a disease. In this case, inhibition of the kinase activity would act either as a cure or palliative for these diseases and as such, inhibitors of protein kinases have long been favorable drug targets.

Glycogen synthase kinase-3 (GSK-3), a proline-directed, serine/threonine kinase for which two isoforms, GSK-3α and GSK-3β, have been identified, phosphorylates the rate-limiting enzyme of glycogen synthesis, glycogen synthase (GS). See, for example, Embi, et al., Eur. J. Biochem., 107, 519-527 (1980). GSK-3α and GSK-3β are highly expressed. See, for example, Woodgett, et al., EMBO, 9, 2431-2438 (1990) and Loy, et al., J. Peptide Res., 54, 85-91 (1999). Besides GS, a number of other GSK-3 substrates have been identified, including metabolic, signaling, and structural proteins. Notable among the signaling proteins regulated by GSK-3 are many transcription factors, including activator protein-1; cyclic AMP response element binding protein (CREB); the nuclear factor (NF) of activated T-cells; heat shock factor-1; beta.-catenin; c-Jun; c-Myc; c-Myb; and NF-.sub.KB. See, for example, C. A. Grimes, et al., Prog. Neurobiol., 65, 391-426 (2001), H. Eldar-Finkelman, Trends in Molecular Medicine, 8, 126-132 (2002), and P. Cohen, et al., Nature, 2, 1-8, (2001).

Targeting GSK-3 activity has significant therapeutic potential in the treatment of conditions including Alzheimer's Disease (A. Castro, et al., Exp. Opin. Ther. Pat., 10, 1519-1527 (2000)): asthma (P. J. Barnes, Ann. Rev. Pharmacol. Toxicol., 42, 81-98 (2002)); cancer (Beals, et al., Science, 275, 1930-1933 (1997), L. Kim, et al., Curr. Opin. Genet. Dev., 10, 508-514 (2000), and Q. Eastman, et al., Curr. Opin. Cell Biol., 11, 233 (1999)); diabetes and its related sequelae, for example, Syndrome X and obesity (S. E. Nikoulina, et al., Diabetes, 51, 2190-2198 (2002), Orena, et al., JBC, 15765-15772 (2000), and Summers, et a/., J. Biol. Chem., 274 17934-17940 (1999)); hair loss (S. E. Millar, et al., Dev. Biol., 207, 133-149 (1999) and E. Fuchs, et al., Dev. Cell, 1, 13-25 (2001)); inflammation (P. Cohen, Eur. J. Biochem., 268, 5001-5010 (2001)); mood disorders, such as depression (A. Adnan, et al., Chem. Rev., 101, 2527-2540 (2001) and R. S. B. Williams, et al., Trends Phamacol. Sci., 21, 61-64 (2000)); neuronal cell death and stroke (D. A. E. Cross, et al., J. Neurochem., 77, 94-102 (2001) and C. Sasaki, et al., Neurol. Res., 23, 588-592 (2001)); bipolar disorder (Klein, et al., PNAS, 93, 8455-8459 (1996)); and in cardio-protection (C. Badorff, et al., J. Clin. Invest., 109, 373-381 (2002), S. Haq, et al., J. Cell Biol., 151, 117-129 (2000), and H. Tong, et al., Circulation Res., 90, 377-379 (2002)).

GSK-3 acts as a negative mediator in multiple cellular pathways, including insulin, IGF-I and Wnt signaling cascades controlling muscle cell proliferation and differentiation (Glass, Int. J. Biochem. and Cell Biol., 37, 1974 (2005); McManus, et al., EMBO J., 24, 1571 (2005); and Rochat, et al., Mol. Biol. Cell., 15, 4544 (2004)). The protein level and activity of GSK-3 are increased in muscle atrophic conditions, such as aging and immobilization of in both rats and human (Cosgrove, et al., Frontiers in Myogenesis, p. 71 (2006); and Funai, et al. Am. J. Physiol. Regul. Integr. Comp. Physiol., 290, R1080 (2006); in denervation-induced atrophy, and in Type II diabetic and obese subjects (Frame, et al., Expert Opin. Ther. Targets, 10, 429 (2006)). GSK-3 inhibition by RNA interference or by small molecules stimulates myotube formation and reduces proteolysis in myocyte cell cultures and in animal models (Van der Velden, et al., Am. J. Physiol. Cell. Physiol., 290, C453-(2006); Li, et al., Int. J. Biochem. and Cell Biol., 37, 2207 (2005); Fang, et al., Endocrinology, 146, 3141 (2005); Evenson, et al., Int. J. Biochem. Cell. Biol., 37, 2226 (2005)). Therefore, inhibition of GSK-3 activity has therapeutic potential in the treatment of conditions or dysfunctions arising from, or associated with, decreases in muscle mass and function. Such conditions or dysfunctions comprise, for example, genetic or traumatic neurological muscle conditions in the young (*e.g.*, muscular dystrophies); conditions arising from chronic illnesses (*e.g.*, congestive heart failure, chronic renal failure, cancer, stroke, and the like); acute illnesses resulting from extended periods of bed rest; conditions related to decreased physical activity in elderly patients; and/or conditions in those experiencing acute injury/illness resulting in extended periods of immobilization and/or bed rest (*e.g.*, hip replacement, major surgery, etc.).

EP1136482A1 discloses pyrimidin-4-one derivatives and their use as GSK3 inhibitors.

### Summary of the Invention

This invention relates to GSK-3 inhibitors of Formulae I and II, or the pharmaceutical acceptable salts thereof, 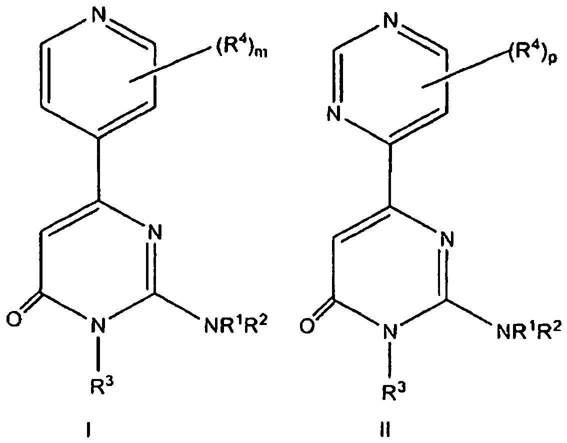
wherein R¹ is hydrogen or a C₁-C₆ alkyl group;
R² is a -(4-15 membered) heterocycloalkyl optionally substituted by one or more substituents selected from the group R⁷;
wherein R³ is hydrogen or C₁-C₆ alkyl;
wherein R⁴ is halogen, C₁-C₆ alkyl, C₁-C₆ haloalkyl,C₁-C₆ alkoxy or C₁-C₆ haloalkoxy;
wherein each R⁷ is independently selected from -OH, halogen, -C₁-C₆ alkyl, -C₂-C₆ alkenyl, -C₂-C₆ alkynyl, -C₁-C₆ alkoxy, -C₂-C₆ alkenoxy, -C₂-C₆ alkynoxy, -C₁-C₆ hydroxyalkyl, -CN, -NO₂, -NR⁸R⁹, -C(=O)N⁸R⁹, -C(=O)R⁸, -C(=O)OR⁸, -S(O)₂NR⁸R⁹, -S(O)ₙR⁸, -NR⁹C(=O)R⁸, -NR⁹SO₂R⁸, -(C_{zero}-C₈ alkylene)-C₆-C₁₅ aryl, -(C_{zero}-C₆ alkylene)-(5-15 membered) heterocycloalkyl, -(C_{zero}-C₆ alkylene)-(5-15 membered) heteroaryl, -(C_{zero}-C₆ alkylene)-C₆-C₁₅ aryloxy and -(C_{zero}-C₆ alkylene)-(5-15 membered) heteroaryloxy, wherein said alkyl, alkenyl, alkynyl, alkoxy, alkenoxy, alkynoxy, hydroxyalkyl, aryl, aryloxy, heteroaryl and heteroaryloxy of R⁷ are each optionally independently substituted with one or more subsitutents selected from halogens, -C₁-C₁₂ alkyl, -C₁-C₄ alkoxy, -NR⁸R⁹, -C(=O)N⁸R⁹. -C(=O)R⁸, -C(=O)OR⁸, -NR⁹C(=O)R⁸. -NR⁹SO₂R⁸,-S(O)₂NR⁶R⁹, -S(O)ₙR⁸ or -OH;
each R⁸ and R⁹ are independently selected from -H, -C₁-C₁₅ alkyl, -C₂-C₁₅ alkenyl,-C₂-C₁₅ alkynyl, -(C_{Zero}-C₄ alkylene)-(C₃-C₁₅ cycloalkyl), -(C_{zero}-C₄ alkylene)-(C₄-C₈ cycloalkenyl), (C_{zero}-C₄ alkylene)-((5-15 membered) heterocycloalkyl), -(C_{zero}-C₄ alkylene)-(C₆-C₁₅ aryl) and -(C_{zero}-C₄ alkylene)-((5-15 membered) heteroaryl), wherein said alkyl, alkenyl, alkynyl, cycloalkyl, cycloalkenyl, heterocycloalkyl, aryl and heteroaryl of R⁸ and R⁹ are each optionally independently substituted with one or more substituents independently selected from -OH. -C₁-C₁₂ alkyl, -C₂-C₁₂ alkenyl, -C₂-C₁₂ alkynyl, -C₁-C₆ alkoxy, -C₂-C₆ alkenoxy, -C₂-C₆ alkynoxy, -C₁-C₆ hydroxyalkyl, halogen, -CN, -NO₂, -CF₃, -NH₂, -NH(C₁-C₆ alkyl), -N(C₁-C₆ alkyl)₂, -C(=O)NH₂, -C(=O)NH(C₁-C₆ alkyl), -C(=O)N(C₁-C₆ alkyl)₂, -SO₂NH₂, -SO₂NH(C₁-C₆ alkyl), -SO₂N(C₁-C₆ alkyl)₂, -C(=O)H, -C(=O)OH and -C(=O)O(C₁-C₆ alkyl);
n is 0, 1 or 2; m is 0, 1, 2, 3 or 4, and p is 0, 1, 2 or 3.

### Detailed description of the invention

The present invention provides compounds of Formula I or II shown above, or a pharmaceutical acceptable salt thereof.

In another embodiment of the present invention for Formulas I or II, R² is a -(5-15 membered) heterocycloalkyl.

In another embodiment, R² is a -(5-15 membered) heterocycloalkyl substituted by R⁷; wherein R⁷ is -C(=O)R⁸, -C(=O)OR⁸ or -S(O)ₙR⁸, and R⁸ is (C_{zero}-C₆ alkylene)-C₆-C₁₅ aryl.

The compounds of Formula I or II may have optical centers and therefore may occur in different enantiomeric and diastereomeric configurations. The present invention includes all enantiomers, diastereomers, and other stereoisomers of such compounds of the Formula I or II, as well as racemic compounds, mixtures, and other mixtures of stereoisomers thereof.

Pharmaceutically acceptable salts of the compounds of Formula I or II include the acid addition and base salts thereof.

Suitable acid addition salts are formed from acids which form non-toxic salts. Examples include, but are not limited to, the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrachloride/chloride, hydrobromide/bromide, hydroiodide/iodide, isethionate, lactate, malate, maleate, malonate, mandelates mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, salicylate, saccharate, stearate, succinate, sulfonate, stannate, tartrate, tosylate, trifluoroacetate, and xinofoate salts.

Suitable base salts are formed from bases which form non-toxic salts. Examples include, but are not limited to, the aluminium, arginine, benzathine, calcium, choline, diethylamine, diolamine, glycine, lysine, magnesium, meglumine, olamine, potassium, sodium, tromethamine, and zinc salts.

Hemisalts of acids and bases may also be formed, for example, hemisulphate and hemicalcium salts.

For a review on these and other suitable salts, see Handbook of Pharmaceutical Salts: Properties, Selection, and Use by Stahl and Wermuth (Wiley-VCH, 2002).

Pharmaceutically acceptable salts of compounds of Formulas I or II may be prepared by:
(i) reacting the compound of Formula I or II with the desired acid or base;
(ii) removing an acid- or base-labile protecting group from a suitable precursor of the compound of Formula I or II or by ring-opening a suitable cyclic precursor, for example, a lactone or lactam, using the desired acid or base; or
(iii) converting one salt of the compound of Formula I or II to another by reaction with an appropriate acid or base or by means of a suitable ion exchange column.

Salt forming reactions are typically carried out in solution. The resulting salt may precipitate or be recovered by evaporation of the solvent. The degree of ionization in the resulting salt may vary from completely ionized to almost non-ionized.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically, such materials do not give distinctive X-ray diffraction patterns and, while exhibiting the properties of a solid, are more formally described as a liquid. Upon heating, a change from solid to liquid properties occurs which is characterized by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterized by a phase change, typically first order ('melting point').

The compounds of the invention may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

A currently accepted classification system for organic hydrates is one that defines isolated site, channel, or metal-ion coordinated hydrates. See, for example, Polymorphism in Pharmaceutical Solids; K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as -COO⁻Na⁺, -COO⁻K⁺, or -SO₃⁻Na⁺) or non-ionic (such as -N⁻N⁺(CH₃)₃) polar head group. For more information, see Crystals and the Polarizing Microscope; N. H. Hartshome and A. Stuart, 4th Edition (Edward Arnold, 1970).

The compounds of the invention include compounds of Formula I or II, as hereinbefore defined, including all polymorphs and crystal habits thereof, prodrugs and isomers thereof (including optical, geometric and tautomeric isomers) as hereinafter defined and isotopically-labeled compounds of Formulas I or II.

Compounds of Formula I or II containing one or more asymmetric carbon atoms can exist as two or more stereoisomers. Where a compound of Formula I or II contains an alkenyl or alkenylene group, geometric *cis*/*trans* (or Z/E) isomers are possible. Where structural isomers are interconvertible *via* a low energy barrier, tautomeric isomerism ('tautomerism') can occur. This can take the form of proton tautomerism in compounds of Formula I or II containing, for example, an imino, keto, or oxime group, or so-called valence tautomerism in compounds that contain an aromatic moiety. It follows that a single compound may exhibit more than one type of isomerism.

Included within the scope of the present invention are all stereoisomers, geometric isomers and tautomeric forms of the compounds of Formula I or II, including compounds exhibiting more than one type of isomerism, and mixtures of one or more thereof. Also included are acid addition or base salts wherein the counterion is optically active, for example, *d*-lactate or *I*-lysine, or racemic, for example, *dl*-tartrate or *dl-*arginine.

*Cis*/*trans* isomers may be separated by conventional techniques well known to those skilled in the art. for example, chromatography and fractional crystallization.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of Formula I or II contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

When any racemate crystallizes, crystals of two different types are possible. The first type is the racemic compound (true racemate) referred to above wherein one homogeneous form of crystal is produced containing both enantiomers in equimolar amounts. The second type is the racemic mixture or conglomerate wherein two forms of crystal are produced in equimolar amounts each comprising a single enantiomer.

While both of the crystal forms present in a racemic mixture have identical physical properties, they may have different physical properties compared to the true racemate. Racemic mixtures may be separated by conventional techniques known to those skilled in the art. See, for example, Stereochemistry of Organic Compounds; E. L. Eliel and S. H. Wilen (Wiley, 1994).

The present invention includes all pharmaceutically acceptable isotopically-labelled compounds of Formula I or II wherein one or more atoms are replaced by atoms having the same atomic number, but an atomic mass or mass number different from the atomic mass or mass number which predominates in nature.

Examples of isotopes suitable for inclusion in the compounds of the invention include, but are not limited to, isotopes of hydrogen, such as ²H and ³H, carbon, such as ¹¹C, ¹³C and ¹⁴C, chlorine, such as ³⁸CI, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulfur, such as ³⁵S.

Certain isotopically-labelled compounds of Formula I or II, for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Isotopically-labeled compounds of Formula I or II can generally be prepared by conventional techniques known to those skilled in the art or by processes analogous to those described in the accompanying Examples and Preparations using an appropriate isotopically-labeled reagent in place of the non-labeled reagent previously employed.

Pharmaceutically acceptable solvates in accordance with the invention include those wherein the solvent of crystallization may be isotopically substituted, *e.g.* D₂O, d₆-acetone, d₆-DMSO.

Specific embodiments of the present invention include the compounds exemplified in the Examples below and their pharmaceutically acceptable salts, complexes, solvates, polymorphs, stereoisomers, metabolites, prodrugs, and other derivatives thereof,

This invention also pertains to a pharmaceutical composition comprising an amount of a compound of Formula I or II effective in treating said disorder or condition.

This invention also pertains to a compound of formula (I) or (II) for treating a disorder selected from Alzheimer's Disease, cancer, diabetes, Syndrome X, obesity, hair loss, inflammation, mood disorders, neuronal cell death, stroke, bipolar disorder, conditions arising from loss of muscle mass and function, decreased sperm motility and cardio-protection.

This invention also provides a compound of formula (I) or (II) for treating a mood disorder or mood episode.

Examples of mood disorders and mood episodes that can be treated according to the present invention include, but are not limited to, major depressive episode of the mild, moderate or severe type, a manic or mixed mood episode, a hypomanic mood episode; a depressive episode with atypical features; a depressive episode with melancholic features; a depressive episode with catatonic features; a mood episode with postpartum onset; post-stroke depression; major depressive disorder; dysthymic disorder; minor depressive disorder; premenstrual dysphoric disorder; post-psychotic depressive disorder of schizophrenia; a major depressive disorder superimposed on a psychotic disorder such as delusional disorder or schizophrenia; a bipolar disorder, for example bipolar I disorder, bipolar II disorder, and cyclothymic disorder.

This invention further provides a compound of formula (I) or (II) for treating a neurodegenerative disorder or condition.

As used herein, and unless otherwise indicated, a "neurodegenerative disorder or condition" refers to a disorder or condition that is caused by the dysfunction and/or death of neurons in the central nervous system. Treatment of these disorders and conditions can be facilitated by administration of an agent which prevents the dysfunction or death of neurons at risk in these disorders or conditions and/or enhances the function of damaged or healthy neurons in such a way as to compensate for the loss of function caused by the dysfunction or death of at-risk neurons. The term "neurotrophic agent" as used herein refers to a substance or agent that has some or all of these properties.

Examples of neurodegenerative disorders and conditions that can be treated according to the present invention include Parkinson's disease; Huntington's disease; dementia, for example Alzheimer's disease, multi-infarct dementia, AIDS-related dementia, and Fronto temperal Dementia; neurodegeneration associated with cerebral trauma; neurodegeneration associated with stroke, neurodegeneration associated with cerebral infarct; hypoglycemia-induced neurodegeneration; neurodegeneration associated with epileptic seizure; neurodegeneration associated with neurotoxin poisoning; and multi-system atrophy.

In one embodiment of the present invention, the neurodegenerative disorder or condition comprises neurodegeneration of striatal medium spiny neurons in a mammal, including a human.

In a further embodiment of the present invention, the neurodegenerative disorder or condition is Huntington's disease.

The term "alkyl", as used herein, unless otherwise indicated, includes saturated monovalent hydrocarbon radicals having straight or branched moieties. Examples of alkyl groups include methyl, ethyl, propyl, isopropyl, and *tert*-butyl.

The term "alkenyl", as used herein, unless otherwise indicated, includes alkyl moieties having at least one carbon-carbon double bond wherein alkyl is as defined above. Examples of alkenyl include ethenyl and propenyl.

The term "alkynyl", as used herein, unless otherwise indicated, includes alkyl moieties having at least one carbon-carbon triple bond wherein alkyl is as defined above. Examples of alkynyl groups include ethynyl and 2-propynyl.

The term "alkoxy", as used herein, unless otherwise indicated, as employed herein alone or as part of another group refers to an alkyl, groups linked to an oxygen atom.

The term "alkylthio" as used herein, unless otherwise indicated, employed herein alone or as part of another group includes any of the above alkyl groups linked through a sulfur atom.

The term "halogen" or "halo" as used herein alone or as part of another group refers to chlorine, bromine, fluorine, or iodine.

The term "haloalkyl" as used herein, unless otherwise indicated, refers to at least one halogen atom linked to an alkyl group. Examples of haloalkyl groups include trifluoromethyl, difluoromethyl, and fluoromethyl groups.

The term "cycloalkyl", as used herein, unless otherwise indicated, includes non-aromatic saturated cyclic alkyl moieties wherein alkyl is as defined above. Examples of cycloalkyl include cyclopropyl, cyclobutyl, cyclopentyl, cyclohexyl, and cycloheptyl.

The term "aryl", as used herein, unless otherwise indicated, includes an organic radical derived from an aromatic hydrocarbon by removal of a hydrogen atom, such as phenyl, naphthyl, indenyl, and fluorenyl. "Aryl" encompasses fused ring groups wherein at least one ring is aromatic.

The terms "heterocyclic", "heterocycloalkyl", and like terms, as used herein, refer to non-aromatic cyclic groups containing one or more heteroatoms, preferably from one to four heteroatoms, each preferably selected from oxygen, sulfur and nitrogen. The heterocyclic groups of this invention can also include ring systems substituted with one or more oxo moieties. Examples of non-aromatic heterocyclic groups are aziridinyl, azetidinyl, pyrrolidinyl, piperidinyl, azepinyl, piperazinyl, 1,2,3,6-tetrahydropyridinyl, oxiranyl, oxetanyl, tetrahydrofuranyl, tetrahydrothienyl, tetrahydropyranyl, tetrahydrothiopyranyl, morpholino, thiomorpholino, thioxanyl, pyrrolinyl, indolinyl, 2H-pyranyl, 4H-pyranyl, dioxanyl, 1,3-dioxolanyl, pyrazolinyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, quinolizinyl, quinuclidinyl, 1,4-dioxaspiro[4.5]decyl, 1,4-dioxaspiro[4.4]nonyl, 1,4-dioxaspiro[4.3]octyl, and 1,4-dioxaspiro[4.2]heptyl.

The term "heteroaryl", as used herein, refers to aromatic groups containing one or more heteroatoms (preferably oxygen, sulfur and nitrogen), preferably from one to four heteroatoms. A multicyclic group containing one or more heteroatoms wherein at least one ring of the group is aromatic is a "heteroaryl group. The heteroaryl groups of this invention can also include ring systems substituted with one or more oxo moieties. Examples of heteroaryl groups are pyridinyl, pyridazinyl, imidazolyl, pyrimidinyl, pyrazolyl, triazolyl, pyrazinyl, quinolyl, isoquinolyl, tetrazolyl, furyl, thienyl, isoxazolyl, thiazolyl, oxazolyl, isothiazolyl, pyrrolyl, indolyl, benzimidazolyl, benzofuranyl, cinnolinyl, indazolyl, indolizinyl, phthalazinyl, triazinyl, isoindolyl, purinyl, oxadiazolyl, thiadiazolyl, furazanyl, benzofurazanyl, benzothiophenyl, benzotriazolyl, benzothiazolyl, benzoxazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl, and azaindolyl.

Unless otherwise indicated, all the foregoing groups derived from hydrocarbons may have up to about 1 to about 20 carbon atoms (*e.g.*, C₁-C₂₀ alkyl, C₂-C₂₀ alkenyl, C₃-C₂₀ cycloalkyl, 3-20 membered heterocycloalkyl; C₆-C₂₀ aryl, 5-20 membered heteroaryl, etc.) or 1 to about 15 carbon atoms (*e.g.*, C₁-C₁₅ alkyl, C₂-C₁₅ alkenyl, C₃-C₁₅ cycloalkyl, 3-15 membered heterocycloalkyl, C₆-C₁₅ aryl, 5-15 membered heteroaryl, etc.), or 1 to about 12 carbon atoms, or 1 to about 8 carbon atoms, or 1 to about 6 carbon atoms.

Generally preferred GSK-3 inhibitors of the instant invention have K*ᵢ* values of less than, or about, 10 µM, more preferably less than or about 0.1 µM.

The term "treating", as in "a method of treating a disorder", refers to reversing, alleviating, or inhibiting the progress of the disorder to which such term applies, or one or more symptoms of the disorder. As used herein, the term also encompasses, depending on the condition of the patient, preventing the disorder, including preventing onset of the disorder or of any symptoms associated therewith, as well as reducing the severity of the disorder or any of its symptoms prior to onset. "Treating" as used herein refers also to preventing a recurrence of a disorder.

The term "mammal", as used herein, refers to any member of the class "Mammalia", including humans, dogs, and cats.

The compound of the invention may be administered either alone or in combination with pharmaceutically acceptable carriers, in either single or multiple doses. Suitable pharmaceutical carriers include inert solid diluents or fillers, sterile aqueous solutions and various organic solvents. The pharmaceutical compositions formed thereby can then be readily administered in a variety of dosage forms such as tablets, powders, lozenges, liquid preparations, syrups, injectable solutions and the like. These pharmaceutical compositions can optionally contain additional ingredients such as flavorings, binders, excipients and the like. Thus, the compound of the invention may be formulated for oral, buccal, intranasal, parenteral (*e.g.*, intravenous, intramuscular or subcutaneous), transdermal (*e.g.*, patch) or rectal administration, or in a form suitable for administration by inhalation or insufflation.

For oral administration, the pharmaceutical compositions may take the form of, for example, tablets or capsules prepared by conventional means with pharmaceutically acceptable excipients such as binding agents (*e.g.*, pregelatinized maize starch, polyvinylpyrrolidone or hydroxypropyl methylcellulose); fillers (*e.g.*, lactose, microcrystalline cellulose or calcium phosphate); lubricants (*e.g.*, magnesium stearate, talc or silica); disintegrants (*e.g.*, potato starch or sodium starch glycolate); or wetting agents (*e.g.*, sodium lauryl sulphate). The tablets may be coated by known methods. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g.*, sorbitol syrup, methyl cellulose or hydrogenated edible fats); emulsifying agents (*e.g.*, lecithin or acacia); non-aqueous vehicles (*e.g.*, almond oil, oily esters or ethyl alcohol); and preservatives (*e.g.*, methyl or propyl p-hydroxybenzoates or sorbic acid).

For buccal administration, the composition may take the form of tablets or lozenges formulated in conventional manner.

The compounds of the invention may be formulated for parenteral administration by injection, including using conventional catheterization techniques or infusion. Formulations for injection may be presented in unit dosage form, *e.g.*, in ampules or in multi-dose containers, with an added preservative. They may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, *e.g.*, sterile, pyrogen-free water, before use.

When a product solution is required, it can be made by dissolving the isolated inclusion complex in water (or other aqueous medium) in an amount sufficient to generate a solution of the required strength for oral or parenteral administration to patients. The compounds may be formulated for fast dispersing dosage forms, which are designed to release the active ingredient in the oral cavity. These have often been formulated using rapidly soluble gelatin-based matrices. These dosage forms are well known and can be used to deliver a wide range of drugs. Most fast dispersing dosage forms utilize gelatin as a carrier or structure-forming agent. Typically, gelatin is used to give sufficient strength to the dosage form to prevent breakage during removal from packaging, but once placed in the mouth, the gelatin allows immediate dissolution of the dosage form. Alternatively, various starches are used to the same effect.

The compounds of the invention may also be formulated in rectal compositions such as suppositories or retention enemas, *e.g.*, containing conventional suppository bases such as cocoa butter or other glycerides.

For intranasal administration or administration by inhalation, the compound of the invention is conveniently delivered in the form of a solution or suspension from a pump spray container that is squeezed or pumped by the patient or as an aerosol spray presentation from a pressurized container or a nebulizer, with the use of a suitable propellant, *e.g.*, dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount. The pressurized container or nebulizer may contain a solution or suspension of the active compound. Capsules and cartridges (made e.g., from gelatin) for use in an inhaler or insufflator may be formulated containing a powder mix of a compound of the invention and a suitable powder base such as lactose or starch.

Aerosol formulations for treatment of the conditions referred to above (e.g. migraine) in the average adult human are preferably arranged so that each metered dose of aerosol contains about 20 mg to about 1,000 mg of the compound of the invention. The overall daily dose with an aerosol will be within the range of about 100 mg to about 10 mg. Administration may be several times daily, e.g. 2, 3, 4 or 8 times, giving for example, 1, 2 or 3 doses each time.

A proposed daily dose of the compound of the invention for oral, parenteral, rectal, or buccal administration to the average adult human may be from about 0.01 mg to about 2,000 mg, preferably from about 0.1 mg to about 200 mg of the active ingredient of Formula I or II per unit dose which could be administered, for example, 1 to 4 times per day.

A proposed daily dose of the compound of the invention for oral, parenteral, rectal or buccal administration to the average adult human may be from about 0.01 mg to about 2,000 mg, preferably from about 0.1 mg to about 200 mg of the active ingredient of Formula I or II per unit dose which could be administered, for example, 1 to 4 times per day.

### Assay protocol for GSK-3β (glycogen synthase kinase) whole cell activity in an inducible cell line

Human recombinant GSK-3β and human recombinant Tau were expressed in a CHO Tet-Off cell line. GSK-3β activity was measured using an immunoassay that detects specific phosphorylation of tau at serine 202 and threonine 205 using cellular lysates of the induced cell line. Cells were grown in Minimum Essential Medium Alpha (Invitrogen) supplemented with 10% tetracycline approved FBS (BD Biosciences Clontech) and 400 pg/ml doxycycline (Sigma). Expression of tau and GSK-3β was induced by growth in medium without doxycycline for 72 hours. The cells were incubated with test agent for 90 minutes and then the medium was removed and the cells lysed with a buffer containing 250mM NaCl, 50mM Tris pH 7.5, 5mM EDTA, 0.1% NP40, 5mM DTT, 1mM sodium orthovanadate, 1uM okadaic acid, and 1X Protease Inhibitor (Roche - Complete tablet). Cell lysates were used in a sandwich immunoassay containing 16ng/well of biotinylated antibody HT7 (Pierce), 20 ng/well of ruthenylated antibody AT8, 10 ug/well of streptavidin magnetic beads M-280 (Bloveris) in a buffer containing 0.5% BSA (Roche), 0.5% Tween 20 (Sigma) in PBS (Sigma). Readout of assay signal was performed on an M-8 Analyzer (Bioveris) after overnight incubation at 4°C with shaking.

### Assay protocol for GSK-3β (glycogen synthase kinase) in a cell free enzyme assay

Recombinant human GSK3β was expressed in SF9/Baculo virus cells. His-tag protein was purified by affinity chromatography to a Ni-NTA Superflow column. Enzyme activity was assayed as the incorporation of [33P] from the gamma phosphate of [33P]ATP (PerkinElmer) into biotinylated peptide substrate bio-LC-S-R-H-S-S-P-H-Q-pS-E-D-E-E-E-OH (Anaspec). Reactions were carried out in a buffer containing 8 mM MOPS (pH 7.0), 10 mM Magnesium Acetate, 0.2 mM EDTA, 1mM DTT and 2 uM cold ATP. The 33P-ATP was added for 0.025uCi/well (120 uL) and the final concentration of substrate was 1.0 uM. Enzyme was preincubated with test agent for 30 minutes at room temperature followed by initiation of the reaction by the addition of substrate mix. Incubations were carried out at RT for 60 min. Reactions were stopped by addition of 0.66 volume of buffer containing: 12.5mM EDTA, 0.25%Triton-X 100, 125uM ATP, and 6.2 mg/ml streptavidin coated SPA beads (Amersham) in PBS without Ca or Mg. Radioactivity associated with the beads was quantified by scintillation counting of CPM in a Trilux counter (PerkinElmer).

The Schemes below depict various methods of preparing the compounds of the present invention. It should be noted that various substitutents illustrated in the schemes (e.g, P, Cap, X1, etc.) are for illustrated purposes only and may be independent of those recited above and in the claims.

The following reaction Schemes are intended to provide an exemplary description of the methodologies employed in the preparation of the Examples. However, it is noted that the compounds prepared according to these Schemes may be modified further to provide new Examples within the scope of this invention. For example, an ester functionality may be reacted further using procedures well known to those skilled in the art to give another ester, an amide, carbinol, or ketone.

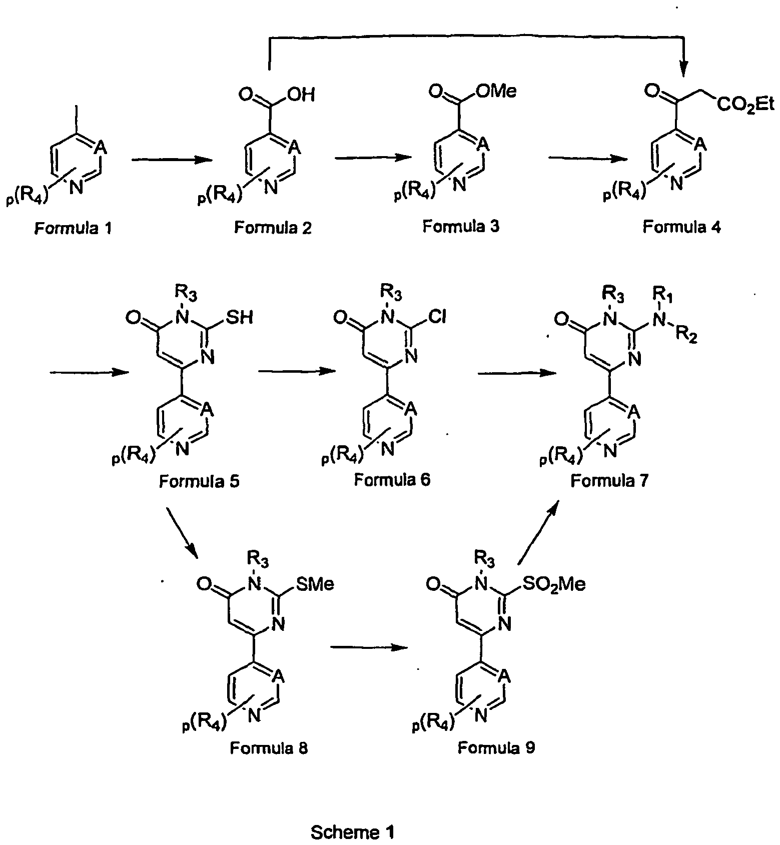

According to Scheme 1, intermediate compounds in Scheme 1, wherein A is a carbon or nitrogen, R¹, R², R³, and R⁴ are as described above may be prepared from compounds of Formula 1 and Formula 4, which may be commercially available, or prepared by methods known to those skilled in the art, such as oxidation with selenium dioxide in a solvent such as pyridine.

As shown in Scheme 1, compounds of Formula 3 may be prepared by esterification of compounds of Formula 2 with an acid, such as sulfuric or hydrochloric acid, in a solvent such as methanol (MeOH), ethanol (EtOH), or isopropanol. The preferred solvent is ethanol, with sulfuric acid as the acid, at a temperature between 0°C and 67°C, preferably 20°C to 67°C.

As shown in Scheme 1, compounds of Formula 4 may be prepared by condensation of ethyl acetate (EtOAc) using a base such as sodium hydride, potassium *tert*-butoxide or metallated hexamethyldisilazine in a polar solvent such as tetrahydrofuran (THF), dimethylformamide (DMF), or EtOAc. The preferred base is potassium *tert*-butoxide, and the preferred solvent is EtOAc/THF at a temperature between 0°C and 67°C, preferably 20°C to 67°C.

Alternatively, compounds of Formula 4 may be prepared by treating compounds of Formula 2 with N,N-carbonyldiimidazole (CDI) in a solvent such as THF to form a reactive intermediate, which can be alkylated with the magnesium salt of ethyl malonate, then heated to afford the decarboxylated product.

As shown in Scheme 1, compounds of Formula 5 may be prepared by condensation of 1-methyl-2-thiourea in the presence of a base such as sodium hydride, potassium *tert-*butoxide or DBU in a solvent such as MeOH, or EtOH. The preferred solvent is ethanol with DBU as the preferred base at a temperature between 0°C and 80°C, preferably 60°C to 80°C.

As shown in Scheme 1, compounds of Formula 6 may be prepared by chlorination of compounds of Formula 5 using a chlorinating agent such as phosphorous oxychloride or phosphorous pentachloride in a solvent such as DMF or DCE. The preferred solvent is DMF with phosphorous oxychloride as the preferred chlorinating agent at a temperature between 0°C and 110°C, preferably 40°C to 80°C.

As shown in Scheme 1, compounds of Formula 7 may be prepared by nucleophilic amine displacement with an amine of Formula 6 or Formula 9 in the presence of a base such as triethylamine (TEA), diisopropylethylamine (DIPEA), or DBU in a solvent such as DMF, DMSO or NMP. The preferred solvent is DMF, with DBU as the preferred base, at a temperature between 0°C and 110°C, preferably 40°C to 80°C.

As shown in Scheme 1, compounds of Formula 8 may be prepared by displacement of methyl iodide with a compound of Formula 5 in the presence of a base such sodium hydroxide, sodium hydride, potassium-*tert*-butoxide or DBU in a solvent such as THF, water, MeOH, or acetonitrile. The preferred solvent is a mixture of water and THF with sodium hydroxide as the preferred base at a temperature between 0°C and 80°C, preferably 0°C to 40°C.

As shown in Scheme 1, compounds of Formula 9 may be prepared by oxidation of the sulfide in the presence of mCPBA or hydrogen peroxide, in a solvent such as THF or dichloromethane. The preferred solvent is dichloromethane with mCPBA at a temperature between 0°C and 80°C, preferably 0°C to 40°C.

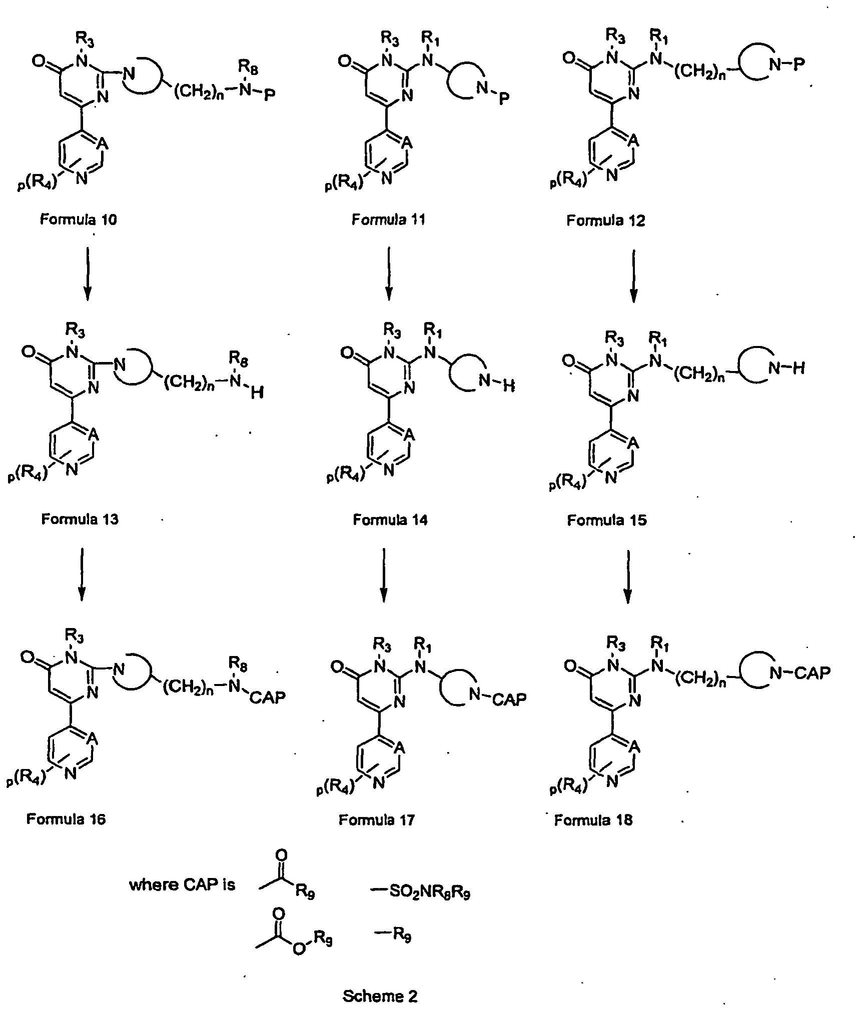

The compounds of Formulae 10, 11, and 12 refer to compounds of Formula 7, as prepared in Scheme 1, where the -NR₁R₂ group of Formula 7 contains an amine group which is protected with a protecting group (e.g., compounds of Formula 10, 11 and 12 where P represents a protecting group such as Boc, Fmoc or CBZ). According to Scheme 2, compounds of Formulae 10, 11, and 12 can be deprotected and then capped to give compounds of Formula 16, 17 or 18. The use of protection/deprotection methods is known to those skilled in the art. See T.W. Greene; Protective Groups in Organic Synthesis; John Wiley & Sons, New York, 1991.

According to Scheme 2, deprotection of compounds of Formulae 10, 11, and 12 is carried out by known methods to afford compounds of Formula 13, 14 and 15. The preferred protecting group is BOC, which can be removed by known methods, preferably trifluoroacetic acid in DCE at a temperature of-78°C and 67°C preferably 0 to 50°.

According to Scheme 2, desired compounds of Formula 16, 17, and 18, wherein CAP refers to an amide group with side chain R₉, may be prepared by acylation of compounds of Formula 13, 14 and 15 with acid chlorides in the presence of an amine base such as TEA, DIPEA, or pyridine in a solvent such as DMSO, DMF, THF, DCE, or acetonitrile. The preferred solvent is DMSO with TEA as the preferred base at a temperature between 20°C and 120°C preferably between 20°C and 60°C.

Alternatively, compounds of Formula 16, 17, and 18, wherein the CAP group is an amide group R₉ as a side chain, may be prepared by treatment of compounds of Formulae 13, 14 and 15 with the carboxylic acid using a suitable coupling reagent such as DCC, or HATU and a base such as TEA, DIPEA, potassium carbonate, or sodium carbonate. The preferred base is DIPEA in a suitable inert solvent such as DMF, THF, methylene chloride, or dioxane. The preferred coupling agent is HATU. The preferred solvent is DMF at a temperature between -40°C and 40°C, preferably 20 to 40°C.

According to reaction Scheme 2, desired compounds of Formulae 16, 17, and 18, wherein CAP group is a carbamate with R₉ as a side chain, may be prepared by reacting compounds of Formula 13, 14 and 15 with the chloroformate in the presence of an amine base such as TEA, DIPEA, or pyridine in a solvent such as DMSO, DMF, THF, DCE, or acetonitrile. The preferred solvent is DCE, with TEA as the preferred amine base, at a temperature between 0°C and 120°C preferably between 0°C and 30°C.

According to reaction Scheme 2, desired compounds of Formula 16, 17, and 18, wherein the CAP is a sulfonamide group with side chain NR₈R₉, may be prepared from compounds of Formulae 13, 14 and 15 with the sulfonychloride in the presence of a base such as TEA, DIPEA, or pyridine in a solvent such as DMSO, DMF, THF, DCE, or acetonitrile. The preferred solvent is DCE, with TEA as the preferred amine base, at a temperature between 0°C and 120°C preferably between 0°C and 30°C.

According to Scheme 2, compounds of Formulae 16, 17, and 18, wherein the CAP is described as R₉, may be prepared by reductive amination of compounds of Formulae 13, 14 and 15 by treatment with an aldehyde or ketone, in the presence of a reducing agent such as sodium borohydride, sodium triacetoxyborohydride, or sodium cyanoborohydride, and optional additives such as acetic acid or sodium acetate. The preferred reducing agent is sodium cyanoborohydride in a solvent such as EtOH, THF, methylene chloride, dioxan, or toluene. The preferred solvent is EtOH at a temperature of -78°C and 67°C, preferably 0 to 50°C.

### Preparation 1: 2-Chloro-3-methyl-6-pyridin-4-yl-3H-pyrimidin-4-one

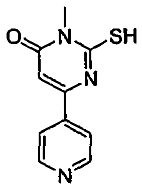

**Step A: 2-Mercapto-3-methyl-6-pyridin-4-yl-3H-pyrimidin-4-one:** A mixture of ethyl isonicotinoylacetate (Acros) (40.6 g, 210 mmol), 1-methyl-2-thiourea (56.8 g, 630 mmol), DBU (31.4 ml, 31.9 g, 210 mmol) and EtOH (400 ml) was heated at reflux for 4 hr. After cooling in an ice-water bath, a solution of methanesulfonic acid (13.6 ml, 20.2 g, 210 mmol) in water (70 ml) was added slowly and the thick precipitate collected by filtration and washed with water. The solid was air-dried overnight to give the title compound (32.6g). Crystals from the mother liquors were collected, washed and dried as above to give more title compound (1.45g). Total yield = 34.03 g (74%) of off-white solid. ¹H-NMR(DMSO): δ ppm 12.88 (s, 1 H), 8.69 - 8.72 (m, 2 H), 7.68 - 7.71 (m, 2 H), 6.37 (s, 1 H), 3.55 (s, 3 H).

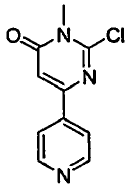

### Step B: 2-Chloro-3-methyl-6-Dyridin-4-yl-3H-pyrimidin-4-one:

Freshly distilled POCl₃ (21.8 ml, 35.8 g, 0.23 mol) was added to DMF (245 ml) with stirring under a nitrogen atmosphere and the mixture stirred for 20 min. The product of Preparation 1, Step A (33.2 g, 0.15 mol), was added portionwise and the resulting mixture stirred at room temperature for 5 min., then heated at 70°C for 4 hr. After cooling (4°C) overnight, the mixture was sealed under nitrogen and EtOAc (865 ml) was added with stirring. After stirring for 30 min., the precipitate was collected, washed with EtOAc and dried. The solid was dissolved in water (550 ml) and the pH adjusted to 10 with 15% aqueous sodium hydroxide. The precipitate was collected and washed with water. The solid was dried at the pump and then in a vacuum oven over phosphorus pentoxide at 45-50°C for 4 days to give a crude product (27.4 g). This solid was recrystallized (hot filtration) from EtOAc (final volume approx 170 ml) to give the title compound (21.0 g) as a light-beige solid, m.p. = 147.8 -148°C. Evaporation of the mother liquor afforded more product (5.60 g). Total yield (26.6 g, 79%). ¹H-NMR(DMSO): δ ppm 8.65 - 8.72 (m, 18 H), 7.89 - 7.96 (m, 21 H), 7.25 (s, 1 H), 3.56 (s, 3 H).

### Preparation 2: 2-Chloro-1-methyl-1H-[4,4']bipyrimidinyl-6-one

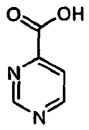

**Step A: Preparation of Pyrimidine-4-carboxylic acid:** To a solution of 4-methyl pyrimidine (Aldrich) (10 g, 0.10 mmol) in pyridine (100 ml) was added SeO₂ (17.8 g, 0.16 mmol). The mixture was heated to 55°C for 2 hr., then 85°C for 3.5 hr. The reaction was allowed to cool to RT and stirred for 36 hr. The solids were filtered through diatomaceous earth. The solvent was evaporated and the residue diluted in 100 ml MeOH. The precipitate was collected to give the title compound as a brown solid (9.7 g, 78%). ¹H-NMR(DMSO-*d*₆): δ ppm 13.4-14.0(broad, 1H), 9.34 (s, 1H), 9.04 (d, *J*=4.98 Hz, 1H) and 8.02((d, *J*=4.98 Hz, 1H). Mass: (M+1) 125 calculated for C₅H₄N₂O₂.

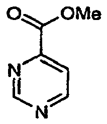

**Step B: Preparation of Pyrimidine-4-carboxylic acid methyl ester:** A solution of the product of Preparation 2, Step A (6.17 g, 49.7 mmol), in MeOH (60 ml) was added to sulfuric acid (0.3 ml) and heated to refluxed for 16 hr. Excess solvent was removed under vacuum to obtain a residue, which was dissolved in 10% MeOH/CHCl₃ (100 ml) and adsorbed onto silica gel. The crude material was purified by column chromatography over silica gel eluting with CHCl₃ then 10% MeOH/CHCl₃ to obtain the title compound as a yellow solid (5.8 g, 85%). ¹H-NMR(DMSO): δ 9.4(s, 1H), 9.0(d, J=4.9 Hz, 1H), 8.0(d, J=4.9 Hz, 1H) and 4.0(s, 3H). Mass: (M+H) 140 calculated for C₆H₇N₂O₂.

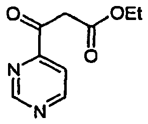

**Step C: 3-Oxo-3-pyrimidin-4-yl-propionic acid ethyl ester:** To a solution of the product of Preparation 2, Step B (5.8 g, 42 mmol), in EtOAc (180 ml) was added 1M potassium *tert-*butoxide in THF (85 ml, 85 mmol) in four portions, with mechanical stirring. The reaction was refluxed for 40 hr. Water (200 ml) was added and layers separated. The aqueous was washed with EtOAc (2 X 100 ml). The aqueous was acidified with conc. HCl to pH 2-3 then extracted with CHCl₃ (3 X 100 ml). The organics were combined, washed with brine, dried over sodium sulfate, and concentrated to give the title compound as an orange solid (7.07g 86%). (Mixture of keto and enol form) Keto: ¹H-NMR(CDCl3) δ ppm 12.22 (s, 1 H), 9.23(s, 1H), 8.89 (d, *J*=4.98 Hz, 1 H), 7.83 - 7.85 (m, 1 H), 7.26(s, 1H), 6.46(s, 1H), 4.30 (q, *J*=7.05 Hz, 2 H), 1.34 (t, *J*=7.26 Hz, 3 H).

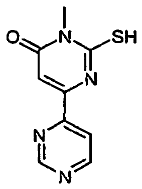

**Step D: 2-Mercapto-1-methyl-1H-[4,4']bipyrimidinyl-6-one:** To a solution of the product of Preparation 2, Step C (8 g, 41.2371 mmol), in EtOH (70 ml) were added N-methyl thiourea (7.43 g, 82.47 mmol) and DBU (6.27 g, 41.29 mmol) at RT. The mixture was heated to 70°C and stirred for 4 hr. The mixture was concentrated and the crude residue purified by column chromatography over 60-120 mesh silica gel column using 40% EtOAc in DCM as eluting solvent to give the title compound as yellow crystalline solid (6 g, 66%). ¹H-NMR (DMSO): δ ppm10.5-10.8(broad, 1H), 9.4(s, 1H), 9.0(d, *J*=5 Hz, 1 H), 7.8(d, *J*=5 Hz, 1 H), 6.6(s, 1H) and 3.75(s, 3H). Mass: (M+H) 221 calculated for C₉H₈N₄OS.

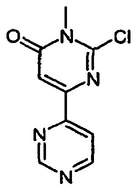

**Step E: 2-Chloro-1-methyl-1H-[4,4']bipyrimidinyl-6-one:** To DMF (50 ml) cooled in an ice bath was added POCl₃ (11 ml). The mixture was stirred for 30 min., and then the product of Preparation 2, Step C (5 g, 22.7 mmol) was added in one portion. The reaction mixture was heated in a 50°C oil bath and stirred for 1hr. The reaction mixture was cooled to RT and poured onto ice water (-200 ml) and stirred until mixture warmed to RT. The solution was neutralized to pH - 7 with solid sodium bicarbonate. The formed solid was collected to yield (3.42g) of brown solid. The crude residue was redissolved in EtOAc and washed with 1N NaOH (2x100ml) then brine, dried over sodium sulfate, and concentrated to give the title compound as a tan solid (1.44g). The neutralized aqueous was extracted with EtOAc (3X). The organics were washed with 1N NaOH (100 ml) then brine, dried over sodium sulfate, and concentrated to give the title compound as a tan solid (0.933 g). Total yield was 2.37 g, 47%. ¹H-NMR(DMSO): δ ppm 9.32(s, 1H), 9.01 (d, *J*=5 Hz, 1 H), 8.14 (d, *J*=5 Hz, 1 H), 7.30 (s, 1H) and 3.57 (s, 3H).

### Preparation 3: 3-Methyl-2-(methylsulfonyl)-6-pyridin-4-yl-3H-pyrimidin-4-one

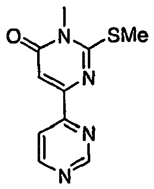

**Step A:_3-Methyl-2-(methylthiol)-6-pyridin-4-yl-3H-Dyrimidin-4-one:** To a suspension of the product of Preparation 2, Step D (250 mg, 1.1 mmol), in THF (3 ml) was added Mel (0.08 ml, 1.2 mmol) then 1N NaOH (1.4 ml, 1.4 mmol). The suspension was stirred for 30 min. The mixture was diluted with water then extracted with CHCl₃ (3X). The organics were combined, washed with brine, dried over sodium sulfate, and concentrated to give the title compound as a yellow crystalline solid (277 mg, 100%). ¹H-NMR (DMSO-*d*₆): δ ppm 9.30 (s, 1 H), 9.02 (d, *J*=5 Hz, 1 H), 8.31 (d, *J*=5 Hz, 1 H), 7.09 (s, 1 H), 3.45 (s, 3 H), 2.70 (s, 3 H).

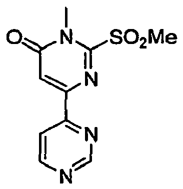

**Step B: 3-Methyl-2-(methylsulfonyl)-6-pyridin-4-yl-3H-pyrimidin-4-one:** To a solution of the product of Preparation 3, Step A (550 mg, 2.3 mmol), in THF (55 ml) was added mCPBA (1.0 g, 5.8 mmol) and stirred for 16 hr. The solvent was removed and the residue redissolved in CHCl₃ and adsorbed onto silica gel. The residue was purified by column chromatography over 60-120 mesh silica gel column eluting with 50% EtOAc in hexane to give a white solid (625 mg, 54%). ¹H-NMR(DMSO-*d*₆): δ ppm 9.35 (s, 1 H), 9.06 (d, J=5 Hz, 1 H), 8.31 (d, *J*=5 Hz, 1 H), 7.09 (s, 1 H), 3.76 (s, 3 H), 3.71 (s, 3 H).

### Preparation 4: 2-Chloro-6-(3-fluoropyridin-4-yl)-3-methylpyrimidin-4(3H)-one

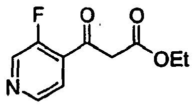

**Step A: Ethyl 3-(3-fluoropyridin-4-yl)-3-oxopropanoate:** To a suspension of 3-fluoroisonicotinic acid (3 g, 21.3 mmol) in THF (50 ml) was added CDI (3.6 g, 22.4 mmol). The mixture was heated at 50°C for about 16 to 18 hr. In a separate flask, potassium ethyl malonate (4.7g, 27.7 mmol) and magnesium chloride (3.2 g, 33.2 mmol) was suspended in THF and stirred at 35°C for 1 hr. To this mixture was added the anhydride mixture from the previous step. The combined mixture was heated at reflux for 1 hr. and then at 50°C for 16-18 hr. The mixture was cooled to RT and acidified with aq. HCl (1 N) to pH -5. After addition of water (5 ml), the organic layer was separated. The aqueous layer was further extracted with EtOAc (3 X 30 ml) and the combined organic layer was dried (sodium sulfate), and evaporated to a crude oil. Addition of MeOH precipitated the title product as a white solid, 3.9 g (88.4%): ¹H-NMR(DMSO-d6) δ 8.52 (d, 1H), 8.41(q, 1H), 7.63 (m, 1H), 5.13 (s, 1H), 4.00 (q, 2H), 3.32 (s, 2H), 1.16 (t, 3H); LCMS 212.2 (M+H).

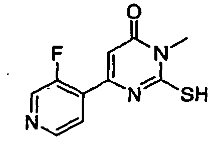

**Step B: 6-(3-Fluoropyridin-4-yl)-2-mercapto-3-methylpyrimidin-4(3H)-one:** To a suspension of the product of Preparation 4, Step A (3.9 g, 18.4 mmol), in toluene (40 ml) was added N-methylthiourea (5.6 g, 62.6 mmol) and DBU (3.0 ml, 20.3 mmol) and the mixture heated at 100°C for 48 hr. 30 ml of EtOH was added and the reaction heated at 100°C for ∼18 hr. The reaction was cooled to RT and water (18 ml) and methanesulfonic acid (2 ml) was added and stirred for 1 hr. The aqueous layer was concentrated to a small volume and the formed precipitate was collected to provide 2.4 g (55%) of yellow solid. ¹H-NMR (MeOH-d₄) δ 8.65 (d, 1H), 8.55(d, 1H), 7.63 (q, 1H), 6.17 (s, 1H), 3.69 (s, 3H); LCMS 238.2 (M+H).

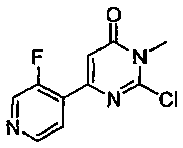

**Step C: Preparation of 2-Chloro-6-(3-fluoropyridin-4-yl)-3-methylpyrimidin-4(3H)-one:** Phosphorous oxychloride (0.41 ml, 4.43 mmol) was added to DMF (5 ml) and stirred at RT for 30 min. To this mixture was added the product of Preparation 4, Step B (700mg, 2.95 mmol), portionwise and the mixture was heated at 62°C for 2 hr. After cooling and concentration, water was added slowly. The mixture was extracted with dichloromethane (5 X 30 ml), dried (sodium sulfate), and concentrated to give the title product (365 mg, 52%) as yellow solid. ¹H-NMR(CDCl₃) δ 8.68 (d, 1H), 8.57(q, 1H), 7.99 (q, 1H), 7.12 (s, 1H), 3.72 (s, 3H); LCMS 240.3 (M+H).

### General Procedure for Examples 1-35

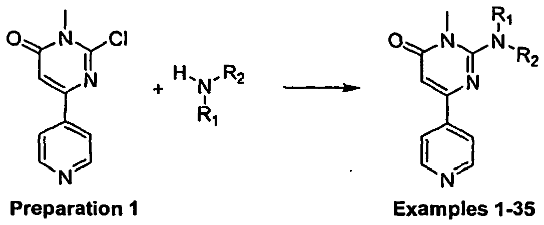

To the amine (80 µmol) was added a solution of the product of Preparation 1 (15.5 mg, 70 µmol) and TEA (16 mg, 160 µmol) in DMF (400 µl). The mixture was sealed and heated to 80°C for 12 hr. with shaking. The mixture was diluted with EtOAc (2 ml) and water (2 ml), then shaken. The organic layer was transferred to tarred vials and aqueous layer extracted with EtOAc (2 ml). The organic layer was transferred to tarred vials. The organics were evaporated and vials weighed for crude mass. The residues were dissolved in DMSO (930 µl) and heated to 60C for 1 hr. Products were purified by Prep HPLC.

### EXAMPLES

The following Examples 1 to 35 were prepared according to the General Procedure described above.

| **Ex.** | **Structure** | **Name** | **Observed Mass (MH+)** | **Enzyme IC₅₀ (nM)** |
|---|---|---|---|---|
| 1 | 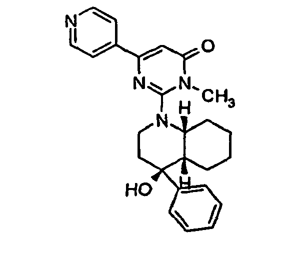 | 2-[(4S,4aS,8aR)-4-Hydroxy-4-phenyloctahydroqui nolin-1(2H)-yl]-3-methyl-6-pyridin-4-ylpyrimidin-4(3H)-one | 417.1 | 0.7 |
| 2 | 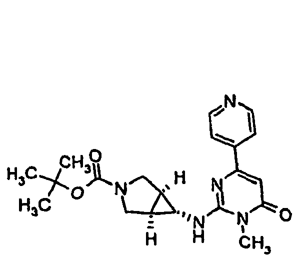 | *tert*-butyl (1R,5S,6s)-6-[(1-Methyl-6-oxo-4-pyridin-4-yl-1,6-dihydropyrimidin-2-yl)amino]-3-azabicyclo[3.1.0]he xane-3-carboxylate | 384.1 | 2.2 |
| 3 | 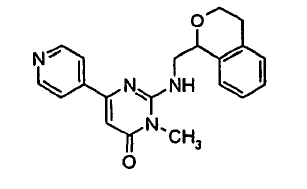 | 2-[(3,4-dihydro-1H-Isochromen-1-ylmethyl)amino]-3-methyl-6-pyridin-4-ylpyrimidin-4(3H)-one | 349.1 | 23 |
| 4 | 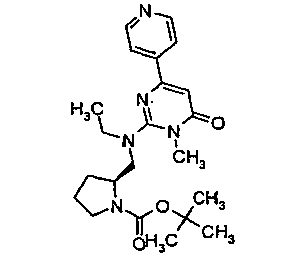 | *tert*-butyl (2S)-2-{[Ethyl(1-methyl-6-oxo-4-pyridin-4-yl-1,6-dihydropyrimidin-2-yl)amino]methyl}pyr rolidine-1-carboxylate | 414.1 | 4.2 |
| 5 | 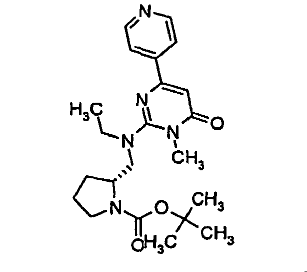 | *tert*-butyl (2R)-2-{[Ethyl(1-methyl-6-oxo-4-pyridin-4-yl-1,6-dihydropyrimidin-2-yl)amino]methyl}pyr rolidine-1-carboxylate | 414.1 | 2.1 |
| 6 | 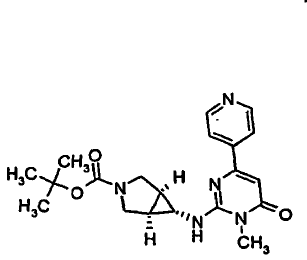 | *tert*-butyl (1R,5S,6s)-6-[(1-Methyl-6-oxo-4-pyridin-4-yl-1,6-dihydropyrimidin-2-yl)amino]-3-azabicyclo[3.1.0]he xane-3-carboxylate | 384.2 | 2.8 |
| 7 | 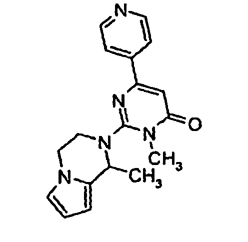 | 3-Methyl-2-(1-methyl-3,4-dihydropyrrolo[1,2-a]pyrazin-2(1H)-yl)-6-pyridin-4-ylpyrimidin-4(3H)-one | 322.1 | 10.7 |
| 8 | 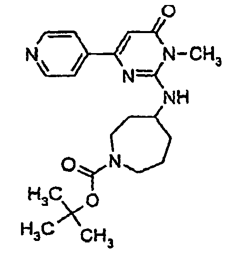 | *tert*-butyl 4-[(1-Methyl-6-oxo-4-Pyridin-4-yl-1,6-dihydropyrimidin-2-yl)amino]azepane-1-carboxylate | 400.1 | 9.1 |
| 9 | 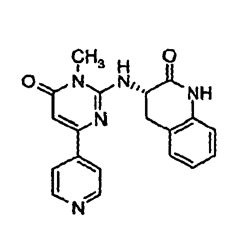 | (3S)-3-[(1-Methyl-6-oxo-4-pyridin-4-yl-1,6-dihydropyrimidin-2-yl)amino]-3,4-dihydroquinolin-2(1H)-one | 348.1 | 30 |
| 10 | 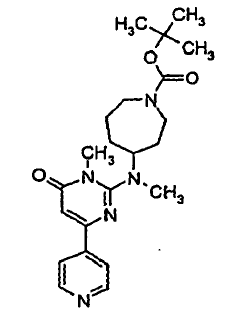 | *tert*-butyl 4-[Methyl(1-methyl-6-oxo-4-pyridin-4-yl-1,6-dihydropyrimidin-2-yl)aminolazepane-1-carboxylate | 414.1 | 14.4 |
| 11 | 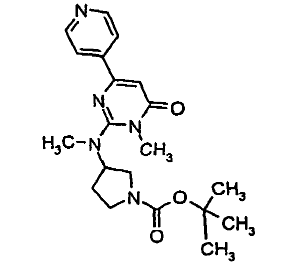 | *tert*-butyl 3-[Methyl(1-methyl-6-oxo-4-pyridin-4-yl-1,6-dihydropyrimidin-2-yl)amino]pyrrolidine -1-carboxylate | 386.1 | 17.7 |
| 12 | 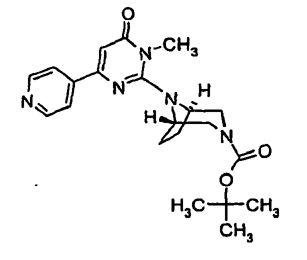 | *tert*-butyl (1R,5S)-8-(1-Methyl-6-oxo-4-pyridin-4-yl-1,6-dihydropyrimidin-2-yl)-3,8-diazabicyclo[3.2.1]o ctane-3-carboxylate | 398.1 | 32.4 |
| 13 | 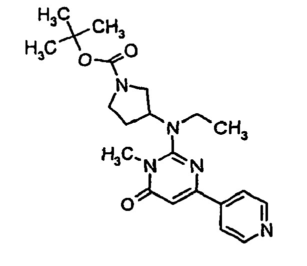 | *tert-*butyl 3-[Ethyl(1-methyl-6-oxo-4-pyridin-4-yl-1,6-dihydropyrimidin-2-yl)amino]pyrrolidine -1-carboxylate | 400.1 | 16.5 |
| 14 | 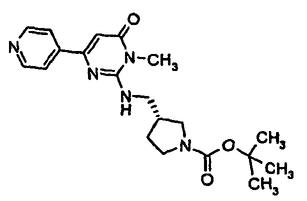 | *tert*-butyl (3R)-3-{[(1-Methyl-6-oxo-4-pyridin-4-yl-1,6-dihydropyrimidin-2-yl)amino]methyl}pyr rolidine-1-carboxylate | 386.1 | 74 |
| 15 | 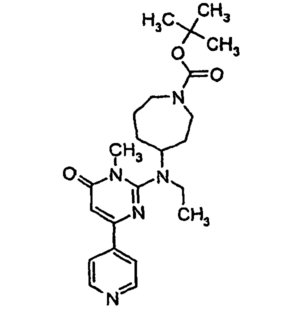 | *tert-*butyl 4-[Ethyl(1-methyl-6-oxo-4-pyridin-4-yl-1,6-dihydropyrimidin-2-yl)amino]azepane-1-carboxylate | 428.2 | 31 |
| 16 | 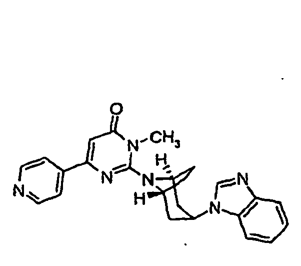 | 2-[(1R,5S)-3-(1H-Benzimidazol-1-yl)-8-azabicyclo[3.2.1]oct -8-yl]-3-methyl-6-pyridin-4-ylpyrimidin-4(3H)-one | 413.1 | 20.5 |
| 17 | 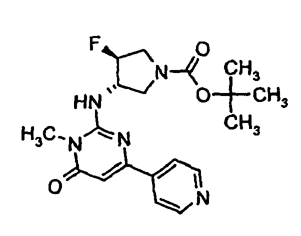 | *tert*-butyl (3S,4S)-3-Fluoro-4-[(1-methyl-6-oxo-4-pyridin-4-yl-1,6-dihydropyrimidin-2-yl)amino]pyrrolidine -1-carboxylate | 390.1 | 26.3 |
| 18 | 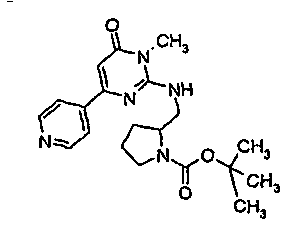 | *tert*-butyl 2-{[(1-Methyl-6-oxo-4-pyridin-4-yl-1,6-dihydropyrimidin-2-yl)amino]methyl}pyr rolidine-1-carboxylate | 386.1 | 107.5 |
| 19 | 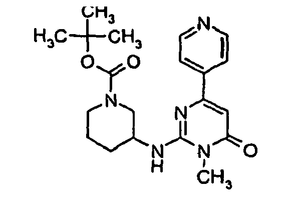 | *tert*-butyl 3-[(1-Methyl-6-oxo-4-pyridin-4-yl-1,6-dihydropyrimidin-2-yl)amino]piperidine-1-carboxylate | 386.1 | 42 |
| 20 | 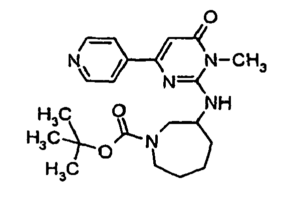 | *tert*-butyl 3-[(1-Methyl-6-oxo-4-Pyridin-4-yl-1,6-dihydropyrimidin-2-yl)amino]azepane-1-carboxylate | 400.1 | 24.1 |
| 21 | 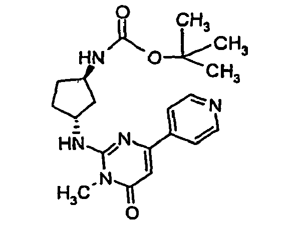 | *tert*-butyl {(1R,3R)-3-[(1-Methyl-6-oxo-4-pyridin-4-yl-1,6-dihydropyrimidin-2-yl)amino]cyclopenty l}carbamate | 386.1 | 52.5 |
| 22 | 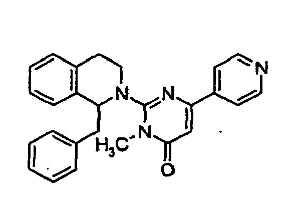 | 2-(1-Benzyl-3,4-dihydroisoquinolin-2(1H)-yl)-3-methyl-6-pyridin-4-ylpyrimidin-4(3H)-one | 409.1 | 37 |
| 23 | 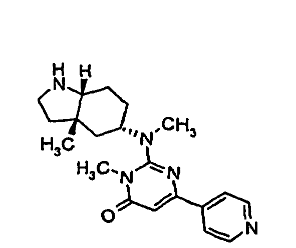 | 3-methyl-2-{Methyl[(3aS,5S,7a R)-3a-methyloctahydro-1H-indol-5-yl]amino}-6-pyridin-4-ylpyrimidin-4(3H)-one | 354.2 | 47 |
| 24 | 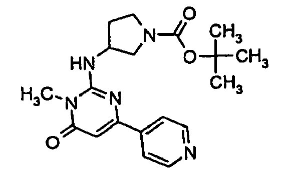 | *tert*-butyl 3-[(1-Methyl-6-oxo-4-pyridin-4-yl-1,6-dihydropyrimidin-2-yl)amino]pyrrolidine -1-carboxylate | 372.1 | 43 |
| 25 | 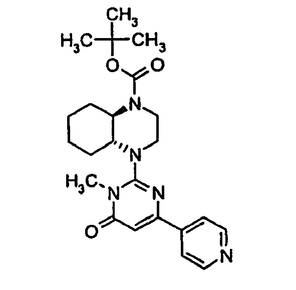 | *tert*-butyl (4aR,8aR)-4-(1-Methyl-6-oxo-4-pyridin-4-yl-1,6-dihydropyrimidin-2-yl)octahydroquinox aline-1 (2H)-carboxylate | 426.1 | 40 |
| 26 | 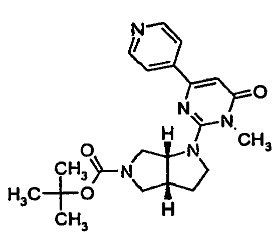 | *tert*-butyl (3aR,6aR)-1-(1-Methyl-6-oxo-4-pyridin-4-yl-1,6-dihydropyrimidin-2-yl)hexahydropyrrolo [3,4-b]pyrrole-5(1H)-carboxylate | 398.1 | 97 |
| 27 | 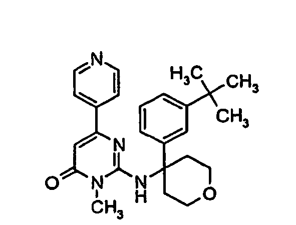 | 2-{[4-(3-*tert*-Butylphenyl)tetrahy dro-2H-pyran-4-yl]amino}-3-methyl-6-pyridin-4-ylpyrimidin-4(3H)-one | 419.1 | 12 |
| 28 | 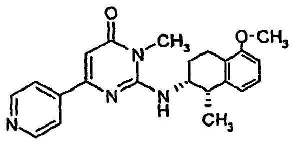 | 2-{[(1S,2R)-5-Methoxy-1-methyl-1,2,3,4-tetrahydronaphthal en-2-yl]amino}-3-methyl-6-pyridin-4-y)pyrimidin-4(3H)-one | 377.1 | 3.9 |
| 29 | 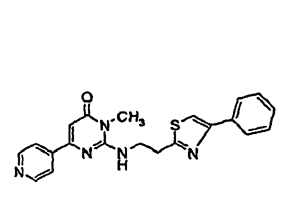 | 3-Methyl-2-{[2-(4-phenyl-1,3-thiazol-2-yl)ethy]amino}-6-pyridin-4-ylpyrimidin-4(3H)-one | 390 | 16 |
| 30 | 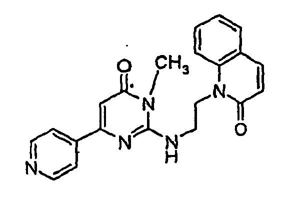 | 1-{2-[(1-Methyl-6-oxo-4-pyridin-4-yl-1,6-dihydropyrimidin-2-yl)amino]ethyl}quin olin-2(1H)-one | 374.1 | 25 |
| 31 | 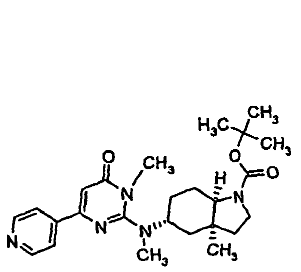 | *tert*-butyl (3aS,5R,7aR)-3a-Methyl-5-[methyl(1-methyl-6-oxo-4-pyridin-4-yl-1,6-dihydropyrimidin-2-yl)amino]octahydro-1H-indole-1-carboxylate | 454.2 | 30.1 |
| 32 | 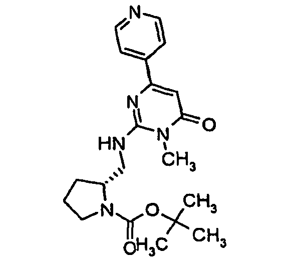 | *tert*-butyl (2R)-2-{[(1-Methyl-6-oxo-4-pyridin-4-yl-1,6-dihydropyrimidin-2-yl)amino]methyl}pyr rolidine-1-carboxylate | 386.1 | 71 |
| 33 | 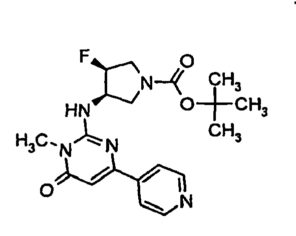 | *tert*-butyl (3S,4R)-3-Fluoro-4-[(1-methyl-6-oxo-4-pyridin-4-yl-1,6-dihydropyrimidin-2-yl)amino]pyrrolidine -1-carboxylate | 390.1 | 77 |
| 34 | 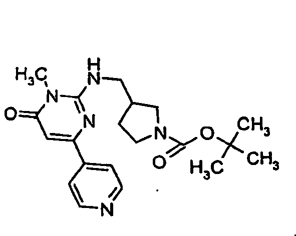 | *tert*-butyl 3-{[(1-Methyl-6-oxo-4-pyridin-4-yl-1,6-dihydropyrimidin-2-yl)amino]methyl}pyr rolidine-1-carboxylate | 386.1 | 113.5 |
| 35 | 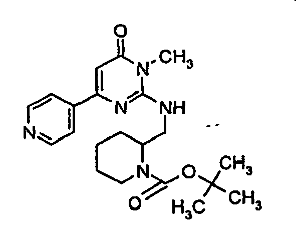 | *tert*-butyl 2-{[(1-Methyl-6-oxo-4-pyridin-4-yl-1,6-dihydropyrimidin-2-yl)amino]methyl}pip eridine-1-carboxylate | 400.1 | 168.5 |

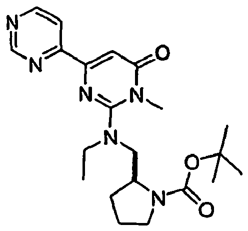

**Example 36: *tert*-butyl (2S)-2-{[Ethyl(1-methyl-6-oxo-4-pyrimidin-4-yl-1,6-dihydropyrimidin-2-yl)amino]methyl}pyrrolidine-1-carboxylate:** To a solution of the product of Preparation 2 (67 mg, 0.30 mmol) in DMF (1.5 ml) was added (S)-*tert*-butyl-2-((ethylamino)methyl)pyrrolidine-1-carboxylate (82 mg, 0.36 mmol), then TEA (0.1 ml, 0.7 mmol). The reaction was heated to 80°C for 16 hr. The reaction was partitioned between EtOAc and water and the organic layer was separated and adsorbed onto silica gel. The crude residue was purified by column chromatography over 60-120 mesh silica gel eluting with a gradient of 50-100% EtOAc in hexanes to give the title compound as a yellow semi-solid (104 mg, 83%). ¹H-NMR(CDCl₃): δ ppm 9.22(s, 1H) 8.90 (d, *J*=5 Hz, 1 H). 8.51 (d, *J*=5 Hz, 1 H), 7.18(s, 1H), 4.19(m, 1H), 3.68(m, 1H), 3.47-3.29(m, 5H), 2.00-1.70(m, 4H), 1.42(s, 9H), 1.25(t, *J*=7 Hz, 3H), Calc MW: 414.5, Found: 415.4 (MH+).

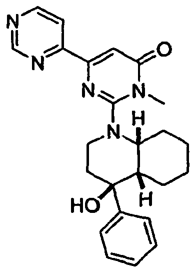

**Example 37: 2-[(4S,4aS,8aR)-4-Hydroxy-4-phenyloctahydroquinolin-1(2H)-yl]-3-methyl-6-pyrimidin-4-ylpyrimidin-4(3H)-one:** To a solution of the product of Preparation 2 (67 mg, 0.30 mmols) in DMF (1.5 ml) was added (4S,4aS,8aR)-4-phenyl-decahydroquinolin-4-ol (8 2mg, 0.36 mmol), then TEA (0.1 ml, 0.7 mmol). The reaction was heated to 80°C for 16 hr. The reaction was partitioned between EtOAc and water and the organic was separated and adsorbed onto silica gel. The crude residue was purified by column chromatography over 60-120 mesh silica gel eluting with a gradient of 50-100% EtOAc in hexanes to give the title compound as a yellow semi-solid (47 mg, 37%). ¹H-NMR(CDCl₃): δ ppm 9.32(s, 1H), 9.05 (d, *J*=5 Hz, 1 H), 8.2 (d, *J*=5 Hz, 1 H), 7.49 (d, *J*=7.5 Hz, 1 H), 7.32 (t, *J*=7.5 Hz, 1 H), 7.20 (t, *J*=7.5 Hz, 1 H), 4.95(s, 1H), 3.49(m, 1H), 3.29(s, 3H), 3.22-3.06(m, 2H), 2.26(m, 1H), 2.05(m, 1H), 1.88(m, 1H), 1.66-1.49(m, 3H), 1.29-0.97(m, 5H). Calc MW: 417.5, Found: 418.5 (MH+). 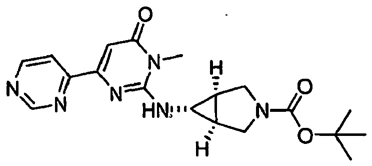 **Example 38: *tert*-butyl (1R,5S,6s)-6-[(1-Methyl-6-oxo-4- pyrimidin -4-yl-1,6-dihydropyrimidin-2-yl)amino]-3-azabicyclo[3.1.0]hexane-3-carboxylate:** To a solution of the product of Preparation 3 (20 mg, 0.07mmol) in DMF (0.5 ml) was added (1R,5S,6s)-*tert-*butyl 6-amino-3-aza-bicyclo[3.1.0]hexane-3-carboxylate (20 mg, 0.10 mmol), followed by the addition of TEA (0.1 ml, 0.7 mmol). The reaction was heated to 80°C for 16 hr., cooled and diluted with DMSO (0.5 ml). The crude mixture was purified on prep. HPLC eluting with mixture of acetonitrile and water, with 0.01% ammonium hydroxide modifier, to give the title compound as a yellow semi-solid (19.9 mg, 68%). ¹H-NMR(CDCl₃): δ ppm 9.22(s, 1H), 8.93 (d, *J*=5 Hz, 1 H), 8.34 (d, J=5 Hz, 1 H), 6.99(s, 1H), 3.75(m, 2H), 3.63(m, 1H), 3.51 (m, 2H), 3.40(s, 3H), 2.55(m, 1H), 1.97(m, 1H), 1.89(m, 1H), 1.48(s, 1H). Calc MW: 384.4, Found 385.4 (MH+).

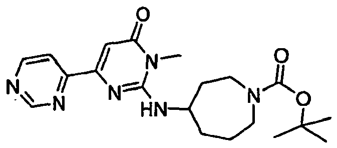

**Example 39: *tert*-butyl 4-[(1-Methyl-6-oxo-4-pyrimidin-4-yl-1,6-dihydropyrimidin-2-yl)amino]azepane-1-carboxylate:** To a solution of product of Preparation 3 (80 mg, 0.30 mmol) in DMF (1.0ml) was added *tert*-butyl 4-aminoazepane-1-carboxylate (75 mg, 0.35 mmol), followed by the addition of TEA (0.15 ml, 1.1 mmol). The reaction was then heated to 80°C for 16 hr., and cooled to RT. The reaction was diluted with DMSO (1.5 ml) and purified on prep. HPLC eluting with mixture of acetonitrile and water, with 0.01% ammonium hydroxide modifier, to give a yellow semi-solid (23.2 mg, 19%). ¹H-NMR(CDCl₃): δ ppm 9.21 (s, 1H), 8.91 (d, *J*=5 Hz, 1 H), 8.32 (d, *J*=5 Hz, 1 H), 8.27 (d, *J*=5 Hz, 1 H), 6.94(s, 1H), 4.27(m, 1H), 3.68-3.36(m, 7H), 2.23(m, 1H), 2.09(m, 1H), 1.99-1.64(m, 4H), 1.49 (d, *J*=5 Hz, 9 H). Calc MW: 400.4, Found: 401.4 (MH+).

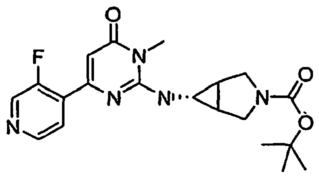

**Example 40: 2-(6-Amino 3-aza-bicyclo[3.1.0]hexane-3-tert-butyl carboxylate)-6-(3-fluoropyridin-4-yl)-3-methylpyrimidin-4(3H)-one:** To a solution of the product of Preparation 4, Step C (50 mg, 0.21 mmol), TEA (58 mg, 0.42 mol), and (1R,5S,6s)-*tert*-butyl 6-amino-3-aza-bicyclo[3.1.0]hexane-3-carboxylate (50 mg, 0.25 mmol) in DMF (0.5 ml) was heated in a microwave (Biotage) at 150 °C for 5 min. Addition of water (5 ml) resulted in a precipitate, which was extracted with EtOAc (2 X 5 ml). The crude residue was purified with prep. TLC using 100% EtOAc as mobile phase to give a white solid (31 mg, 37%). ¹H-NMR (500 MHz, CD3OD): δ ppm 8.57(d, 1H), 8.51 (d, 1 H), 8.16 (q, 1 H), 6.52 (s, 1 H), 3.71 (m, 2H), 3.48 (m, 2H), 3.39 (s, 3H), 2.5 (t, H), 1.9 (m, 2H), 1.46 (s, 9 H). Calc. MW: 401.4, Found: 402.5 (MH+).

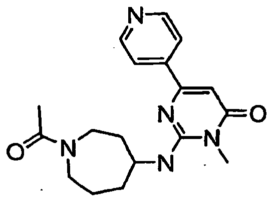

**Example 41: 2-(1-Acetylazepan-4-ylamino)-3-methyl-6-(pyridin-4-yl)pyrimidin-4(3H)-one:** To the product of Example 8 (80 µmol) was added a soln. of TFA (2 ml) in DCE (2 ml). The reaction was shaken for 4 hr. The solvent was evaporated to give a crude residue, which was dissolved in DMF (500 µl). TEA (160 µmol) in DMF (0.2 ml) was added followed by 1-hydroxybenzotrazole/dimethylsulfoxide-N-methylpyrrolidinone (HBTU) (80 µmol) in DMF (0.2 ml). To this was added acetic acid (80 µmol) in DMF (0.1 ml). The reaction was shaken at room temperature for 16 hr. The crude mixture was evaporated, dissolved in DMSO, and purified by prep. HPLC to give the title compound (1.9 mg). Calc. MW: 341.2, Found: 342 (MH+), Retention time 1.37 min.

### General Procedure for Examples 42 to 60

Examples 42 to 60 were prepared using the analogous procedure described to prepare Example 41, substituting the appropriate starting material (Examples 1 to 35) and coupling with the appropriate acid. The assay protocol used is the percent inhibition at 1 µM for GSK-3p in the cell free enzyme assay described above.

| **Ex** | **Structure** | **Name** | **Observed Mass (MH+)** | **Percent inhibition at 1 µM** |
|---|---|---|---|---|
| 42 | 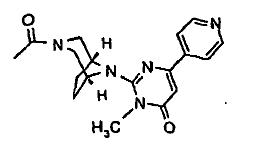 | 2-[(1R,5S)-8-Acetyl-38-diazabicyclo[3.2.1]oct-3-yl]-3-methyl-6-pyridin-4-y)pyrimidin-4(3H)-one | 340 | 74 |
| 43 | 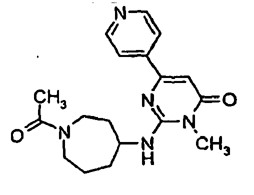 | 2-[(1-Acetylazepan-4-yl)amino]-3-methyl-6-pyridin-4-ylpyrimidin-4(3H)-one | 342 | 84 |
| 44 | 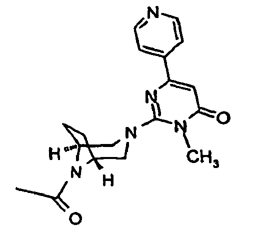 | 2-[(1R,5S)-3-Acetyl-38-diazabicyclo[3.2.1]oct-8-yl]-3-methyl-6-pyridin-4-ylpyrimidin-4(3H)-one | 340 | 72 |
| 45 | 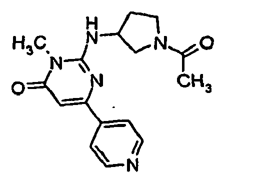 | 2-[(1-Acetylpyrrolidin-3-yl)amino]-3-methyl-6-pyridin-4-ylpyrimidin-4(3H)-one | 314 | 39 |
| 46 | 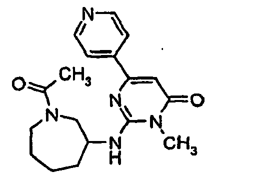 | 2-[(1-Acetylazepan-3-yl)amino]-3-methyl-6-pyridin-4-ylpyrimidin-4(3H)-one | 482 | 51 |
| 47 | 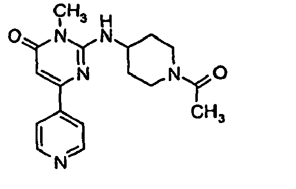 | 2-[(1-Acetylpiperidin-4-yl)amino]-3-methyl-6-pyridin-4-ylpyrimidin-4(3H)-one | 328 | 44 |
| 48 | 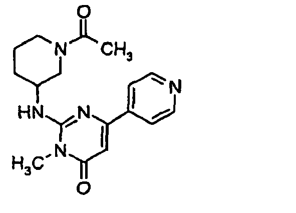 | 2-[(1-Acetylpiperidin-3-yl)amino]-3-methyl-6-pyridin-4-ylpyrimidin-4(3H)-one | 328 | 55 |
| 49 | 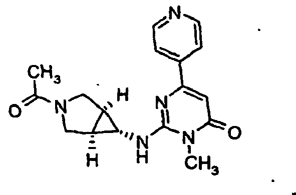 | 2-{[(1R,5S,6s)-3-Acetyl-3-azabicyclo[3.1.0]hex-6-yl]amino}-3-methyl-6-pyridin-4-ylpyrimidin-4(3H)-one | 326 | 75 |
| 50 | 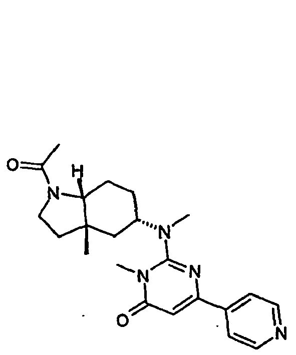 | 2-{[(3a,S5,S7,aR)-1-Acetyl-3a-methyloctahydro-1H-indol-5-yl](methyl)amino}-3-methyl-6-pyridin-4-ylpyrimidin-4(3H)-one | 342 | 82 |
| 51 | 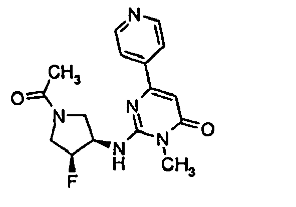 | 2-{[(3R,4S)-1-Acetyl-4-fluoropyrrolidin-3-yl]amino}-3-methyl-6-pyridin-4-ylpyrimidin-4(3H)-one | 332 | 78 |
| 52 | 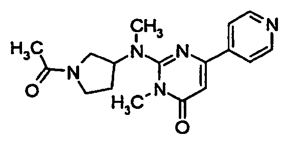 | 2-[(1-Acetylpyrrolidin-3-yl)(methyl)amino]-3-methyl-6-pyridin-4-ylpyrimidin-4(3H)-one | 328 | 51 |
| 53 | 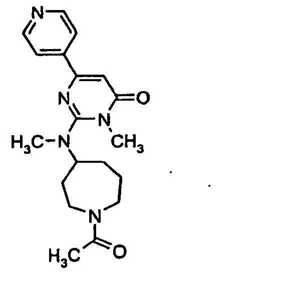 | 2-[(1-Acetylazepan-4-yl)(methyl)amino]-3-methyl-6-pyridin-4-y)pyrimidin-4(3H)-one | 356 | 79 |
| 54 | 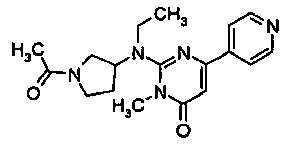 | 2-[(1-Acetylpyrrolidin-3-yl)(ethyl)amino]-3-methyl-6-pyridin-4-ylpyrimidin-4(3H)-one | 342 | 64 |
| 55 | 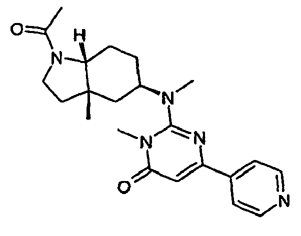 | 2-{[(3a,S5,R7,aR)-1-Acetyl-3a-methyloctahydro-1H-indol-5-yl](methyl)amino}-3-methyl-6-pyridin-4-ylpyrimidin-4(3H)-one | 396 | 71 |
| 56 | 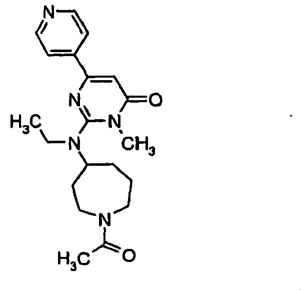 | 2-[(1-Acetylazepan-4-yl)(ethyl)amino]-3-methyl-6-pyridin-4-ylpyrimidin-4(3H)-one | 370 | 85 |
| 57 | 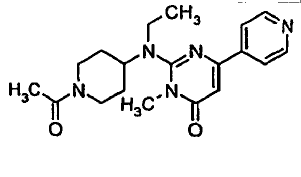 | 2-[(1-Acetylpiperidin-4-yl)(ethyl)amino]-3-methyl-6-pyridin-4-ylpyrimidin-4(3H)-one | 356 | 93 |
| 58 | 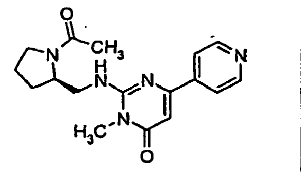 | 2-({[(2R)-1-Acetylpyrrolidin-2-yl]methyl}amino)-3-methyl-6-pyridin-4-ylpyrimidin-4(3H)-one | 328 | 64 |
| 59 | 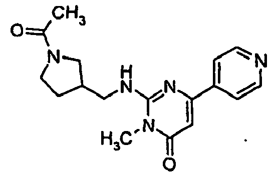 | 2-{[(1-Acetylpyrrolidin-3-yl)methyl]amino}-3-methyl-6-pyridin-4-ylpyrimidin-4(3H)-one | 328 | 37 |
| 60 | 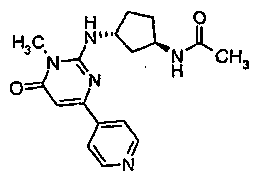 | N-{(1R,3R)-3-[(1-Methyl-6-oxo-4-pyridin-4-yl-16-dihydropyrimidin-2-yl)amino]cyclopentyl}ac etamide | 328 | 77 |

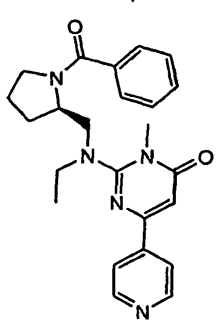

**Example 61: (R)-2-(((1-Benzoylpyrrolidin-2-yl)methyl)(ethyl)amino)-3-methyl-6-(pyridin-4-yl)pyrimidin-4(3H)-one:** To the product of Example 4 (80 µmol) was added TFA (2 ml) in DCE (2 ml) and the mixture was shaken for 4 hr. The solvent was removed and the residue was dissolved in DMF (500 µl). TEA (160 µmol) in DMF (0.2 ml) was added followed by benzoylchloride (80 µmol) in DMF (0.2 ml). The reaction was shaken at RT for 16 hr. The crude mixture was evaporated, then dissolved in DMSO and purified by prep. HPLC to give the title compound (4.0 mg). Calc. MW: 417.5, Found: 418 (MH+), Retention time: 1.89 min.

### General Procedure for Examples 62 to 155

Examples 62 to 155 were prepared by using the analogous procedure described to prepare Example 61, substituting the appropriate starting material (Examples 1 to 35) and coupling with the appropriate acid chloride.

| **Ex.** | **Structure** | **Name** | **Observed Mass (MH+)** | **Enzyme IC₅₀ (nM)** |
|---|---|---|---|---|
| 62 | 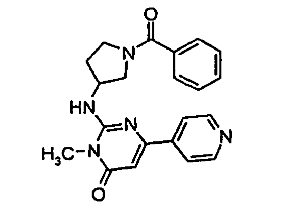 | 2-[(1-Benzoylpyrrolidin-3-yl)amino]-3-methyl-6-pyridin-4-ylpyrimidin-4(3H)-one | 376 | 77.5 |
| 63 | 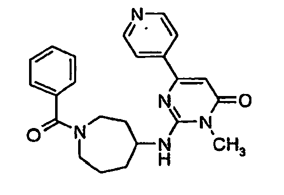 | 2-[(1-Benzoylazepan-4-yl)amino]-3-methyl-6-pyridin-4-ylpyrimidin-4(3H)-one | 404 | 12.4 |
| 64 | 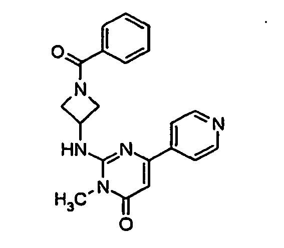 | 2-[(1-Benzoylazetidin-3-yl)amino]-3-methyl-6-pyridin-4-ylpyrimidin-4(3H)-one | 362 | 367 |
| 65 | 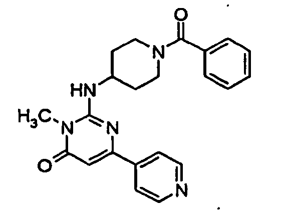 | 2-[(1-Benzoylpiperidin-4-yl)amino]-3-methyl-6-pyridin-4-ylpyrimidin-4(3H)-one | 390 | 202 |
| 66 | 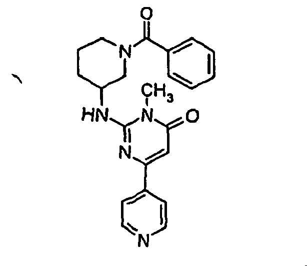 | 2-[(1-Benzoylpiperidin-3-yl)amino]-3-methyl-6-pyridin-4-ylpyrimidin-4(3H)-one | 390 | 1,000 |
| 67 | 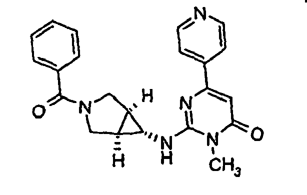 | 2-{[(1R,5S,6s)-3-Benzoyl-3-azabicyclo[3.1.0]hex-6-yl]amino}-3-methyl-6-pyridin-4-ylpyrimidin-4(3H)-one | 388 | 2.00 |
| 68 | 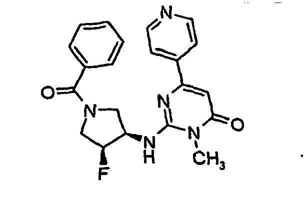 | 2-{[(3R,4S)-1-Benzoyl-4-fluoropyrrolidin-3-yl]amino}-3-methyl-6-pyridin-4-ylpyrimidin-4(3H)-one | 394 | 56.0 |
| 69 | 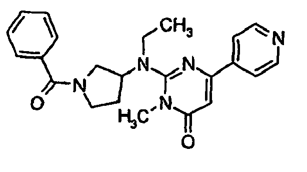 | 2-[(1-Benzoylpyrrolidin-3-yl)(ethyl)amino]-3-methyl-6-pyridin-4-ylpyrimidin-4(3H)-one | 404 | 96.1 |
| 70 | 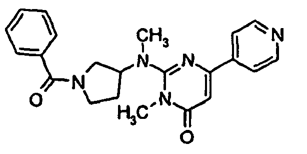 | 2-[(1-Benzoylpyrrolidin-3-yl)(methyl)amino]-3-methyl-6-pyridin-4-ylpyrimidin-4(3H)-one | 390 | 170.3 |
| 71 | 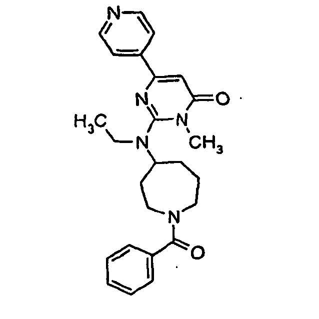 | 2-[(1-Benzoylazepan-4-yl)(ethyl)aminol-3-methyl-6-pyridin-4-ylpyrimidin-4(3H)-one | 432 | 36.3 |
| 72 | 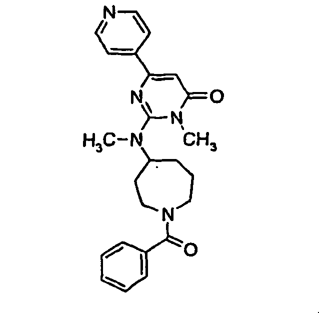 | 2-[(1-Benzoylazepan-4-yl)(methyl)amino]-3-methyl-6-pyridin-4-ylpyrimidin-4(3H)-one | 418 | 61.5 |
| 73 | 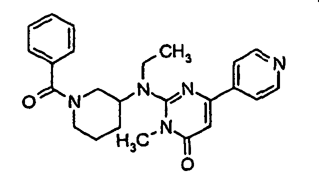 | 2-[(1-Benzoylpiperidin-3-yl)(ethyl)amino]-3-methyl-6-pyridin-4-ylpyrimidin-4(3H)-one | 418 | 345 |
| 74 | 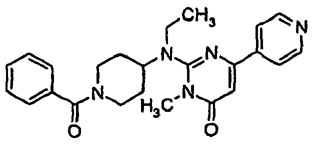 | 2-[(1-Benzoylpiperidin-4-yl)(ethyl)amino]-3-methyl-6-pyridin-4-ylpyrimidin-4(3H)-one | 418 | 143.9 |
| 75 | 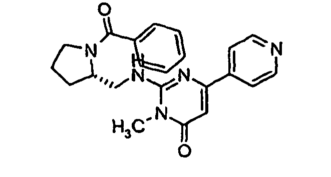 | 2-({[(2S)-1-Benzoylpyrrolidin-2-yl]methyl}amino)-3-methyl-6-pyridin-4-ylpyrimidin-4(3H)-one | 390 | 183 |
| 76 | 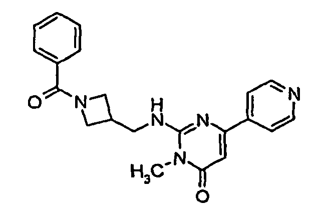 | 2-{[(1-Benzoylazetidin-3-yl)methyl]amino}-3-methyl-6-pyridin-4-ylpyrimidin-4(3H)-one | 376 | 72.4 |
| 77 | 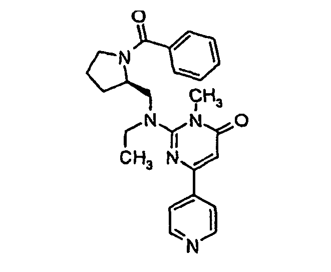 | 2-[{[(2R)-1-Benzoylpyrrolidin-2-yl]methyl}(ethyl)amino]-3-methyl-6-pyridin-4-ylpyrimidin-4(3H)-one | 418 | 29.5 |
| 78 | 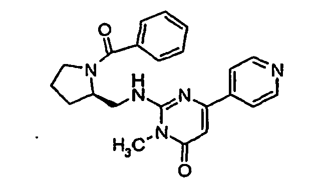 | 2-({[(2R)-1-Benzoylpyrrolidin-2-yl]methyl}amino)-3-methyl-6-pyridin-4-ylpyrimidin-4(3H)-one | 390 | 277 |
| 79 | 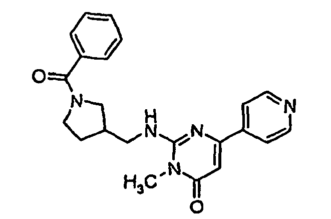 | 2-{[(1-Benzoylpyrrolidin-3-yl)methyl]amino}-3-methyl-6-pyridin-4-ylpyrimidin-4(3H)-one | 390 | 69.9 |
| 80 | 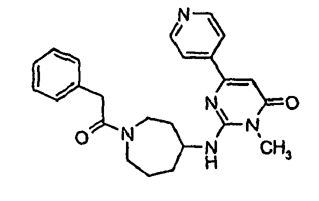 | 3-Methyl-2-{[1-(phenylacetyl)azepan-4-yl]amino}-6-pyridin-4-ylpyrimidin-4(3H)-one | 418 | 53.8 |
| 81 | 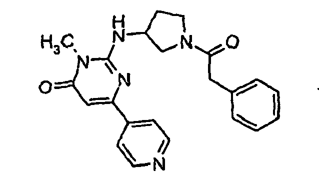 | 3-Methyl-2-{[1-(phenylacetyl)pyrrolidin-3-yl]amino}-6-pyridin-4-ylpyrimidin-4(3H)-one | 390 | 111.4 |
| 82 | 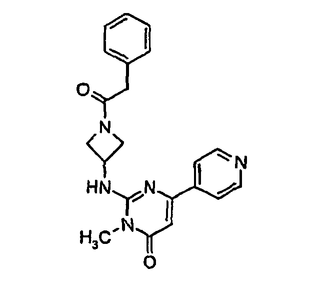 | 3-Methyl-2-{[1-(phenylacetyl)azetidin-3-yl]amino}-6-pyridin-4-ylpyrimidin-4(3H)-one | 376 | 818 |
| 83 | 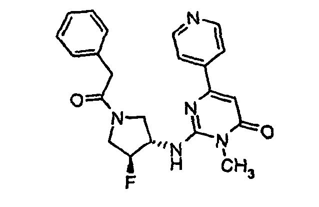 | 2-{[(3S,4S)-4-Fluoro-1-(phenylacetyl)pyrrolidin-3-yl]amino}-3-methyl-6-pyridin-4-ylpyrimidin-4(3H)-one | 408 | 39 |
| 84 | 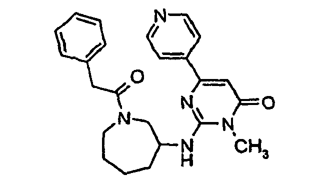 | 3-Methyl-2-{[1-(phenylacetyl)azepan-3-yl]amino}-6-pyridin-4-ylpyrimidin-4(3H)-one | 418 | 674 |
| 85 | 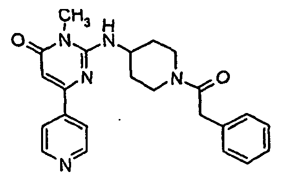 | 3-Methyl-2-{[1-(phenylacetyl)piperidin-4-yl]amino}-6-pyridin-4-ylpyrimidin-4(3H)-one | 404 | 333 |
| 86 | 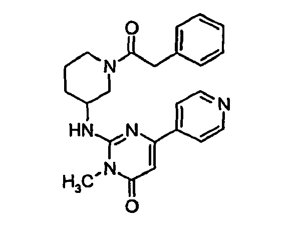 | 3-Methyl-2-{[1-(phenylacetyl)piperidin-3-yl]amino}-6-pyridin-4-ylpyrimidin-4(3H)-one | 404 | 136 |
| 87 | 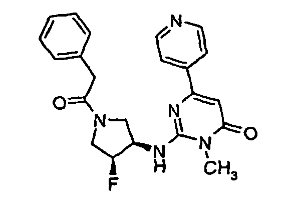 | 2-{[(3R,4S)-4-Fluoro-1-(phenylacetyl)pyrrolidin-3-yl]amino}-3-methyl-6-pyridin-4-ylpyrimidin-4(3H)-one | 408 | 285 |
| 88 | 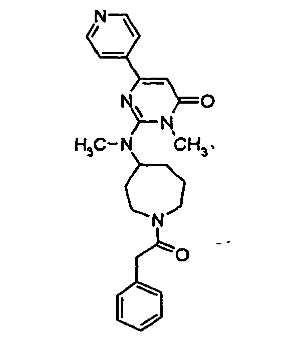 | 3-Methyl-2-{methyl[1-(phenylacetyl)azepan-4-yl]amino}-6-pyridin-4-ylpyrimidin-4(3H)-one | 432 | 102 |
| 89 | 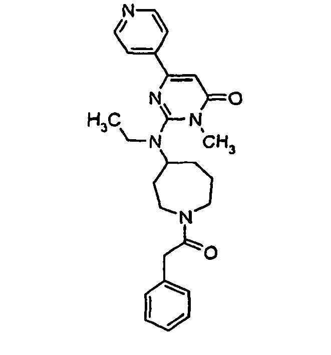 | 2-{Ethyl[1-(phenylacetyl)azepan-4-yl]amino}-3-methyl-6-pyridin-4-ylpyrimidin-4-(3H)-one | 446 | 64.6 |
| 90 | 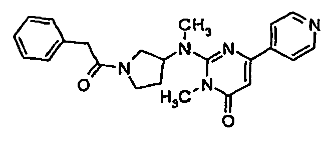 | 3-Methyl-2-{methyl[1-(phenylacetyl)pyrrolidin-3-yl]amino}-6-pyridin-4-ylpyrimidin-4(3H)-one | 404 | 132.6 |
| 91 | 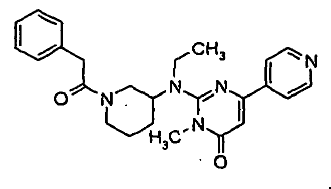 | 2-{Ethyl[1-(phenylacetyl)piperidin-3-yl]amino}-3-methyl-6-pyridin-4-ylpyrimidin-4(3H)-one | 432 | 147 |
| 92 | 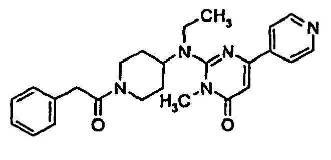 | 2-{Ethyl[1-(phenylacetyl)piperidin-4-yl]amino}-3-methyl-6-pyridin-4-ylpyrimidin-4(3H)-one | 432 | 105 |
| 93 | 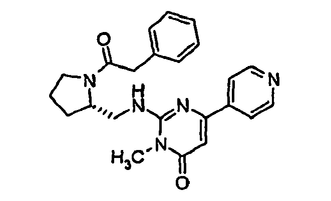 | 3-Methyl-2-({[(2S)-1-(phenylacetyl)pyrrolidin-2-yl]methyl}amino)-6-pyridin-4-ylpyrimidin-4(3H)-one | 404 | 878 |
| 94 | 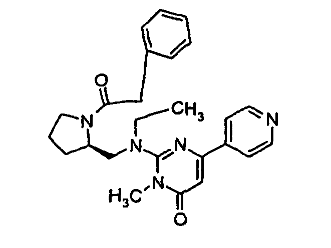 | 2-(Ethyl{[(2R)-1-(phenylacetyl)pyrrolidin-2-yl]methyl}amino)-3-methyl-6-pyridin-4-ylpyrimidin-4(3H)-one | 432 | 18.2 |
| 95 | 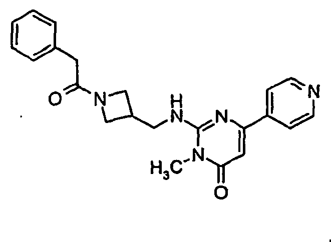 | 3-Methyl-2-({[1-(phenylacetyl)azetid in-3-yl]methyl}amino)-6-pyridin-4-ylpyrimidin-4(3H)-one | 390 | 1,381 |
| 96 | 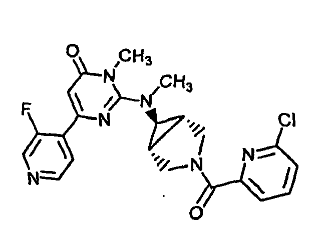 | 2-[{(1R,5S,6s)-3-[(6-Chloropyridin-2-yl)carbonyl]-3-azabicyclo[3.1.0]hex-6-yl}(methyl)amino]-6-(3-fluoropyridin-4-yl)-3-methylpyrimidin-4(3H)-one | 455 | 3.92 |
| 97 | 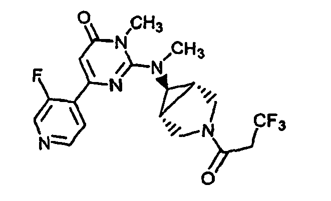 | 6-(3-Fluoropyridin-4-yl)-3-methyl-2-{methyl[(1R,5S,6s)-3-(3,3,3-trifluoropropanoyl)-3-azabicyclo[3.1.0]hex-6-yl]amino}pyrimidin-4(3H)-one | 426 | 55.4 |
| 98 | 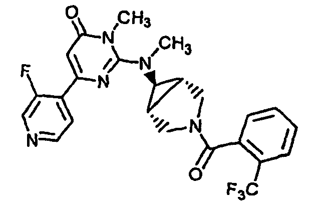 | 6-(3-Fluoropyridin-4-yl)-3-methyl-2-(methyl{(1R,5S,6s)-3-[2-(trifluoromethyl)benzoyl]-3-azabicyclo[3.1.0]hex-6-yl}amino)pyrimidin-4(3H)-one | 488 | 4.39 |
| 99 | 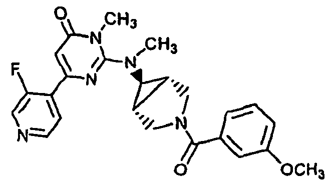 | 6-(3-Fluoropyridin-4-yl)-2-{[(1R,5S,6s)-3-(3-methoxybenzoyl)-3-azabicyclo[3.1.0]hex-6-yl](methyl)amino}-3-methylpyrimidin-4(3H)-one | 450 | 1.46 |
| 100 | 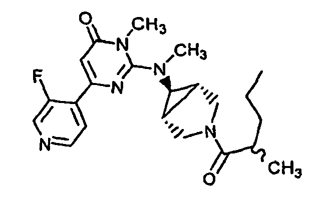 | 6-(3-Fluoropyridin-4-yl)-3-methyl-2-{methyl[(1R,5S,6s)-3-(2-methylpentanoyl)-3-azabicyclo[3.1.0]hex-6-yl]amino}pyrimidin-4(3H)-one | 414 | 9.02 |
| 101 | 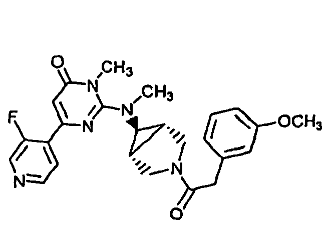 | 6-(3-Fluoropyridin-4-yl)-2-[{(1R,5S,6s)-3-[(3-methoxyphenyl)acetyl]-3-azabicyclo[3.1.0]hex-6-yl}(methyl)amino]-3-methylpyrimidin-4(3H)-one | 464 | 13.6 |
| 102 | 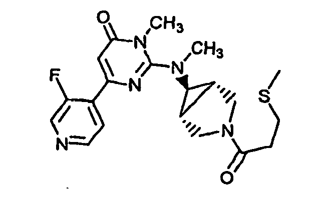 | 6-(3-Fluoropyridin-4-yl)-3-methyl-2-(methyl{(1R,5S,6s)-3-[3-(methylthio)propanoyl]-3-azabicyclo[3.1.0]hex-6-yl}amino)pyrimidin-4(3H)-one | 418 | 16.0 |
| 103 | 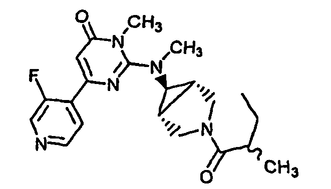 | 6-(3-Fluoropyrdin-4-yl)-3-methyl-2-{methyl[(1R,5S,6s)-3-(2-methylbutanoyl)-3-azabicyclo[3.1.0]hex-6-yl]amino}pyrimidin-4(3H)-one | 400 | 2.77 |
| 104 | 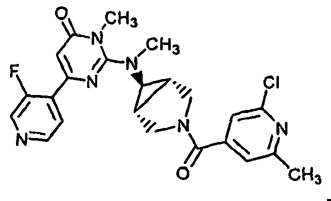 | 2-{[(1R,5S,6s)-3-(2-Chloro-6-methylisonicotinoyl)-3-azabicyclo[3.1.0]hex-6-yl](methyl)amino}-6-(3-fluoropyridin-4-yl)-3-methylpyrimidin-4(3H)-one | 469 | 16.1 |
| 105 | 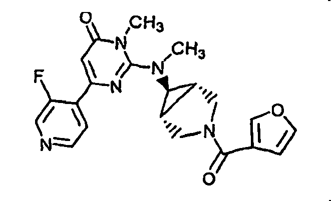 | 6-(3-Fluoropyridin-4-y)-2-{[(1R,5S,6s)-3-(3-furoyl)-3-azabicyclo[3.1.0]hex-6-yl](methyl)amino}-3-methylpyrimidin-4(3H)-one | 410 | 8.81 |
| 106 | 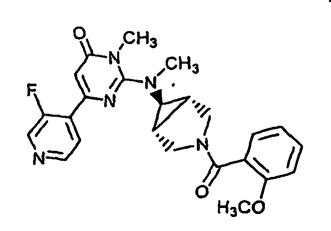 | 6-(3-Fluoropyridin-4-yl)-2-{[(1R,5S,6s)-3-(2-methoxybenzoyl)-3-azabicyclo[3.1.0]hex-6-yl](methyl)amino}-3-methylpyrimidin-4(3H)-one | 450 | 0.91 |
| 107 | 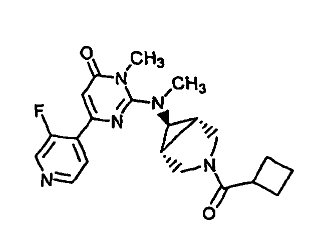 | 2-{[(1R,5S.6s)-3-(Cyclobutylcarbonyl)-3-azabicyclo[3.1.0]hex-6-yl](methyl)amino}-6-(3-fluoropyridin-4-yl)-3-methylpyrimidin-4(3H)-one | 398 | 18.4 |
| 108 | 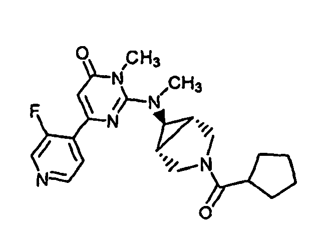 | 2-{[(1R,5S,6s)-3-(Cyclopentylcarbonyl)-3-azabicyclo[3.1.0]hex-6-yl](methyl)amino}-6-(3-fluoropyridin-4-yl)-3-methylpyrimidin-4(3H)-one | 412 | 6.39 |
| 109 | 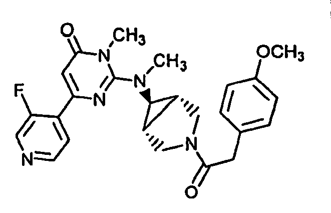 | 6-(3-Fluoropyridin-4-yl)-2-[{(1R,5S,6S)-3-[(4-methoxyphenyl)acetyl]-3-azabicyclo[3.1.0]hex-6-yl}(methyl)amino]-3-methylpyrimidin-4(3H)-one | 464 | 4.03 |
| 110 | 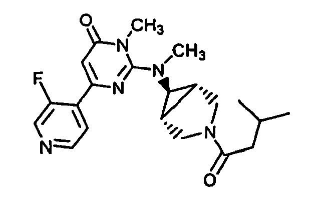 | 6-(3-Fluoropyridin-4-yl)-3-methyl-2-{methyl[(1R,5S,6s)-3-(3-methylbutanoyl)-3-azabicyclo[3.1.0]hex-6-yl]amino}pyrimidin-4(3H)-one | 400 | 18.3 |
| 111 | 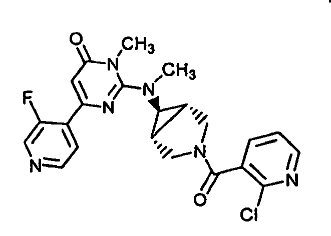 | 2-[{(1R,5S,6s)-3-[(2-Chloropyridin-3-yl)carbonyl]-3-azabicyclo[3.1.0]hex-6-yl}(methyl)amino]-6-(3-fluoropyridin-4-yl)-3-methylpyrimidin-4(3H)-one | 455 | 2.20 |
| 112 | 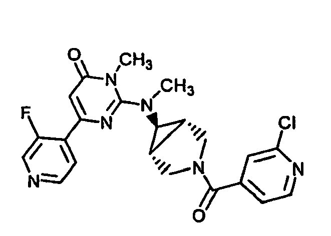 | 2-{[(1R,5S,6s)-3-(2-Chloroisonicotinoyl)-3-azabicyclo[3.1.0]hex-6-yl](methyl)amino}-6-(3-fluoropyridin-4-yl)-3-methylpyrimidin-4(3H)-one | 455 | 2.63 |
| 113 | 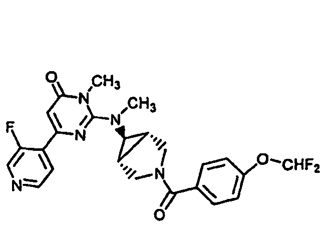 | 2-[{(1R,5S,6s)-3-[4-(Difluoromethoxy)benzoyl]-3-azabicyclo[3.1.0]hex-6-yl}(methyl)amino]-6-(3-fluoropyridin-4-yl)-3-methylpyrimidin-4(3H)-one | 486 | 8.57 |
| 114 | 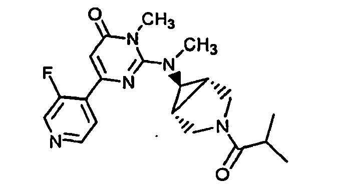 | 6-(3-Fluoropyridin-4-yl)-2-{[(1R,5S,6s)-3-isobutyryl-3-azabicyclo[3.1.0]hex-6-yl](methyl)amino}-3-methylpyrimidin-4(3H)-one | 386 | 15.3 |
| 115 | 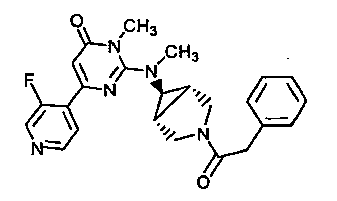 | 6-(3-Fluoropyridin-4-yl)-3-methyl-2-{methyl[(1R,5S,6s)-3-(phenylacetyl)-3-azabicyclo[3.1.0]hex-6-yl]amino}pyrimidin-4(3H)-one | 434 | 14.2 |
| 116 | 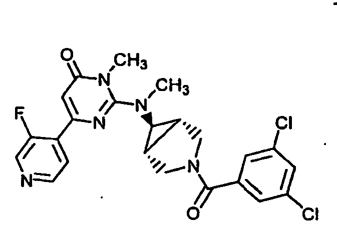 | 2-{[(1R,5S,6s)-3-(3,5-Dichlorobenzoyl)-3-azabicyclo[3.1.0]hex-6-yl](methyl)amino}-6-(3-fluoropyridin-4-yl)-3-methylpyrimidin-4(3H)-one | 488 | 4.40 |
| 117 | 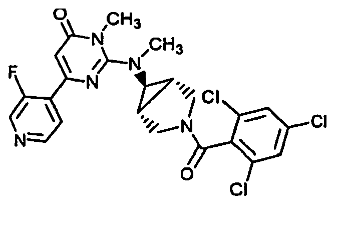 | 6-(3-Fluoropyridin-4-yl)-3-methyl-2-{methyl[(1R,5S,6s)-3-(2,4,6-trifluorobenzoyl)-3-azabicyclo[3.1.0]hex-6-yl]amino}pyrimidin-4(3H)-one | 474 | 1.84 |
| 118 | 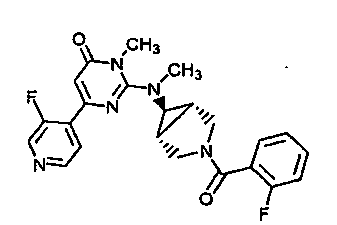 | 2-{[(1R,5S,6s)-3-(2-Fluorobenzoyl)-3-azabicyclo[3.1.0]hex-6-yl](methyl)amino}-6-(3-fluoropyridin-4-yl)-3-methylpyrimidin-4(3H)-one | 438 | 1.11 |
| 119 | 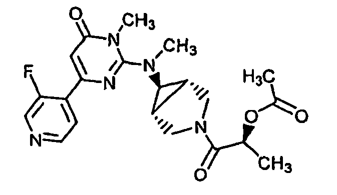 | (1S)-2-[(1R,5S,6R)-6-{[4-(3-Fluoropyridin-4-yl)-1-methyl-6-oxo-1,6-dihydropyrimidin-2-yl](methyl)amino}-3-azabicyclo[3.1.0]hex-3-yl]-1-methyl-2-oxoethyl acetate | 430 | 32.9 |
| 120 | 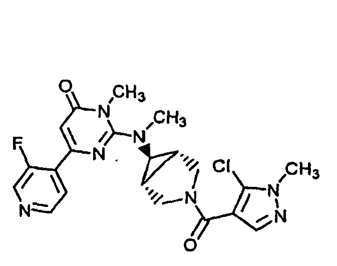 | 2-[{(1R,5S,6s)-3-[(5-Chloro-1-methyl-1H-pyrazol-4-yl)carbonyl]-3-azabicyclo[3.1.0]hex-6-yl}(methyl)amino]-6-(3-fluoropyridin-4-yl)-3-methylpyrimidin-4(3H)-one | 458 | 4.52 |
| 121 | 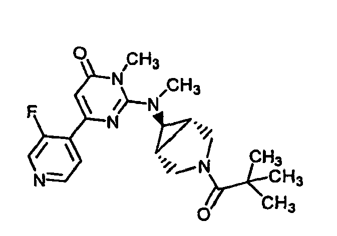 | 2-{[(1R,5S,6s)-3-(2,2-Dimethylpropanoyl)-3-azabicyclo[3.1.0]hex-6-yl](methyl)amino}-6-(3-fluoropyridin-4-yl)-3-methylpyrimidin-4(3H)-one | 400 | 1.41 |
| 122 | 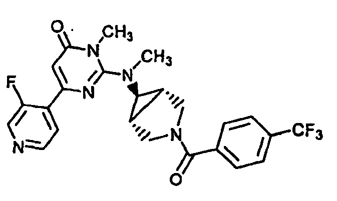 | 6-(3-Fluoropyridin-4-yl)-3-methyl-2-(methyl{(1R,5S,6s)-3-[4-(trifluoromethyl)benzoyl]-3-azabicyclo[3.1.0]hex-6-yl}amino)pyrimidin-4(3H)-one | 488 | 15.1 |
| 123 | 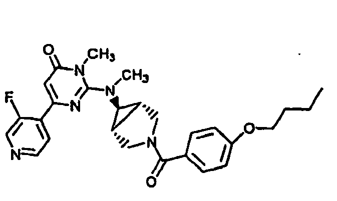 | 2-{[(1R,5S,6s)-3-(4-Butoxybenzoyl)-3-azabicyclo[3.1.0]hex-6-yl](methyl)amino}-6-(3-fluoropyridin-4-yl)-3-methylpyrimidin-4(3H)-one | 492 | 13.6 |
| 124 | 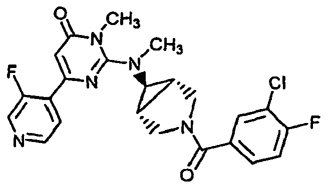 | 2-{[(1R,5S,6s)-3-(3-Chloro-4-fluorobenzoyl)-3-azabicyclo[3.1.0]hex-6-yl](methyl)amino}-6-(3-fluoropyridin-4-yl)-3-methylpyrimidin-4(3H)-one | 472 | 4.58 |
| 125 | 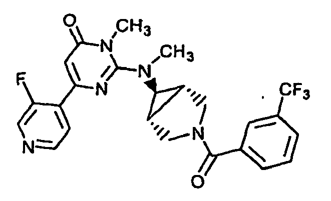 | 6-(3-Fluoropyridin-4-yl)-3-methyl-2-(methyl{(1R,5S,6s)-3-[3-(trifluoromethyl)benzoyl]-3-azabicyclo[3.1.0]hex-6-yl}amino)pyrimidin-4(3H)-one | 488 | 2.11 |
| 126 | 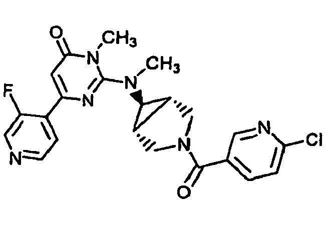 | 2-[{(1R,5S,6s)-3-[(6-Chloropyridin-3-yl)carbonyl]-3-azabicyclo[3.1.0]hex-6-yl}(methyl)amino]-6-(3-fluoropyridin-4-yl)-3-methylpyrimidin-4(3H)-one | 455 | 6.98 |
| 127 | 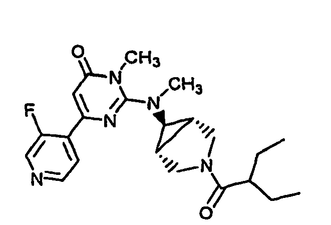 | 2-{[(1R,5S,6s)-3-(2-Ethylbutanoyl)-3-azabicyclo[3.1.0]hex-6-yl](methyl)amino}-6-(3-fluoropyridin-4-yl)-3-methylpyrimidin-4(3H)-one | 414 | 4.41 |
| 128 | 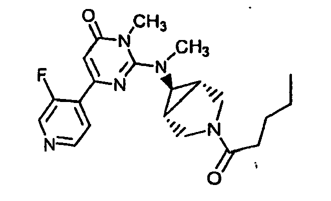 | 6-(3-Fluoropyridin-4-yl)-3-methyl-2-{methyl[(1R,5S,6s)-3-pentanoyl-3-azabicyclo[3.1.0]hex-6-yl]amino}pyrimidin-4(3H)-one | 400 | 16.4 |
| 129 | 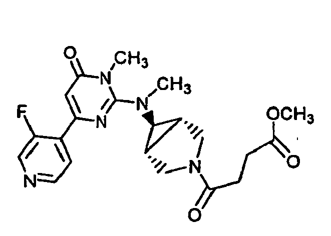 | Methyl 4-[(1R,5S,6s)-6-{[4-(3-fluoropyridin-4-yl)-1-methyl-6-oxo-1,6-dihydropyrimidin-2-yl](methyl)amino}-3-azabicyclo[3.1.0]hex-3-yl]-4-oxobutanoate | 430 | 84.4 |
| 130 | 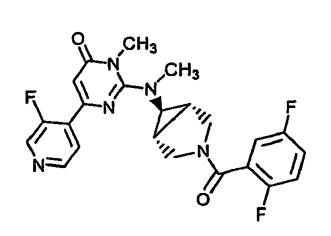 | 2-{[(1R,5S,6s)-3-(2,5-Difluorobenzoyl)-3-azabicyclo[3.1.0]hex-6-yl](methyl)amino}-6-(3-fluoropyridin-4-yl)-3-methylpyrimidin-4(3H)-one | 456 | 1.69 |
| 131 | 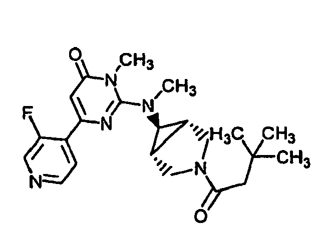 | 2-{[(1R,5S,6s)-3-(3,3-Dimethylbutanoyl)-3-azabicyclo[3.1.0]hex-6-yl](methyl)amino}-6-(3-fluoropyridin-4-yl)-3-methylpyrimidin-4(3H)-one | 414 | 1.57 |
| 132 | 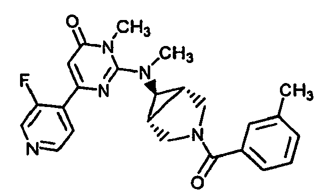 | 6-(3-Fluoropyridin-4-yl)-3-methyl-2-{methyl[(1R,5S,6s)-3-(3-methylbenzoyl)-3-azabicyclo[3.1.0]hex-6-yl]amino}pyrimidin-4(3H)-one | 434 | 1.05 |
| 133 | 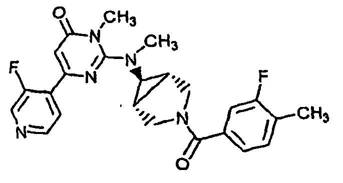 | 2-{[(1R,5S,6s)-3-(3-Fluoro-4-methylbenzoyl)-3-azabicyclo[3.1.0]hex-6-yl](methyl)amino}-6-(3-fluoropyridin-4-yl)-3-methylpyrimidin-4(3H)-one | 452 | 2.91 |
| 134 | 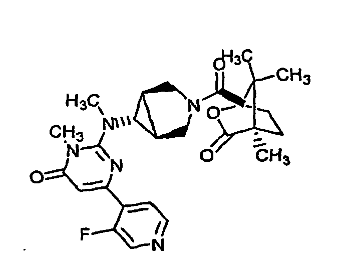 | 6-(3-Fluoropyridin-4-yl)-3-methyl-2-{methyl[(1R,5S,6R)-3-{[(1S,4R)-4,7,7-trimethyl-3-oxo-2-oxabicyclo[2.2.1]hept-1-yl]carbonyl}-3-azabicyclo[3.1.0]hex-6-yl]amino)pyrimidin-4(3H)-one | 496 | 65.7 |
| 135 | 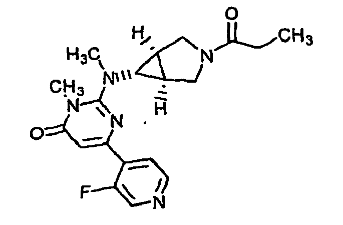 | 6-(3-Fluoropyridin-4-yl)-3-methyl-2-{methyl[(1R,5S,6s)-3-propionyl-3-azabicyclo[3.1.0]hex-6-yl]amino}pyrimidln-4(3H)-one | 372 | 5.46 |
| 136 | 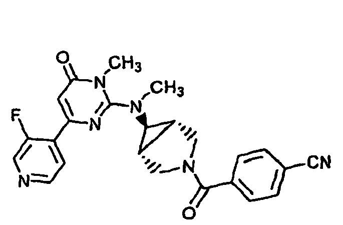 | 4-{[(1R,5S,6s)-6-{[4-(3-Fluoropyridin-4-yl)-1-methyl-6-oxo-1,6-dihydropyrimidin-2-yl](methyl)amino}-3-azabicyclo[3.1.0]hex-3-yl]carbonyl}benzonitrile | 445 | 42.6 |
| 137 | 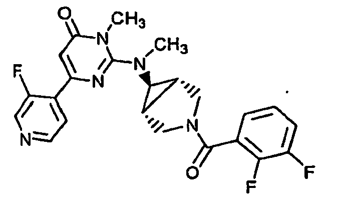 | 2-{[(1R,5S,6s)-3-(2,3-Difluorobenzoyl)-3-azabicyclo[3.1.0]hex-6-yl](methyl)amino}-6-(3-fluoropyridin-4-yl)-3-methylpyrimidin-4(3H)-one | 456 | 3.29 |
| 138 | 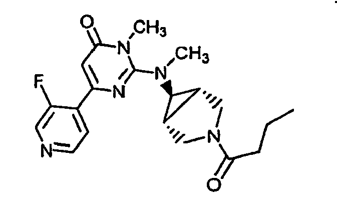 | 2-{[(1R,5S,6s)-3-Butyryl-3-azabicyclo[3.1.0]hex-6-yl](methyl)amino}-6-(3-fluoropyridin-4-yl)-3-methylpyrimidin-4(3H)-one | 386 | 13.8 |
| 139 | 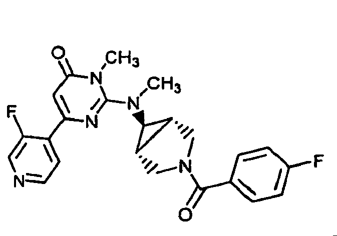 | 2-{[(1R,5S,6s)-3-(4-Fluorobenzoyl)-3-azabicyclo[3.1.0]hex-6-yl](methyl)amino}-6-(3-fluoropyridin-4-yl)-3-methylpyrimidin-4(3H)-one | 438 | 5.67 |
| 140 | 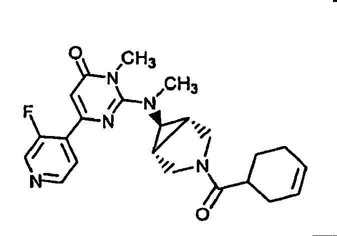 | 2-{[(1R,5S,6s)-3-(Cyclohex-3-en-1-ylcarbonyl)-3-azabicyclo[3.1.0]hex-6-yl](methyl)amino}-6-(3-fluoropyridin-4-yl)-3-methylpyrimidin-4(3H)-one | 424 | 8.72 |
| 141 | 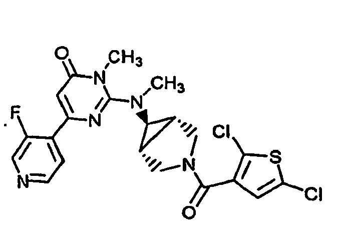 | 2-[{(1R,5S,6s)-3-[(2,5-Dichloro-3-thienyl)carbonyl]-3-azabicyclo[3.1.0]hex-6-yl}(methyl)amino]-6-(3-fluoropyridin-4-yl)-3-methylpyrimidin-4(3H)-one | 494 | 1.04 |
| 142 | 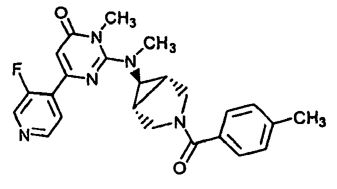 | 6-(3-Fluoropyridin-4-yl)-3-methyl-2-{methyl[(1R,5S,6s)-3-(4-methylbenzoyl)-3-azabicyclo[3.1.0]hex-6-yl]amino}pyrimidin-4(3H)-one | 434 | 3.51 |
| 143 | 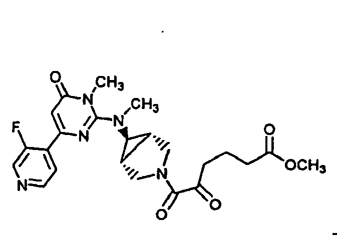 | Methyl 5-[(1R,5S,6s)-6-{[4-(3-fluoropyridin-4-yl)-1-methyl-6-oxo-1,6-dihydropyrimidin-2-yl](methyl)amino}-3-azabicyclo[3.1.0]hex-3-yl]-5-oxopentanoate | 444 | 57.4 |
| 144 | 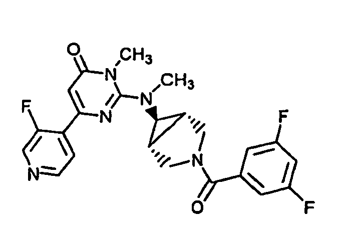 | 2-{[(1R,5S,6s)-3-(3,5-Difluorobenzoyl)-3-azabicyclo[3.1.0]hex-6-yl](methyl)amino}-6-(3-fluoropyridin-4-yl)-3-methylpyrimidin-4(3H)-one | 456 | 4.09 |
| 145 | 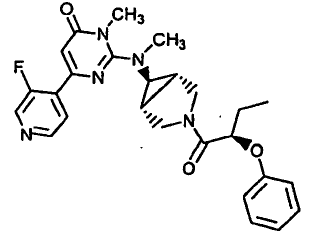 | 6-(3-Fluoropyridin-4-yl)-3-methyl-2-{methyl[(1R,5S,6s)-3-(2-phenoxybutanoyl)-3-azabicyclo[3.1.0]hex-6-yl]amino}pyrimidin-4(3H)-one | 478 | 13.2 |
| 146 | 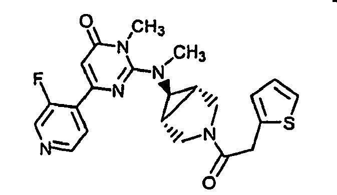 | 6-(3-Fluoropyridin-4-yl)-3-methyl-2-{methyl[(1R,5S,6s)-3-(2-thienylacetyl)-3-azabicyclo[3.1.0]hex-6-yl]amino}pyrimidin-4(3H)-one | 440 | 6.16 |
| 147 | 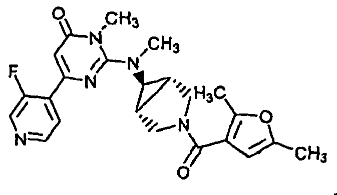 | 2-{[(1R,5S,6s)-3-(2,5-Dimethyl-3-furoyl)-3-azabicyclo[3.1.0]hex-6-yl](methyl)amino}-6-(3-fluoropyridin-4-yl)-3-methylpyrimidin-4(3H)-one | 438 | 3.70 |
| 148 | 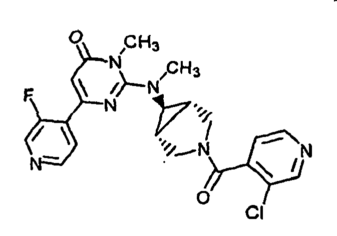 | 2-{[(1R,5S,6s)-3-(3-Chloroisonicotinoyl)-3-azabicyclo[3.1.0]hex-6-yl](methyl)amino}-6-(3-fluoropyridin-4-yl)-3-methylpyrimidin-4(3H)-one | 455 | 36.1 |
| 149 | 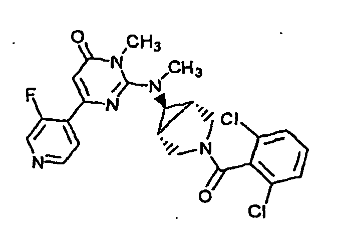 | 2-{[(1R,5S,6s)-3-(2,6-Dichlorobenzoyl)-3-azabicyclo[3.1.0]hex-6-yl](methyl)amino}-6-(3-fluoropyridin-4-yl)-3-methylpyrimidin-4(3H)-one | 488 | 3.81 |
| 150 | 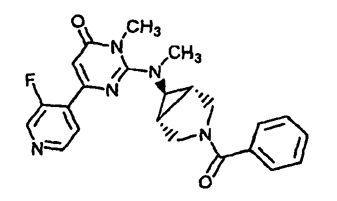 | 2-{[(1R,5S,6s)-3-Benzoyl-3-azabicyclo[3.1.0]hex-6-yl](methyl)amino}-6-(3-fluoropyridin-4-yl)-3-methylpyrimidin-4(3H)-one | 420 | 1.51 |
| 151 | 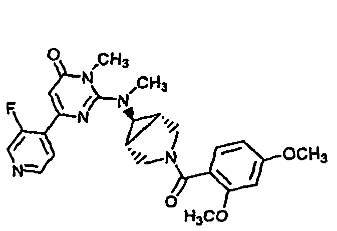 | 2-{[(1R,5S,6s)-3-(2,4-Dimethoxybenzoyl)-3-azabicyclo[3.1.0]hex-6-yl](methyl)amino}-6-(3-fluoropyridin-4-yl)-3-methylpyrimidin-4(3H)-one | 480 | 1.22 |
| 152 | 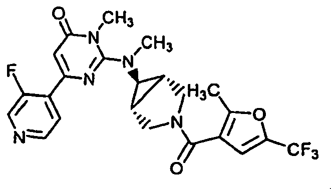 | 6-(3-Fluoropyridin-4-yl)-3-methyl-2-(methyl{(1R,5S,6s)-3-[5-methyl-2-(trifluoromethyl)-3-furoyl]-3-azabicyclo[3.1.0]hex-6-yl}amino)pyrimidin-4(3H)-one | 492 | 11.9 |
| 153 | 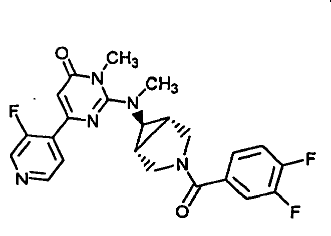 | 2-{[(1R,5S,6s)-3-(3,4-Difluorobenzoyl)-3-azabicyclo[3.1.0]hex-6-yl](methyl)amino}-6-(3-fluoropyridin-4-yl)-3-methylpyrimidin-4(3H)-one | 456 | 7.23 |
| 154 | 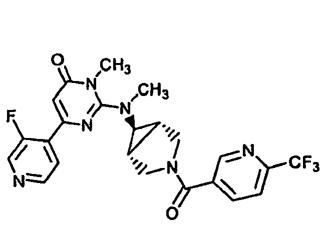 | 6-(3-Fluoropyridin-4-yl)-3-methyl-2-{methyl[(1R,5S,6s)-3-{[6-(trifluoromethyl)pyridin-3-yl]carbonyl}-3-azabicyclo[3.1.0]hex-6-yl]amino}pyrimidin-4(3H)-one | 489 | 26.4 |
| 155 | 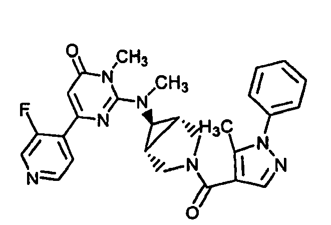 | 6-(3-Fluoropyridin-4-yl)-3-methyl-2-(methyl{(1R,5S,6s)-3-[(5-methyl-1-phenyl-1H-pyrazol-4-yl)carbonyl]-3-azabicyclo[3.1.0]hex-6-yl}amino)pyrimidin-4(3H)-one | 500 | 102 |

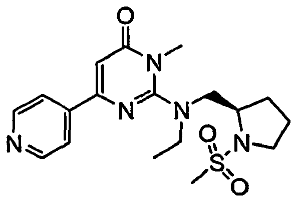

**Example 156: (R)-2-(Ethyl((1-(methylsulfonyl)pyrrolidin-2-yl)methyl)amino)-3-methyl-6-(pyridin-4-yl)pyrimidin-4(3H)-one:** To the product of Example 32 (80 µmol) was added a soln. of TFA (2 ml) in DCE (2 ml) and the mixture was shaken for 4 hr. The solvent was removed to give a residue, which was dissolved in DCE (500 µl). TEA (160 µmol) in DCE (0.2 ml) was added followed by methanesulfonylchloride (80 µmol) in DCE (0.2 ml). The reaction was shaken at RT for 16 hr. The mixture evaporated, then dissolved in DMSO and purified by prep. HPLC to give the title compound (8.9 mg). Calc. MW: 391.5, Found: 392 (MH+), Retention time: 2.41 min.

### General Procedure for Examples 157 to 199

Examples 157 to 199 were prepared by using the analogous procedure described to prepare Example 156 substituting the appropriate starting material (Examples 1 to 35) and coupling with the appropriate sulfonyl chloride.

| **Ex.** | **Structure** | **Name** | **Observed Mass (MH+)** | **Percent inhibition at 1 µM** |
|---|---|---|---|---|
| 157 | 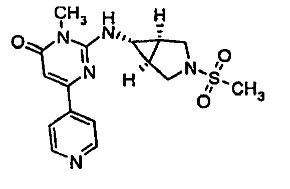 | 3-Methyl-2-{[(1R,5S,6s)-3-(methylsulfonyl)-3-azabicyclo[3.1.0]hex-6-yl]amino}-6-pyridin-4-ylpyrimidin-4(3H)-one | 362 | 61 |
| 158 | 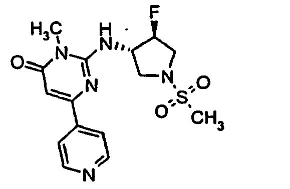 | 2-{[(3S,4S)-4-Fluoro-1-(methylsulfonyl)pyrrolidin-3-yl]amino}-3-methyl-6-pyridin-4-ylpyrimidin-4(3H)-one | 368 | 74 |
| 159 | 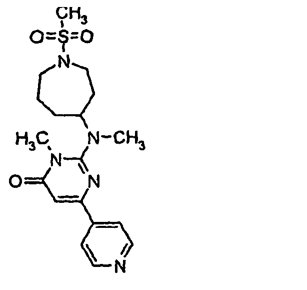 | 3-Methyl-2-{methyl[1-(methylsulfonyl)azepan-4-yl]amino}-6-pyridin-4-ylpyrimidin-4(3H)-one | 392 | 100 |
| 160 | 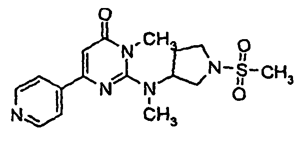 | 3-Methyl-2-{methyl[1-(methylsulfonyl)pyrrolidin-3-yl]amino}-6-pyridin-4-ylpyrimidin-4(3H)-one | 364 | 79 |
| 161 | 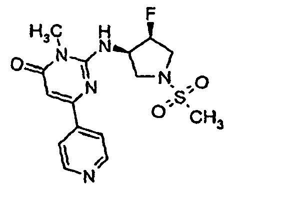 | 2-{[(3R,4S)-4-Fluoro-1-(methylsulfonyl)pyrrolidin-3-yl]amino}-3-methyl-6-pyridin-4-ylpyrimidin-4(3H)-one | 368 | 93 |
| 162 | 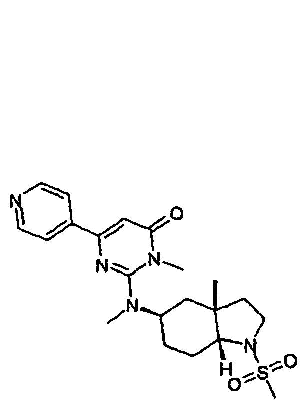 | 3-Methyl-2-{methyl[(3a,S5,R7,aR)-3a-methyl-1-(methylsulfonyl)octahydro -1H-indol-5-yl]amino}-6-pyridin-4-ylpyrimidin-4(3H)-one | 432 | 84 |
| 163 | 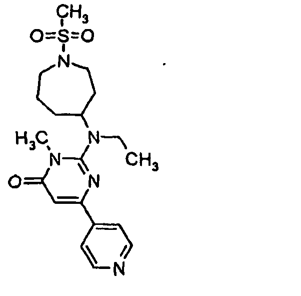 | 2-{Ethyl[1-(methylsulfonyl)azepan-4-yl]amino}-3-methyl-6-pyridin-4-ylpyrimidin-4(3H)-one | 406 | 100 |
| 164 | 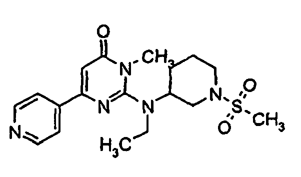 | 2-{Ethyl[1-(methylsulfonyl)piperidin-3-yl]amino}-3-methyl-6-pyridin-4-ylpyrimidin-4(3H)-one | 392 | 82 |
| 165 | 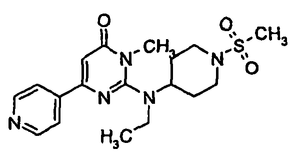 | 2-{Ethyl[1-(methylsulfonyl)piperidin-4-yl]amino}-3-methyl-6-pyridin-4-ylpyrimidin-4(3H)-one | 392 | 80 |
| 166 | 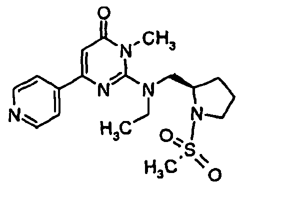 | 2-(Ethyl{[(2R)-1-(methylsulfonyl)pyrrolidin-2-yl]methyl}amino)-3-methyl-6-pyridin-4-ylpyrimidin-4(3H)-one | 392 | 74 |
| 167 | 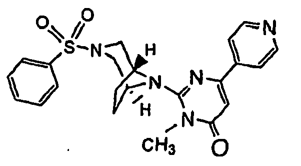 | 3-Methyl-2-[(1R,5S)-8-(phenylsulfonyl)-38-diazabicyclo[3.2.1]oct-3-yl]-6-pyridin-4-ylpyrimidin-4(3H)-one | 438 | 98 |
| 168 | 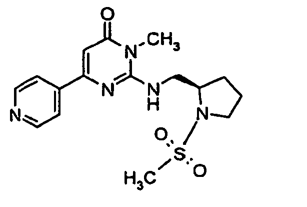 | 3-Methyl-2-({[(2R)-1-(methylsulfonyl)pyrrolidin-2-yl]methyl}amino)-6-pyridin-4-ylpyrimidin-4(3H)-one | 364 | 78 |
| 169 | 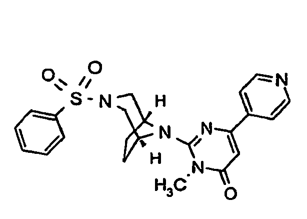 | 3-Methyl-2-[(1R,5S)-3-(phenylsulfonyl)-38-diazabicyclo[3.2.1]oct-8-yl]-6-pyridin-4-ylpyrimidin-4(3H)-one | 438 | 92 |
| 170 | 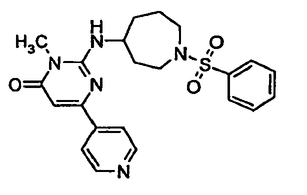 | 3-Methyl-2-{[1-(phenylsulfonyl)azepan-4-yl]amino}-6-pyridin-4-ylpyrimidin-4(3H)-one | 440 | 99 |
| 171 | 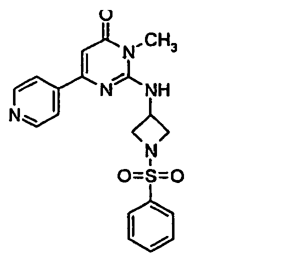 | 3-Methyl-2-{[1-(phenylsulfonyl)azetidin-3-yl]amino}-6-pyridin-4-ylpyrimidin-4(3H)-one | 398 | 61 |
| 172 | 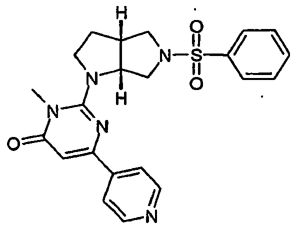 | 3-Methyl-2-[(3a,R6,aR)-5-(phenylsulfonyl)hexahydr opyrrolo[34-b]pyrrol-1(2H)-yl]-6-pyridin-4-ylpyrimidin-4(3H)-one | 438 | 81 |
| 173 | 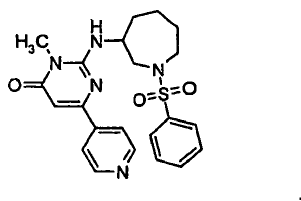 | 3-Methyl-2-{[1-(phenylsulfonyl)azepan-3-yl]amino}-6-pyridin-4-ylpyrimidin-4(3H)-one | 440 | 51 |
| 174 | 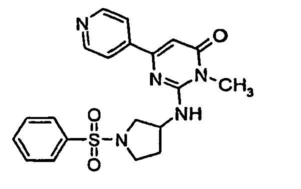 | 3-Methyl-2-{[1-(phenylsulfonyl)pyrrolidin-3-yl]amino}-6-pyridin-4-ylpyrimidin-4(3H)-one | 412 | 95 |
| 175 | 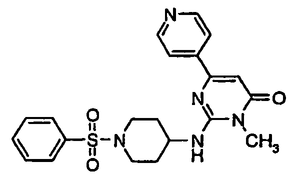 | 3-Methyl-2{[1-(phenylsulfonyl)piperidin-4-yl]amino}-6-pyridin-4-ylpyrimidin-4(3H)-one | 426 | 49 |
| 176 | 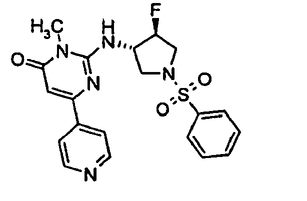 | 2-{[(3S,4S)-4-Fluoro-1-(phenylsulfonyl)pyrrolidin-3-yl]amino}-3-methyl-6-pyridin-4-ylpyrimidin-4(3H)-one | 430 | 96 |
| 177 | 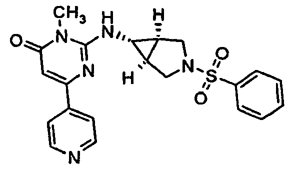 | 3-Methyl-2-{[(1R,5S,6s)-3-(phenylsulfonyl)-3-azabicyclo[3.1.0]hex-6-yl]amino}-6-pyridin-4-ylpyrimidin-4(3H)-one | 424 | 97 |
| 178 | 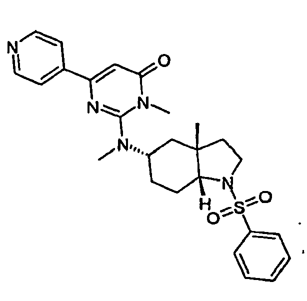 | 3-Methyl-2-{methyl[(3a,S5,S7,aR)-3a-methyl-1-(phenylsulfonyl)octahydro -1H-indol-5-yl]amino}-6-pyridin-4-ylpyrimidin-4(3H)-one | 494 | 100 |
| 179 | 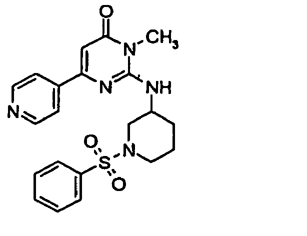 | 3-Methyl-2-{[1-(phenylsulfonyl)piperidin-3-yl]amino}-6-pyridin-4-ylpyrimidin-4(3H)-one | 426 | 93 |
| 180 | 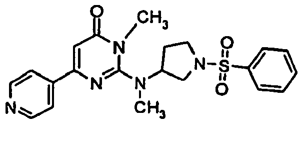 | 3-Methyl-2-{methyl[1-(phenylsulfonyl)pyrrolidin-3-yl]amino}-6-pyridin-4-ylpyrimidin-4(3H)-one | 426 | 93 |
| 181 | 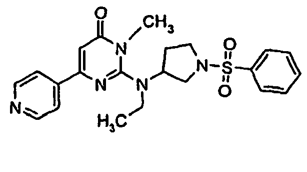 | 3-Methyl-2-{methyl[1-(phenylsulfonyl)azepan-4-yl]amino}-6-pyridin-4-ylpyrimidin-4(3H)-one | 454 | 96 |
| 182 | 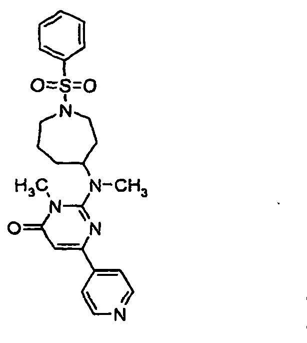 | 3-Methyl-2-{methyl[1-(phenylsulfonyl)azepan-4-yl]amino}-6-pyridin-4-ylpyrimidin-4(3H)-one | 454 | 96 |
| 183 | 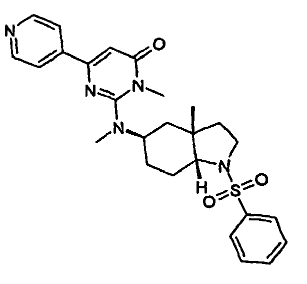 | 3-Methyl-2-{methyl[(3,aS5,R7,aR)-3a-methyl-1-(phenylsulfonyl)octahydro -1H-indol-5-yl]amino}-6-pyridin-4-ylpyrimidin-4(3H)-one | 494 | 100 |
| 184 | 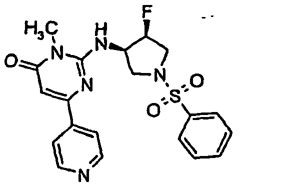 | 2-{[(3R,4S)-4-Fluoro-1-(phenylsulfonyl)pyrrolidin-3-yl]amino}-3-methyl-6-pyridin-4-ylpyrimidin-4(3H)-one | 430 | 100 |
| 185 | 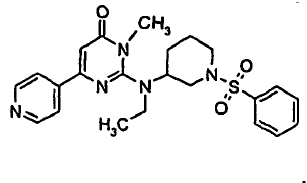 | 2-{Ethyl[1-(phenylsulfonyl)piperidin-3-yl]amino}-3-methyl-6-pyridin-4-ylpyrimidin-4(3H)-one | 454 | 101 |
| 186 | 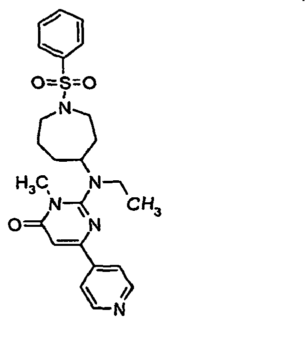 | 2-{Ethyl[1-(phenylsulfonyl)azepan-4-yl]amino}-3-methyl-6-pyridin-4-ylpyrimidin-4(3H)-one | 468 | 101 |
| 187 | 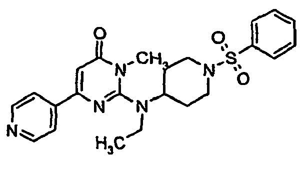 | 2-{Ethyl[1-(phenylsulfonyl)piperidin-4-yl]amino}-3-methyl-6-pyridin-4-ylpyrimidin-4(3H)-one | 454 | 102 |
| 188 | 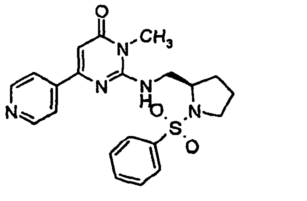 | 3-Methyl-2-({[(2R)-1-(phenylsulfonyl)pyrrolidin-2-yl]methyl}amino)-6-pyridin-4-ylpyrimidin-4(3H)-one | 426 | 94 |
| 189 | 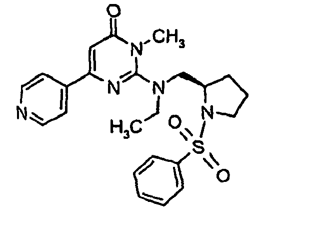 | 2-(Ethyl{[(2R)-1-(phenylsulfonyl)pyrrolidin-2-yl]methyl}amino)-3-methyl-6-pyridin-4-ylpyrimidin-4(3H)-one | 454 | 95 |
| 190 | 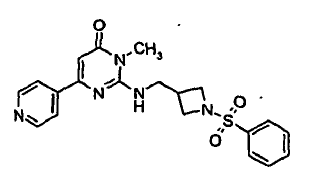 | 3-Methyl-2-({[1-(phenylsulfonyl)azetidin-3-yl]methyl}amino)-6-pyridin-4-ylpyrimidin-4(3H)-one | 412 | 35 |
| 191 | 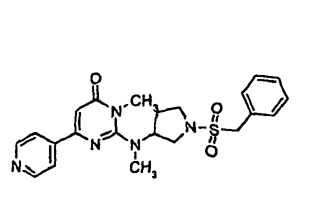 | 3-Methyl-2-({[1-(phenylsulfonyl)pyrrolidin-3-yl]methyl}amino)-6-pyridin-4-ylpyrimidin-4(3H)-one | 426 | 76 |
| 192 | 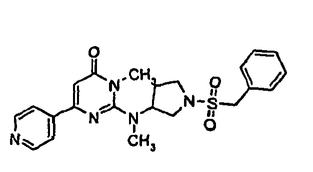 | 2-{[1-(Benzylsulfonyl)azepan-4-yl]amino}-3-methyl-6-pyridin-4-ylpyrimidin-4(3H)-one | 454 | 81 |
| 193 | 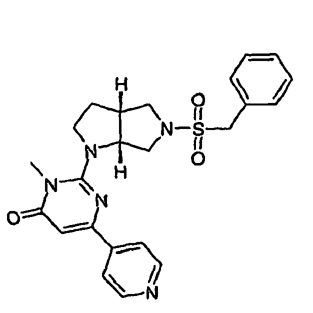 | 2-[(3a,R6,aR)-5-(Benzylsulfonyl)hexahydr opyrrolo[34-b]pyrrol-1(2H)-yl]-3-methyl-6-pyridin-4-ylpyrimidin-4(3H)-one | 452 | 80 |
| 194 | 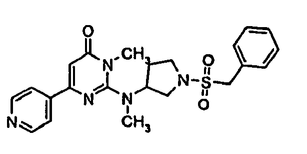 | 2-{[1-(Benzylsulfonyl)pyrrolidin-3-yl](methyl)amino}-3-methyl-6-pyridin-4-ylpyrimidin-4(3H)-one | 440 | 90 |
| 195 | 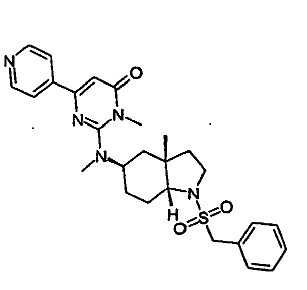 | 2-{[(3,aS5,R7,aR)-1-(Benzylsulfonyl)-3a-methyloctahydro-1H-indol-5-yl](methyl)amino}-3-methyl-6-pyridin-4-ylpyrimidin-4(3H)-one | 508 | 92 |
| 196 | 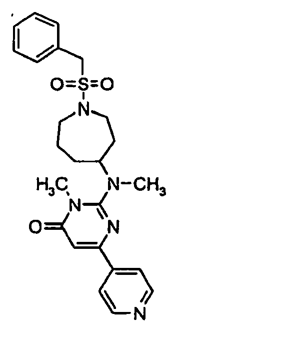 | 2-{[1-(Benzylsulfonyl)azepan-4-yl](methyl)amino}-3-methyl-6-pyridin-4-ylpyrimidin-4(3H)-one | 468 | 94 |
| 197 | 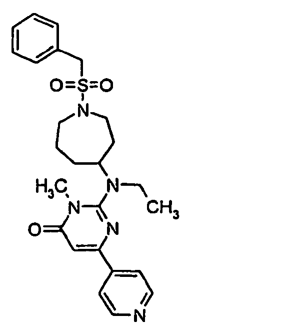 | 2-{[1-(Benzylsulfonyl)azepan-4-yl](ethyl)amino}-3-methyl-6-pyridin-4-ylpyrimidin-4(3H)-one | 344 | 93 |
| 198 | 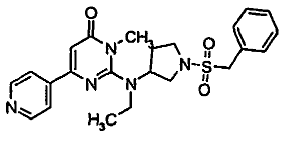 | 2-{[1-(Benzylsulfonyl)pyrrolidin-3-yl](ethyl)amino}-3-methyl-6-pyridin-4-ylpyrimidin-4(3H)-one | 454 | 97 |
| 199 | 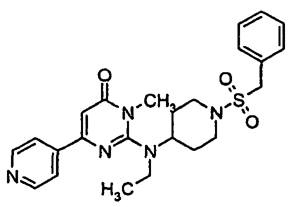 | 2-{[1-(Benzylsulfonyl)piperidin-4-yl](ethyl)amino}-3-methyl-6-pyridin-4-ylpyrimidin-4(3H)-one | 468 | 89 |

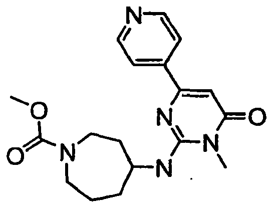

**Example 200: (R)-2-(Ehyl((1-(methylsulfonyl)pyrrolidin-2-yl)methyl)amino)-3-methyl-6-(pyridin-4-yl)pyrimidin-4(3H)-one:** To the product of Example 8 (80 µmol) was added a soln. of TFA (2 ml) in DCE (2 ml) and the mixture was shaken for 4 hr. The solvent was removed to give a crude residue, which was dissolved in DCE (500 ml). TEA (160 µmol) in DCE (0.2 ml) was added followed by methylchloroformate (80 µmol) in DCE (0.2 ml). The reaction was shaken at RT for 16 hr. The crude mixture was evaporated, then dissolved in DMSO and purified by prep. HPLC to give the title compound (3.9 mg). Calc. MW: 357.4, Found: 358 (MH+), Retention time: 1.7min.

### General Procedure for Examples 201 to 221

Examples 201 to 221 were prepared by using the analogous procedure described to prepare Example 200, substituting the appropriate starting material (Examples 1 to 35) and coupling with the appropriate chloroformate.

| **Ex.** | **Structure** | **Name** | **Observed Mass (MH+)** | **Percent inhibition at 1 µM** |
|---|---|---|---|---|
| 201 | 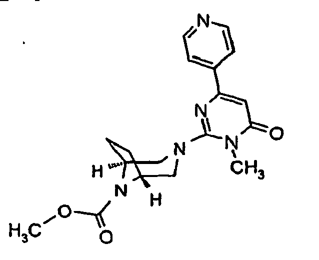 | Methyl (1 R,5S)-3-(1-methyl-6-oxo-4-pyridin-4-yl-16-dihydropyrimidin-2-yl)-38-diazabicyclo[3.2.1]octane-8-carboxylate | 314 | 89 |
| 202 | 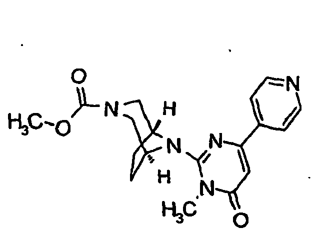 | Methyl (1R,5S)-8-(1-methyl-6-oxo-4-pyridin-4-yl-16-dihydropyrimidin-2-yl)-38-diazabicyclo[3.2.1]octane-3-carboxylate | 314 | 93 |
| 203 | 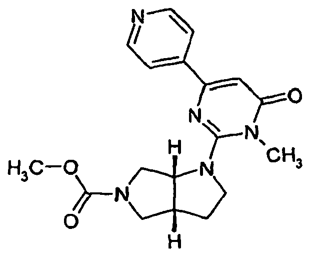 | Methyl (3a,R6,aR)-1-(1-methyl-6-oxo-4-pyridin-4-yl-16-dihydropyrimidin-2-yl)hexahydropyrrolo[34-b]pyrrole-5(1H)-carboxylate | 356 | 32 |
| 204 | 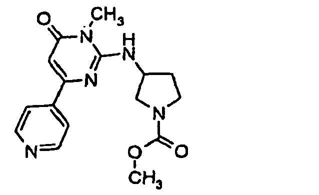 | Methyl 3-[(1-methyl-6-oxo-4-pyridin-4-yl-16-dihydropyrimidin-2-yl)amino]pyrrolidine-1-carboxylate | 330 | 82 |
| 205 | 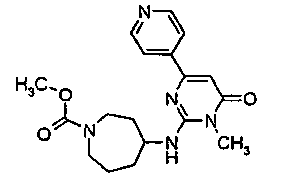 | Methyl 4-[(1-methyl-6-oxo-4-pyridin-4-yl-16-dihydropyrimidin-2-yl)amino]azepane-1-carboxylate | 358 | 86 |
| 206 | 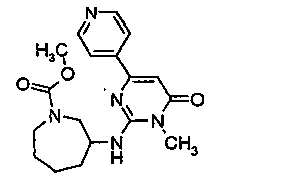 | Methyl 3-[(1-methyl-6-oxo-4-pyridin-4-yl-16-dihydropyrimidin-2-yl)amino]azepane-1-carboxylate | 358 | 94 |
| 207 | 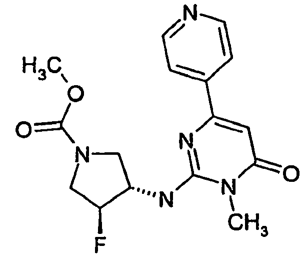 | Methyl (3S,4S)-3-fluoro-4-[(1-methyl-6-oxo-4-pyridin-4-yl-16-dihydropyrimidin-2-yl)amino]pyrrolidine-1-carboxylate | 348 | 74 |
| 208 | 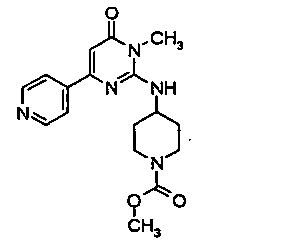 | Methyl 4-[(1-methyl-6-oxo-4-pyridin-4-yl-16-dihydropyrimidin-2-yl)amino]piperidine-1-carboxylate | 344 | 77 |
| 209 | 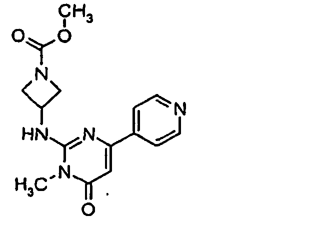 | Methyl 3-[(1-methyl-6-oxo-4-pyridin-4-yl-16-dihydropyrimidin-2-yl)amino]azetidine-1-carboxylate | 356 | 80 |
| 210 | 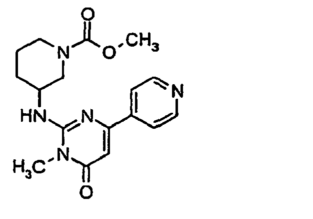 | Methyl 3-[(1-methyl-6-oxo-4-pyridin-4-yl-16-dihydropyrimidin-2-yl)amino]piperidine-1-carboxylate | 344 | 99 |
| 211 | 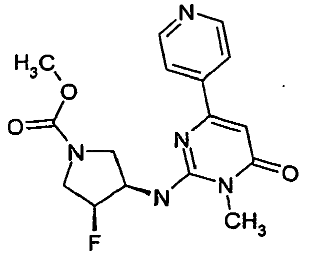 | Methyl (3S,4R)-3-fluoro-4-[(1-methyl-6-oxo-4-pyridin-4-yl-16-dihydropyrimidin-2-yl)amino]pyrrolidine-1-carboxylate | 348 | 67 |
| 212 | 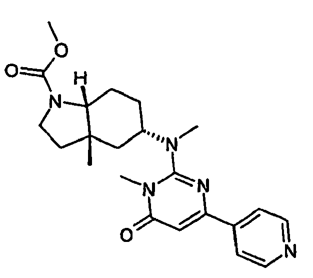 | Methyl (3a,S5,S7,aR)-3a-methyl-5-[methyl(1-methyl-6-oxo-4-pyridin-4-yl-16-dihydropyrimidin-2-yl)amino]octahydro-1H-indole-1-carboxylate | 356 | 93 |
| 213 | 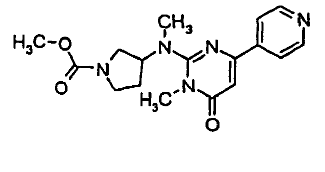 | Methyl 3-[methyl(1-methyl-6-oxo-4-pyridin-4-yl-16-dihydropyrimidin-2-yl)amino]pyrrolidine-1-carboxylate | 344 | 95 |
| 214 | 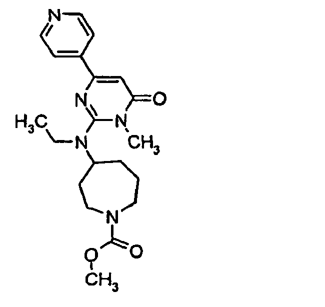 | Methyl 4-[methyl(1-methyl-6-oxo-4-pyridin-4-yl-16-dihydropyrimidin-2-yl)amino]azepane-1-carboxylate | 372 | 99 |
| 215 | 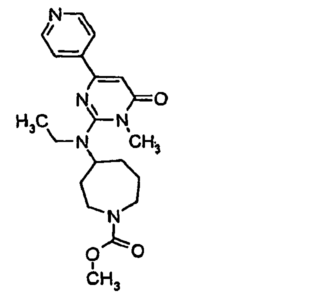 | Methyl 4-[ethyl(1-methyl-6-oxo-4-pyridin-4-yl-16-dihydropyrimidin-2-yl)amino]azepane-1-carboxylate | 386 | 93 |
| 216 | 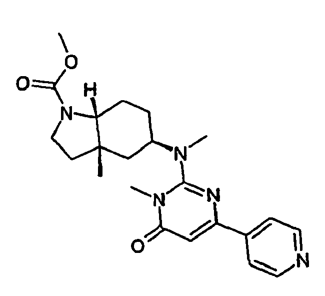 | Methyl (3a,S5,R7,aR)-3a-methyl-5-[methyl(1-methyl-6-oxo-4-pyridin-4-yl-16-dihydropyrimidin-2-yl)amino]octahydro-1H-indole-1-carboxylate | 412 | 88 |
| 217 | 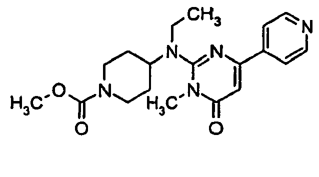 | Methyl 4-[ethyl(1-methyl-6-oxo-4-pyridin-4-yl-16-dihydropyrimidin-2-yl)amino]piperidine-1-carboxylate | 372 | 84 |
| 218 | 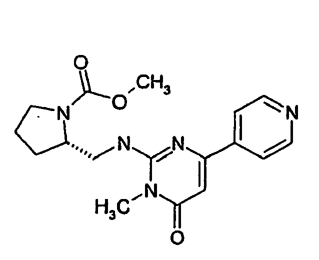 | Methyl (2S)-2-{[(1-methyl-6-oxo-4-pyridin-4-yl-16-dihydropyrimidin-2-yl)amino]methyl}pyrrolidine-1-carboxylate | 344 | 68 |
| 219 | 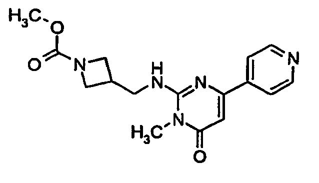 | Methyl 3-{[(1-methyl-6-oxo-4-pyridin-4-yl-16-dihydropyrimidin-2-yl)amino]methyl}azetidine-1-carboxylate | 330 | 72 |
| 220 | 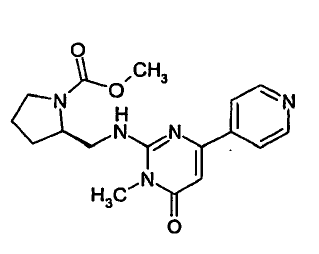 | Methyl (2R)-2-{[(1-methyl-6-oxo-4-pyridin-4-yl-16-dihydropyrimidin-2-yl)amino]methyl}pyrrolidine-1-carboxylate | 344 | 91 |
| 221 | 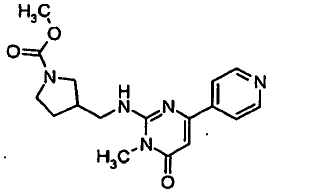 | Methyl 3-{[(1-methyl-6-oxo-4-pyridin-4-yl-16-dihydropyrimidin-2-yl)amino]methyl}pyrrolidine-1-carboxylate | 344 | 71 |

Examples 222 to 234 were prepared by using the analogous procedure described to prepare Example 200, substituting the appropriate starting material (Examples 1 to 35) and coupling with the appropriate chloroformate.

| **Ex.** | **Structure** | **Name** | **Observed Mass (MH+)** | **Enzyme IC₅₀ (nM)** |
|---|---|---|---|---|
| 222 | 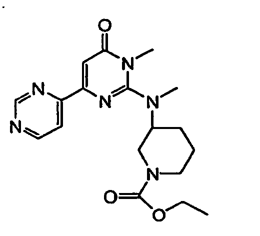 | Ethyl 3-[methyl(1-methyl-6-oxo-1,6-dihydro-4,4'-bipyrimidin-2-yl)amino]piperidine-1-carboxylate | 372 | 1.14 |
| 223 | 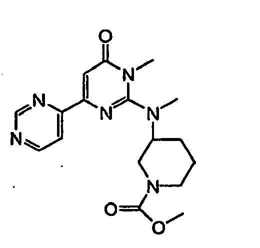 | Methyl 3-[methyl(1-methyl-6-oxo-1,6-dihydro-4,4'-bipyrimidin-2-yl)amino]piperidine-1-carboxylate | 358 | 1.04 |
| 224 | 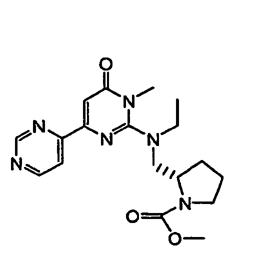 | Methyl (2S)-2-{[ethyl(1-methyl-6-oxo-1,6-dihydro-4,4'-bipyrimidin-2-yl)amino]methyl}pyrrolidine-1-carboxylate | 372 | 1.11 |
| 225 | 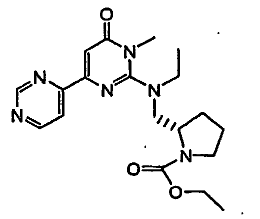 | Ethyl (2S)-2-{[ethyl(1-methyl-6-oxo-1,6-dihydro-4,4'-bipyrimidin-2-yl)amino]methyl}pyrrolidine-1-carboxylate | 386 | 0.755 |
| 226 | 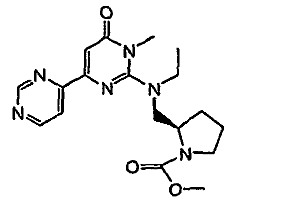 | Methyl (2R)-2-{[ethyl(1-methyl-6-oxo-1,6-dihydro-4,4'-bipyrimidin-2-yl)amino]methyl}pyrrolidine-1-carboxylate | 372 | 1.00 |
| 227 | 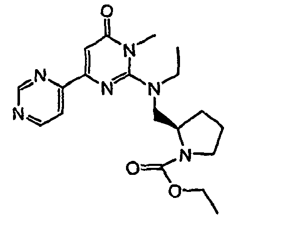 | Ethyl (2R)-2-{[ethyl(1-methyl-6-oxo-1,6-dihydro-4,4'-bipyrimidin-2-yl)amino]methyl}pyrrolidine-1-carboxylate | 386 | 0.825 |
| 228 | 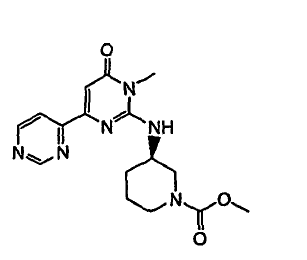 | Methyl (3R)-3-[(1-methyl-6-oxo-1,6-dihydro-4,4'-bipyrimidin-2-yl)amino]piperidine-1-carboxylate | 344 | 0.994 |
| 229 | 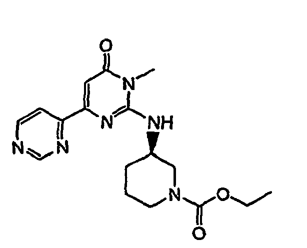 | Ethyl (3R)-3-[(1-methyl-6-oxo-1,6-dihydro-4,4'-bipyrimidin-2-yl)amino]piperidine-1-carboxylate | 359 | 1.99 |
| 230 | 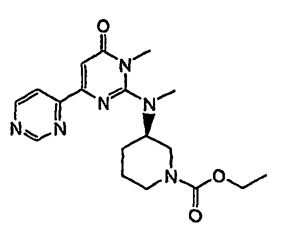 | Ethyl (3R)-3-[methyl(1-methyl-6-oxo-1,6-dihydro-4,4'-bipyrimidin-2-yl)amino]piperidine-1-carboxylate | 373 | 0.95 |
| 231 | 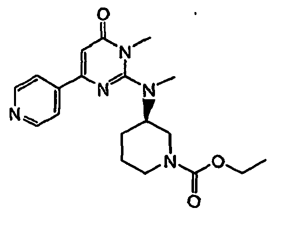 | Ethyl (3R)-3-[methyl(1-methyl-6-oxo-4-pyridin-4-yl-1,6-dihydropyrimidin-2-yl)amino]piperidine-1-carboxylate | 372 | 1.15 |
| 232 | 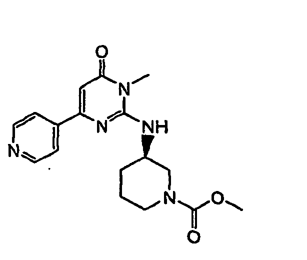 | Methyl (3R)-3-[(1-methyl-6-oxo-4-pyridin-4-yl-1,6-dihydropyrimidin-2-yl)amino]piperidine-1-carboxylate | 344 | 0.854 |
| 233 | 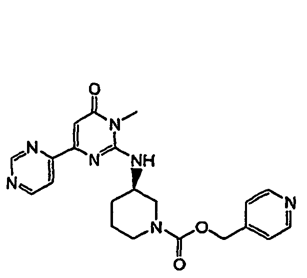 | Pyridin-4-ylmethyl (3R)-3-[(1-methyl-6-oxo-1,6-dihydro-4,4'-bipyrimidin-2-yl)amino]piperidine-1-carboxylate | 422 | 5.00 |
| 234 | 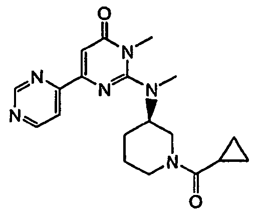 | 2-{[(3R)-1-(Cyclopropylcarbonyl)piperid in-3-yl](methyl)amino}-1-methyl-4,4'-bipyrimidin-6(1H)-one | 369 | 1.68 |

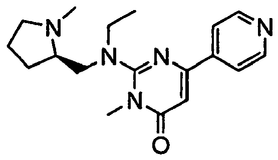

**Example 235: (R)-2-(Ethyl((1-methylpyrrolidin-2-yl)methyl)amino)-3-methyl-6-(pyridin-4-yl)pyrimidin-4(3H)-one:** To the product of Example 4 (80 µmol) was added a soln. of TFA (2 ml) in DCE (2 ml) and the mixture was shaken for 4 hr. The solvent was removed to give a residue, which was dissolved in MeOH (0.5 ml). TEA (160 µmol) in MeOH (0.2 ml) was added, followed by formaldehyde (80 µmol) in MeOH (0.2 ml). Sodium cyanoborohydride (100 umol) was added, dissolved in MeOH (0.5 ml). The reaction was shaken at RT for 16 hr. The mixture was evaporated, dissolved in DMSO, and purified by prep. HPLC to give the title compound (5.0 mg). Calc. MW: 327.4, Found: 328 (MH+), Retention time: 2.13 min.

### General Procedure for Examples 236 to 262

Examples 236 to 262 may be prepared by using the analogous procedure described to prepare Example 235, substituting the appropriate starting material (Examples 1 to 35) and coupling with the appropriate aldehyde.

| **Ex.** | **Structure** | **Name** | **Observed MW** | **Percent inhibition at 1 µM** |
|---|---|---|---|---|
| 236 | 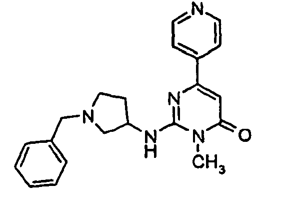 | 2-[(1-Benzylpyrrolidin-3-yl)amino]-3-methyl-6-pyridin-4-ylpyrimidin-4(3H)-one | 362 | 63 |
| 237 | 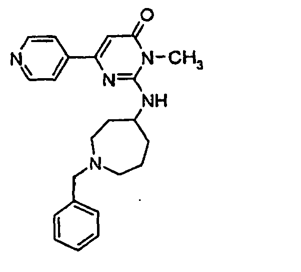 | 2-[(1-Benzylazepan-4-yl)amino]-3-methyl-6-pyridin-4-ylpyrimidin-4(3H)-one | 390 | 84 |
| 238 | 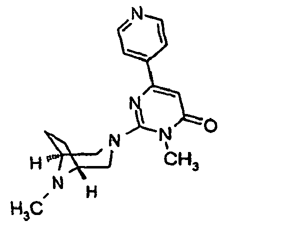 | 3-Methyl-2-[(1R,5S)-8-methyl-38-diazabicyclo[3.2.1]oct -3-yl]-6-pyridin-4-ylpyrimidin-4(3H)-one | 312 | 47 |
| 239 | 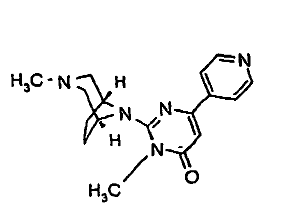 | 3-Methyl-2-[(1R,5S)-3-methyl-38-diazabicyclo[3.2.1]oct -8-yl]-6-pyridin-4-ylpyrimidin-4(3H)-one | 312 | 57 |
| 240 | 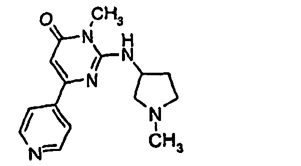 | 3-Methyl-2-[(1-methylpyrrolidin-3-yl)amino]-6-pyridin-4-ylpyrimidin-4(3H)-one | 286 | 49 |
| 241 | 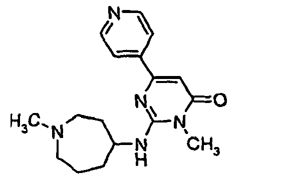 | 3-Methyl-2-[(1-methylazepan-4-yl)amino]-6-pyridin-4-ylpyrimidin-4(3H)-one | 314 | 72 |
| 242 | 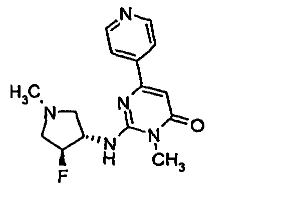 | 2-{[(3S,4S)-4-Fluoro-1-methylpyrrolidin-3-yl]amino}-3-methyl-6-pyridin-4-ylpyrimidin-4(3H)-one | 304 | 43 |
| 243 | 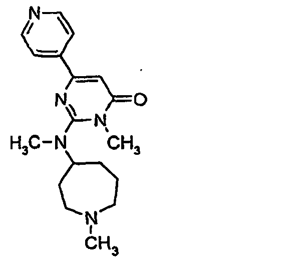 | 3-Methyl-2-[methyl(1-methylazepan-4-yl)amino]-6-pyridin-4-ylpyrimidin-4(3H)-one | 328 | 60 |
| 244 | 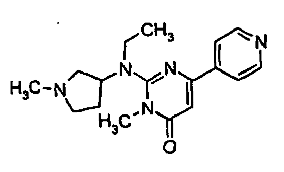 | 2-[ethyl(1-Methylpyrrolidin-3-yl)amino]-3-methyl-6-pyridin-4-ylpyrimidin-4(3H)-one | 314 | 60 |
| 245 | 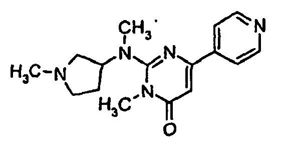 | 3-Methyl-2-[methyl(1-methylpyrrolidin-3-yl)amino]-6-pyridin-4-ylpyrimidin-4(3H)-one | 300 | 52 |
| 246 | 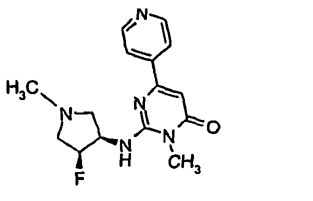 | 2-{[(3R,4S)-4-Fluoro-1-methylpyrrolidin-3-yl]amino}-3-methyl-6-pyridin-4-ylpyrimidin-4(3H)-one | 304 | 67 |
| 247 | 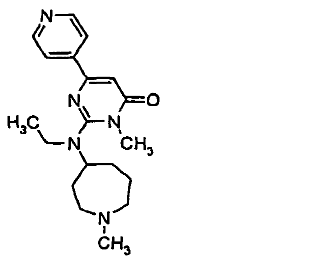 | 2-[Ethyl(1-methylazepan-4-yl)amino]-3-methyl-6-pyridin-4-ylpyrimidin-4(3H)-one | 342 | 78 |
| 248 | 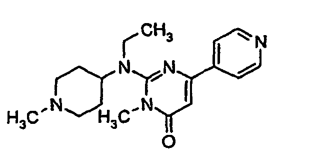 | 2-[Ethyl(1-methylpiperidin-4-yl)amino]-3-methyl-6-pyridin-4-ylpyrimidin-4(3H)-one | 328 | 39 |
| 249 | 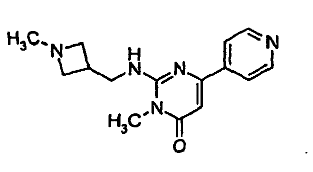 | 3-Methyl-2-{[(1-methylazetidin-3-yl)methyl]amino}-6-pyridin-4-ylpyrimidin-4(3H)-one | 286 | 33 |
| 250 | 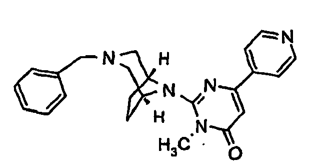 | 2-[(1R,5S)-3-Benzyl-38-diazabicyclo[3.2.1]oct -8-yl]-3-methyl-6-pyridin-4-ylpyrimidin-4(3H)-one | 300 | 69 |
| 251 | 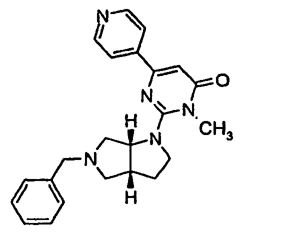 | 2-[(3a,R6,aR)-5-Benzylhexahydropyrr olo[34-b]pyrrol-1(2H)-yl]-3-methyl-6-pyridin-4-ylpyrimidin-4(3H)-one | 388 | 47 |
| 252 | 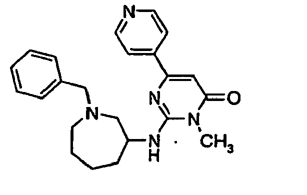 | 2-[(1-Benzylazepan-3-yl)amino]-3-methyl-6-pyridin-4-ylpyrimidin-4(3H)-one | 390 | 74 |
| 253 | 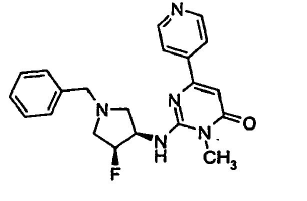 | 2-{[(3R,4S)-1-Benzyl-4-fluoropyrrolidin-3-yl]amino}-3-methyl-6-pyridin-4-ylpyrimidin-4(3H)-one | 380 | 91 |
| 254 | 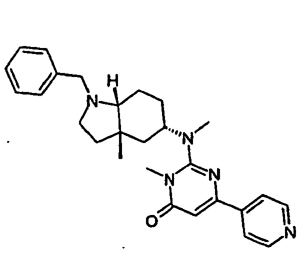 | 2-{[(3a,S5,S7,aR)-1-Benzyl-3a-methyloctahydro-1H-indol-5-yl](methyl)amino}-3-methyl-6-pyridin-4-ylpyrimidin-4(3H)-one | 444 | 47 |
| 255 | 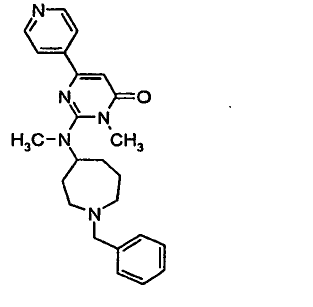 | 2-[(1-Benzylazepan-4-yl)(methyl)amino]-3-methyl-6-pyridin-4-ylpyrimidin-4(3H)-one | 404 | 76 |
| 256 | 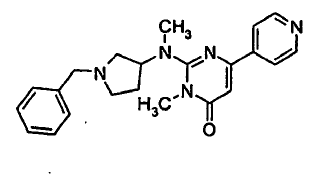 | 2-[(1-Benzylpyrrolidin-3-yl)(methyl)amino]-3-methyl-6-pyridin-4-ylpyrimidin-4(3H)-one | 376 | 83 |
| 257 | 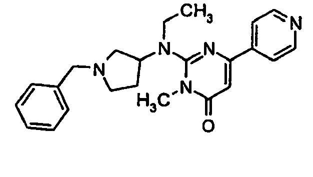 | 2-[(1-Benzylpyrrolidin-3-yl)(ethyl)amino]-3-methyl-6-pyridin-4-ylpyrimidin-4(3H)-one | 390 | 71 |
| 258 | 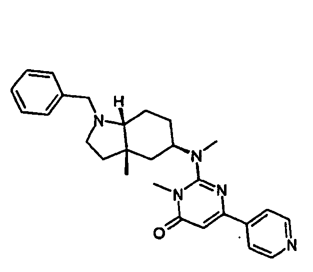 | 2-{[(3a,S5,R7,aR)-1-Benzyl-3a-methyloctahydro-1H-indol-5-yl](methyl)amino}-3-methyl-6-pyridin-4-ylpyrimidin-4(3H)-one | 444 | 43 |
| 259 | 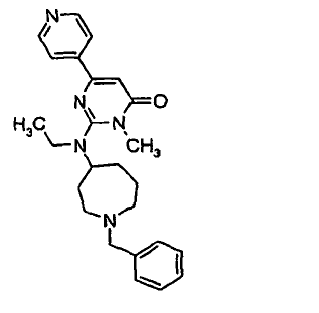 | 2-[(1-Benzylazepan-4-yl)(ethyl)amino]-3-methyl-6-pyridin-4-ylpyrimidin-4(3H)-one | 418 | 83 |
| 260 | 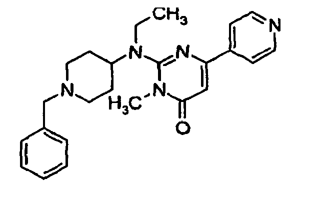 | 2-[(1-Benzylpiperidin-4-yl)(ethyl)amino]-3-methyl-6-pyridin-4-ylpyrimidin-4(3H)-one | 404 | 44 |
| 261 | 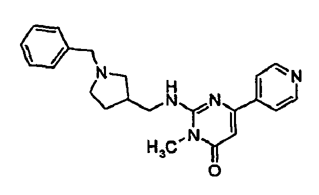 | 2-{[(1-Benzylpyrrolidin-3-yl)methyl]amino}-3-methyl-6-pyridin-4-ylpyrimidin-4(3H)-one | 376 | 70 |
| 262 | 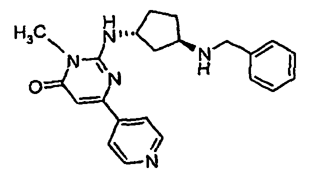 | 2-{[(1R,3R)-3-(Benzylamino)cyclop entyl]amino}-3-methyl-6-pyridin-4-ylpyrimidin-4(3H)-one | 376 | 48 |

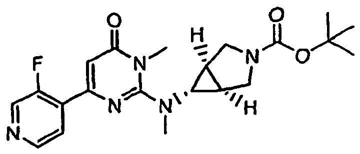

**Example 263: (1S,5R 6s)-tert-butyl 6-((4-(3-Fluoropyridin-4-yl)-1-methyl-6-oxo-1,6-dihydropyrimidin-2-yl)(methyl)amino)-3-aza-bicyclo[3.1.0]hexane-3-carboxylate:** To a solution of (1S,5R,6s)-*tert*-butyl-6-(4-(3-fluoropyridin-4-yl)-1-methyl-6-oxo-1,6-dihydropyrimidin-2-ylamino)-3-aza-bicyclo[3.1.0]hexane-3-carboxylate (1.2 g, 3 mmol) in DMF (15 ml) was added 60% NaH (956 mg, 6 mmol) and the mixture was stirred at RT for 10 min. To the mixture was added a solution of 0.4 ml Mel in 2 ml DMF and the mixture was stirred at room temperature for 2 hr. The mixture was partitioned between EtOAc and water and the organic layer was separated and dried over magnesium sulfate. The residue was purified by column chromatography eluting with a gradient of 5-100% EtOAc in hexanes to give the title compound as a white solid (864 mg, 72%).

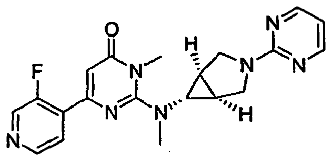

**Example 264: 6-(3-Fluoropyridin-4-yl)-3-methyl-2-(methyl((1S,5R,6s)-3-(pyrimidin-2-yl)-3-aza-bicyclo[3.1.0]hexan-6-yl)amino)pyrimidin-4(3H)-one**: To the product of Example 274 was added a solution of TFA (5 ml) in DCM (5 ml) and the mixture was shaken for 1 hr. The solvents were evaporated to give a crude residue (35 mg, 85 µmol), which was dissolved in DMF (1 ml) followed by addition of TEA (90 µl, 510 µmol), then 2-chloropyrimidine (19 mg, 166 µmol). The reaction was carried out at 170°C in Biotage Microwave Reactor for 10 min. The reaction was partitioned between EtOAc and water and the organic layer was separated and dried over magnesium sulfate. The residue was purified by column chromatography using a gradient of 100% EtOAc to 10% MeOH in EtOAc as eluting solvent to give the title compound (5 mg, 11%). ¹H-NMR(CDCl₃): δ ppm 8.48 (d, 1H), 8.46 (d, 1H), 8.28 (d, 2H), 7.95 (t, 1H), 6.77 (s, 1H), 6.53 (t, 1H), 3.94 (d, 2H), 3.60 (d, 2H), 3.49 (s, 3H), 3.01 (s, 3H), 2.68 (t, 1H), 1.88 (m, 1H); LCMS 394.3 (M+H).

### General Procedure for Examples 265 to 291

Examples 265 to 291 were prepared by using the analogous procedure described to prepare Examples 263 and 264, substituting the appropriate starting material and coupling with the appropriate reagent.

| **Ex.** | **Structure** | **Name** | **Observed MW** | **Enzyme IC₅₀ (nM)** |
|---|---|---|---|---|
| 265 | 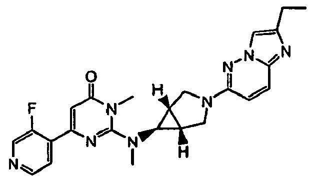 | 2-{[(1R,5S,6s)-3-(2-Ethylimidazo[1,2-b]pyridazin-6-yl)-3-azabicyclo[3.1.0]hex-6-yl](methyl)amino}-6-(3-fluoropyridin-4-yl)-3-methylpyrimidin-4(3H)-one | 460 | 52.4 |
| 266 | 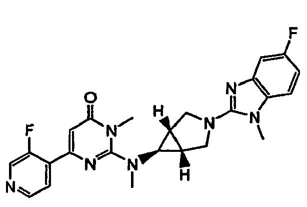 | 2-{[(1R,5S,6s)-3-(5-Fluoro-1-methyl-1H-benzimidazol-2-yl)-3-azabicyclo[3.1.0]hex-6-yl](methyl)amino}-6-(3-fluoropyridin-4-yl)-3-methylpyrimidin-4(3H)-one | 463 | 16.7 |
| 267 | 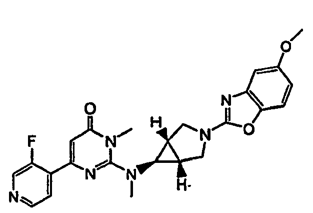 | 6-(3-Fluoropyridin-4-yl)-2-{[1R,5S,6s)-3-(5-methoxy-1,3-benzoxazol-2-yl)-3-azabicyclo[3.1.0]hex-6-yl](methyl)amino}-3-methylpyrimidin-4(3H)-one | 462 | 14.2 |
| 268 | 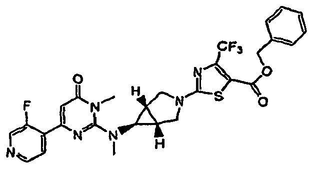 | Benzyl 2-[(1R,5S,6s)-6-{[4-(3-fluoropyridin-4-yl)-1-methyl-6-oxo-1,6-dihydropyrimidin-2-yl](methyl)amino)-3-azabicyclo[3.1.0]hex-3-yl]-4-(trifluoromethyl)-1,3-thiazole-5-carboxylate | 600 | 126 |
| 269 | 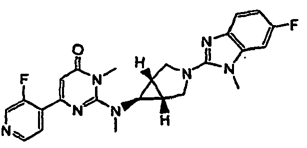 | 2-([(1R,5S,6s)-3-(6-Fluoro-1-methyl-1H-benzimidazol-2-yl)-3-azabicyclo[3.1.0]hex-6-yl](methyl)amino}-6-(3-fluoropyridin-4-yl)-3-methylpyrimidin-4(3H)-one | 463 | 18.8 |
| 270 | 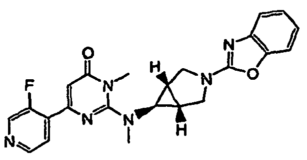 | 2-{[(1R,5S,6s)-3-(1,3-Benzoxazol-2-yl)-3-azabicyclo[3.1.0]hex-6-yl](methyl)amino}-6-(3-fluoropyridin-4-yl)-3-methylpyrimidin-4(3H)-one | 432 | 24.6 |
| 271 | 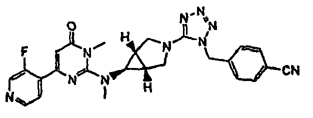 | 4-({5-[(1R,5S,6s)-6-{[4-(3-Fluoropyridin-4-yl)-1-methyl-6-oxo-1,6-dihydropyrimidin-2-yl](methyl)amino}-3-azabicyclo[3.1.0]hex-3-yl]-1H-tetrazol-1-yl}methyl)benzonitrile | 499 | 8.8 |
| 272 | 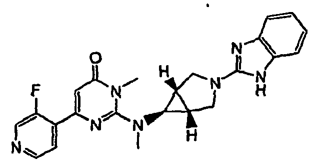 | 2-{[(1R,5S,6s)-3-(1H-Benzimidazol-2-yl)-3-azabicyclo[3.1.0]hex-6-yl](methyl)amino}-6-(3-fluoropyridin-4-yl)-3-methylpyrimidin-4(3H)-one | 431 | 76.1 |
| 273 | 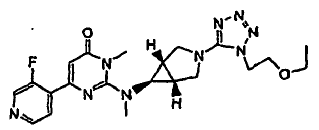 | 2-({(1R,5S,6s)-3-(1-(2-Ethoxyethyl)-1H-tetrazol-5-yl]-3-azabicyclo[3.1.0]hex-6-yl}(methyl)amino]-6-(3-fluoropyridin-4-yl)-3-methylpyrimidin-4(3H)-one | 455 | 29.0 |
| 274 | 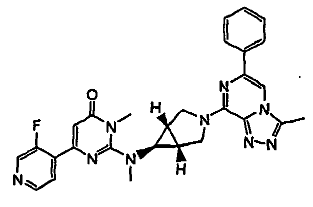 | 6-(3-Fluoropyridin-4-yl)-3-methyl-2-{methyl[(1R,5S,6s)-3-(3-methyl-6-phenyl[1,2,4]triazolo[ 4,3-a]pyrazin-8-yl)-3-azabicyclo[3.1.0]hex-6-yl]amino}pyrimidin-4(3H)-one | 524 | 234 |
| 275 | 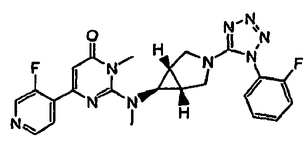 | 2-[{(1R,5S,6s)-3-[1-(2-Fluorophenyl)-1H-tetrazol-5-yl]-3-azabicyclo[3.1.0]hex-6-yl}(methyl)amino]-6-(3-fluoropyrid in-4-yl)-3-methylpyrimidin-4(3H)-one | 477 | 1.12 |
| 276 | 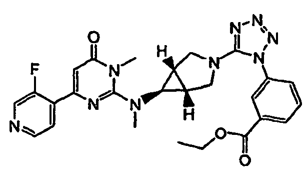 | Ethyl 3-{5-[(1R,5S,6s)-6-{[4-(3-fluoropyridin-4-yl)-1-methyl-6-oxo-1,6-dihydropyrimidin-2-yl](methyl)amino}-3-azabicyclo[3.1.0]hex-3-yl]-1H-tetrazol-1-yl}benzoate | 532 | 6.21 |
| 277 | 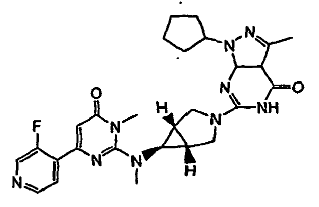 | 1-Cyclopentyl-6-[(1R,5S,6s)-6-{[4-(3-fluoropyridin-4-yl)-1-methyl-6-oxo-1,6-dihydropyrimidin-2-yl](methyl)amino}-3-azabicyclo[3.1.0]hex-3-yl]-3-methyl-1,5-dihydro-4H-pyrazolo[3,4-d]pyrimidin-4-one | 532 | 51.7 |
| 278 | 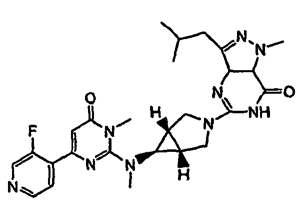 | 5-[(1R,5S,6s)-6-{[4-(3-Fluoropyridin-4-yl)-1-methyl-6-oxo-1,6-dihydropyrimidin-2-yl](methyl)amino}-3-azabicyclo[3.1.0]hex-3-yl]-3-isobutyl-1-methyl-1,6-dihydro-7H-pyrazolo[4,3-d]pyrimidin-7-one | 520 | 109 |
| 279 | 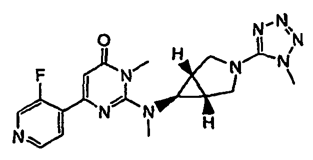 | 6-(3-Fluoropyridin-4-yl)-3-methyl-2-(methyl[(1R,5S,6s)-3-(1-methyl-1H-tetrazol-5-yl)-3-azabicyclo[3.1.0]hex-6-yl]amino}pyrimidin-4(3H)-one | 397 | 58.6 |
| 280 | 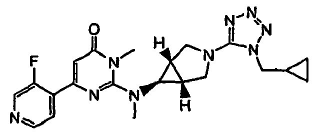 | 2-[{(1R,5S,6s}-3-[1-(Cyclopropylmethyl)-1H-tetrazol-5-yl]-3-azabicyclo[3.1.0]hex-6-yl}(methyl)amino]-6-(3-fluoropyridin-4-yl)-3-methylpyrimidin-4(3H)-one | 437 | 4.48 |
| 281 | 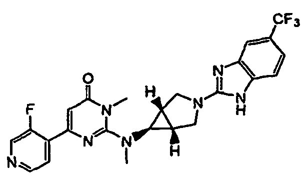 | 6-(3-Fluoropyridin-4-yl)-3-methyl-2-(methyl{(1R,5S,6s)-3-[5-(trifluoromethyl)-1H-benzimidazol-2-yl]-3-azabicyclo[3.1.0]hex-6-yl}amino)pyrimidin-4(3H)-one | 499 | 36.3 |
| 282 | 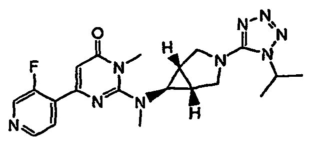 | 6-(3-Fluoropyridin-4-yl)-2-{[(1R,5S,6s)-3-(1-isopropyl-1H-tetrazol-5-yl)-3-azabicyclo[3.1.0]hex-6-yl](methyl)amino}-3-methylpyrimidin-4(3H)-one | 426 | 3.25 |
| 283 | 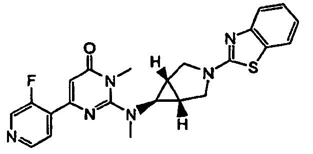 | 2-{[(1R,5S,6s)-3-(1,3-Benzothiazol-2-yl)-3-azabicyclo[3.1.0]hex-6-yl](methyl)amino}-6-(3-fluoropyridin-4-yl)-3-methylpyrimidin-4(3H)-one | 448 | 55.7 |
| 284 | 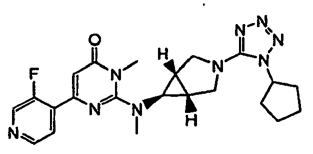 | 2-([(1R,5S,6s)-3-(1-Cyclopentyl-1H-tetrazol-5-yl)-3-azabicyclo[3.1.0]hex-6-yl](methyl)amino}-6-(3-fluoropyridin-4-yl)-3-methylpyrimidin-4(3H)-one | 451 | 12.5 |
| 285 | 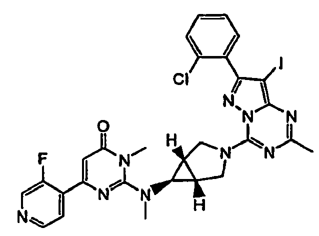 | 2-[{(1R,5S,6s)-3-[7-(2-Chlorophenyl)-8-iodo-2-methylpyrazolo[1,5-a][1,3,5]triazin-4-yl]-3-azabicyclo[3.1.0]hex-6-yl)(methyl)amino]-6-(3-fluoropyridin-4-yl)-3-methylpyrimidin-4(3H)-one | 683 | 5666 |
| 286 | 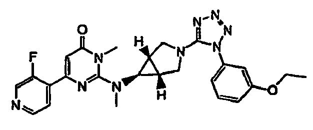 | 2-[{(1R,5S,6s)-3-[1-(3-Ethoxyphenyl)-1H-tetrazol-5-yl]-3-azabicyclo[3.1.0]hex-6-yl}(methyl)amino]-6-(3-fluoropyridin-4-yl)-3-methylpyrimidin-4(3H)-one | 504 | 3.20 |
| 287 | 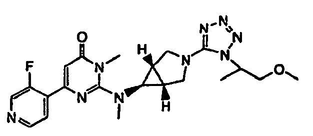 | 6-(3-Fluoropyridin-4-yl)-2-[{(1R,5S,6s)-3-[1-(2-methoxy-1-methylethyl)-1H-tetrazol-5-yl]-3-azabicyclo[3.1.0]hex-6-yl}(methyl)amino]-3-methylpyrimidin-4(3H)-one | 455 | 3.06 |
| 288 | 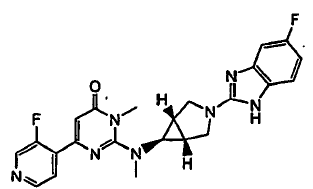 | 2-([(1R,5S,6s)-3-(5-Fluoro-1H-benzimidazol-2-yl)-3-azabicyclo[3.1.0]hex-6-yl](methyl)amino}-6-(3-fluoropyridin-4-yl)-3-methylpyrimidin-4(3H)-one | 449 | 116 |
| 289 | 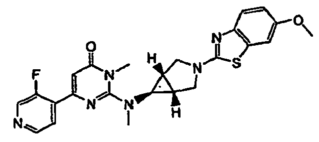 | 6-(3-Fluoropyridin-4-yl)-2-([(1R,5S,6s)-3-(6-methoxy-1,3-benzothiazol-2-yl)-3-azabicyclo[3.1.0]hex-6-yl](methyl)amino}-3-methylpyrimidin-4(3H)-one | 478 | 25.1 |
| 290 | 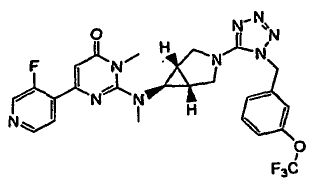 | 6-(3-Fluoropyridin-4-yl)-3-methyl-2-{methyl[(1R,5S,6s)-3-{1-[3-(trifluoromethoxy)ben zyl]-1H-tetrazol-5-yl}-3-azabicyclo[3.1.0]hex-6-yl]amino}pyrimidin-4(3H)-one | 558 | 27.7 |
| 291 | 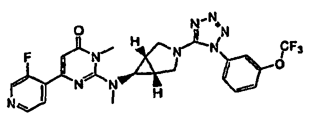 | 6-(3-Fluoropyridin-4-yl)-3-methyl-2-{methyl[(1 R,5S,6s)-3-{1-[3-(trifluoromethoxy)phe nyl]-1H-tetrazol-5-yl}-3-azabicyclo[3.1.0]hex-6-yl]amino}pyrimidin-4(3H)-one | 543 | 5.99 |

## Claims

1. A compound of Formula I or Formula II, 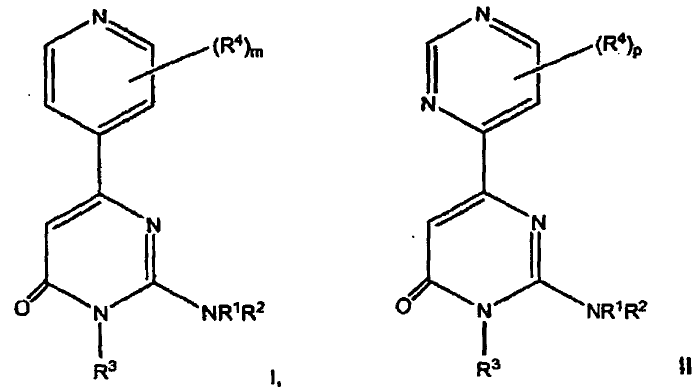 or a pharmaceutically acceptable salt, or solvate thereof, wherein:
R¹ is hydrogen or a C₁-C₆ alkyl group;
R² is a -(4-15 membered) heterocycloalkyl, optionally substituted by one or more substituents selected from the group R⁷;
wherein R³ is hydrogen or C₁-C₆ alkyl;
wherein R⁴ is halogen, C₁-C₆ alkyl, C₁-C₆ haloalkyl, C₁-C₆ alkoxy or C₁-C₆ haloalkoxy; wherein each R⁷ is independently selected from -OH, halogen, -C₁-C₆ alkyl, -C₂-C₆ alkenyl, -C₂-C₆ alkynyl, -C₁-C₆ alkoxy, -C₂-C₆ alkenoxy, -C₂-C₆ alkynoxy, -C₁-C₆ hydroxyalkyl, -CN, -NO₂, -NR⁸R⁹, -C(=O)N⁸R⁹, -C(=O)R⁸, -O(=O)OR⁸, -S(O)₂NR⁸R⁹, -S(O)ₙR⁸, -NR⁹C(=O)R⁸, -NR⁹SO₂R⁸, -(C_{zero}-C₆ alkylene)-C₆-C₁₅ aryl, -(C_{zero}-C₆ alkylene)-(5-15 membered) heterocycloalkyl, -(C_{zero}-C₆ alkylene)-(5-15 membered) heteroaryl, -(C_{zero}-C₆ alkylone)-C₆-C₁₅ aryloxy and -(C_{zero}-C₆ alkylene)-(5-15 membered) heteroaryloxy, wherein said alkyl, alkenyl, alkynyl, alkoxy, alkenoxy, alkynoxy, hydroxyalkyl, aryl, aryloxy, heteroaryl and heteroaryloxy of R⁷ are each optionally independently substituted with one or more subsitutents selected from halogens, -C₁-C₁₂ alkyl, -C₁-C₄ alkoxy, -NR⁸R⁹, -C(=O)N⁸R⁹, -C(=O)R⁸, -C(=O)OR⁸, -NR⁹C(=O)R⁸, -NR⁹SO₂R⁸, -S(O)₂NR⁸R⁹, -S(O)ₙR⁸ or -ON;
each R⁸ and R⁹ are independently selected from -H, -C₁-C₁₅ alkyl, -C₂-C₁₅ alkenyl,-C₂-C₁₅ alkynyl, -(C_{zero}-C₄ alkylene)-(C₃-C₁₅ cycloalkyl), -(C_{zero}-C₄ alkylene)-(C₄-C₆ cycloalkenyl), -(C_{zero}-C₄ alkylene)-((5-15 membered) heterocycloalkyl -(C_{zero}-C₄ alkylene)-(C₆-C₁₅ aryl) and -(C_{zero}-C₄ alkylene)-((5-15 membered) heteroaryl), wherein said alkyl, alkenyl, alkynyl, cycloalkyl, cycloalkenyl, heterocycloalkyl, aryl and heteroaryl of R⁸ and R⁹ are each optionally independently substituted with one or more substituents independently selected from -OH, -C₁-C₁₂ alkyl, -C₂-C₁₂ alkenyl, -C₂-C₁₂ alkynyl, C₁-C₆ alkoxy, -C₂-C₆ alkenoxy, -C₂-C₆ alkynoxy, -C₁-C₆ hydroxyalkyl, halogen, -CN, -NO₂, -CF₃, -NH₂, -NH(C₁₋C₆ alkyl), -N(C₁-C₆ alkyl)₂, -C(=O)NH₂, -C(=O)NH(C₁-C₆ alkyl), -C(=O)N(C₁-C₆ alkyl)₂, -SO₂NH₂, -SO₂NH(C₁-C₆ alkyl), -SO₂N(C₁-C₆ alkyl)₂, -C(=O)H, -C(=O)OH and -C(=O)O(C₁-C₆ alkyl);
n is 0, 1 or 2; m is 0, 1, 2, 3 or 4, and p is 0, 1, 2 or 3.

2. The compound, salt or solvate of claim 1, wherein R² is a -(5-15 membered) heterocycloalkyl.

3. The compound of claim 1 or a pharmaceutically acceptable salt, or solvate thereof, wherein R² is a -(5-15 membered) heterocycloalkyl substituted by R⁷; wherein R⁷ is -C(=O)R⁸b -C(=O)OR⁸ or -S(O)ₙR⁸, and R⁸ is -(C_{zero}-C₆ alkylene)-C₆-C₁₅ aryl.

4. The compound of any of claims 1 to 3 or a pharmaceutically acceptable salt, or solvate thereof, which is a compound of Formula 1.

5. The compound of any of claims 1 to 3 or a pharmaceutically acceptable salt, or solvate thereof, which is a compound of Formula II.

6. A pharmaceutical composition comprising an amount of a compound of any of claims 1 to 5 or a pharmaceutically acceptable salt, or solvate thereof, and a pharmaceutically acceptable carrier, vehicle or diluent.

7. A compound as defined in any of claims 1 to 5 or a pharmaceutically acceptable salt, or solvate thereof, for use as a medicament.

8. A compound as defined in any of claims 1 to 5 or a pharmaceutically acceptable salt or solvate thereof, for treating a disorder selected from: Alzheimer's Disease, cancer, diabetes, Syndrome X, obesity, hair loss, inflammation, mood disorders, neuronal cell death, stroke, bipolar disorder, conditions arising from loss of muscle mass and function, frailty, and cardio-protection.

## Patentansprüche

1. Verbindung der Formel I oder der Formel II 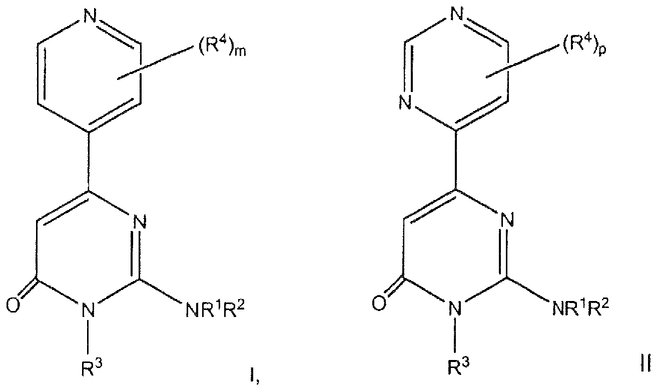 oder ein pharmazeutisch verträgliches Salz oder Solvat davon, worin:
R¹ Wasserstoff oder eine C₁-C₆-Alkylgruppe ist;
R² ein -(4-15-gliedriges) Heterocycloalkyl ist, das gegebenenfalls mit einem oder mehreren Substituenten, ausgewählt aus der Gruppe R⁷, substituiert ist;
worin R³ Wasserstoff oder C₁-C₆-Alkyl ist;
worin R⁴ Halogen, C₁-C₆-Alkyl, C₁-C₆-Halogenalkyl, C₁-C₆-Alkoxy oder C₁-C₆-Halogenalkoxy ist;
worin jedes R⁷ unabhängig ausgewählt ist aus -OH, Halogen, -C₁-C₆-Alkyl, -C₂-C₆-Alkenyl, -C₂-C₆-Alkinyl, -C₁-C₆-Alkoxy, -C₂-C₆-Alkenoxy, -C₂-C₆-Alkinoxy, -C₁-C₆-Hydroxyalkyl, -CN, -NO₂, -NR⁸R⁹, -C(=O)N⁸R⁹, -C(=O)R⁸, -C(O)OR⁸, -S(O)₂NR⁸R⁹, -S(O)ₙR⁸, -NR⁹C(=O)R⁸, -NR⁹SO₂R⁸, - (C_{Null}-C₆-Alkylen) -C₆-C₁₅-aryl, -(C_{Null}-C₆-Alkylen)-(5-15-gliedriges) heterocycloalkyl, -(C_{Null}-C₆-Alkylen)-(5-15-gliedriges) heteroaryl, -C(_{Null}-C₆-Alkylen)-C₆-C₁₅-aryloxy und -(C_{Null}-C₆-Alkylen)-(5-15-gliedriges) heteroaryloxy, wobei das Alkyl, Alkenyl, Alkinyl, Alkoxy, Alkenoxy, Alkinoxy, Hydroxyalkyl, Aryl, Aryloxy, Heteroaryl und Heteroaryloxy von R⁷ jeweils gegebenenfalls unabhängig substituiert ist mit einem oder mehreren Substituenten, ausgewählt aus Halogenen, -C₁-C₁₂-Alkyl, -C₁-C₄-Alkoxy, -NR⁸R⁹, -C(=O)N⁸R⁹, -C(=O)R⁸, -C(=O)OR⁸, -NR⁹C (=O)R⁸, -NR⁹SO₂R⁸, -S (O)₂NR⁸R⁹, -S(O)ₙR⁸ und -OH;
jedes R⁸ und R⁹ unabhängig ausgewählt ist aus -H, -C₁-C₁₅-Alkyl, -C₂-C₁₅-Alkenyl, -C₂-C₁₅-Alkinyl, -(C_{Null}-C₄-Alkylen)-(C₃-C₁₅-cycloalkyl), -(C_{Null}-C₄-Alkylen)-(C₄-C₈-cycloalkenyl) , - (C_{Null}C₄-Alkylen)-((5-15-gliedriges) heterocycloalkyl), - (C_{Null}-C₄-Alkylen) - (C₆-C₁₅-aryl) und -(C_{Null}-C₄Alkylen)-((5-15-gliedriges) heteroaryl), wobei das Alkyl, Alkenyl, Alkinyl, Cycloalkyl, Cycloalkenyl, Heterocycloalkyl, Aryl und Heteroaryl von R⁸ und R⁹ jeweils gegebenenfalls unabhängig substituiert ist mit einem oder mehreren Substituenten, unabhängig ausgewählt aus -OH, -C₁-C₁₂-Alkyl, -C₂-C₁₂-Alkenyl, -C₂-C₁₂-Alkinyl, C₁-C₆-Alkoxy, -C₂-C₆-Alkenoxy, -C₂-C₆-Alkinoxy, -C₁-C₆-Hydroxyalkyl, Halogen, -CN, -NO₂, -CF₃, -NH₂, -NH(C₁-C₆-Alkyl), -N (Cₗ-C_{E}-Alkyl) ₂, -C(=O) NH₂, -C(=O)NH(C₁-C₆-Alkyl), -C (=O)N (C₁-C₆-Alkyl) ₂, -SO₂NH₂, -SO₂NH (C₁-C₆-Alkyl), -SO₂N (C₁-C₆-Alkyl)₂, -C(=O)H, -C(=O)OH und -C(=O)O(C₁-C₆-Alkyl);
n 0, 1 oder 2 ist; m 0, 1, 2, 3 oder 4 ist und p 0, 1, 2 oder 3 ist.

2. Verbindung, Salz oder Solvat gemäß Anspruch 1, wobei R² ein -(5-15-gliedriges) Heterocycloalkyl ist.

3. Verbindung gemäß Anspruch 1 oder ein pharmazeutisch verträgliches Salz oder Solvat davon, wobei R² ein mit R⁷ substituiertes -(5-15-gliedriges) Heterocycloalkyl ist, wobei R⁷ -C(=O)R⁸, -C(=O)OR⁸ oder -S(O)ₙR⁸ ist und R⁸ -(C_{Null}-C₆-Alkylen) -C₆-C₁₅-aryl ist.

4. Verbindung gemäß einem der Ansprüche 1 bis 3 oder ein pharmazeutisch verträgliches Salz oder Solvat davon, nämlich eine Verbindung der Formel I ist.

5. Verbindung gemäß einem der Ansprüche 1 bis 3 oder ein pharmazeutisch verträgliches Salz oder Solvat davon, nämlich eine Verbindung der Formel II ist.

6. Pharmazeutische Zusammensetzung, umfassend eine Menge einer Verbindung gemäß einem der Ansprüche 1 bis 5 oder eines pharmazeutisch verträglichen Salzes oder Solvats davon und einen pharmazeutisch verträglichen Träger, ein pharmazeutisch verträgliches Vehikel oder Verdünnungsmittel.

7. Verbindung, wie sie in einem der Ansprüche 1 bis 5 definiert ist, oder ein pharmazeutisch verträgliches Salz oder Solvat davon zur Verwendung als Medikament.

8. Verbindung, wie sie in einem der Ansprüche 1 bis 5 definiert ist, oder ein pharmazeutisch verträgliches Salz oder Solvat davon zur Behandlung einer Störung, ausgewählt aus: Alzheimer'scher Krankheit, Krebs, Diabetes, Syndrom X, Adipositas, Haarausfall, Entzündung, Gemütsstörungen, neuronalem Zelltod, Schlaganfall, bipolarer Störung, Zuständen, die aus einem Verlust der Muskelmasse und -funktion entstehen, Gebrechlichkeit und Kardioschutz.

## Revendications

1. Composé de Formule I ou de Formule II, 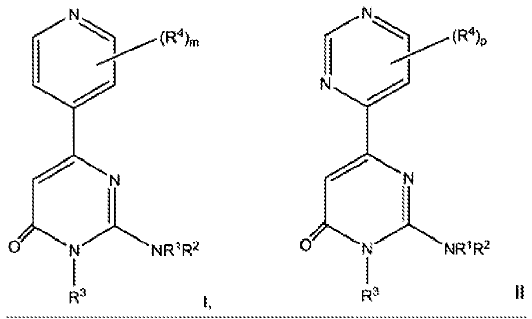 ou sel ou solvate pharmaceutiquement acceptable correspondant, dans lequel :
R¹ représente l'hydrogène ou un groupe alkyle en C₁-C₆ ;
R² est un groupe -(hétérocycloalkyle ayant de 4 à 15 éléments) facultativement substitué par un ou plusieurs substituants sélectionnés parmi les définitions du groupe R⁷ ;
R³ représente l'hydrogène ou un groupe alkyle en C₁-C₆ ;
R⁴ représente halogéno, alkyle en C₁-C₆, halogénoalkyle en C₁-C₆, alcoxy en C₁-C₆ ou halogénoalcoxy en C₁-C₆ ;
chaque R⁷ étant indépendamment sélectionné parmi -OH, halogéno, -(alkyle en C₁-C₆), -(alkényle en C₂-C₆), - (alkynyle en C₂-C₆), - (alcoxy en C₁-C₆), -(alkénoxy en C₂-C₆) , - (alkynoxy en C₂-C₆) , - (hydroxyalkyle en C₁-C₆), -CN, -NO₂, -NR⁸R⁹, -C (=O) N⁸R⁹, -C(=O)R⁸, -C(=O)OR⁸, -S (O) ₂NR⁸R⁹, -S(O)ₙR⁸, -NR⁹C (=O) R⁸, -NR⁹SO₂R⁸, - (alkylène en C_{zéro}-C₆) - (aryle en C₆-C₁₅), - (alkylène en C_{zéro}-C₆) - (hétérocycloalkyle ayant de 5 à 15 éléments), -(alkylène en C_{zéro}-C₆)-(hétéroaryle ayant de 5 à 15 éléments), -(alkylène en C_{zéro}-C₆)-(aryloxy en C₆-C₁₅) et - (alkylène en C_{zéro}-C₆) - (hétéroaryloxy ayant de 5 à 15 éléments), lesdits groupes alkyle, alkényle, alkynyle, alcoxy, alkénoxy, alkynoxy, hydroxyalkyle, aryle, aryloxy, hétéroaryle et hétéroaryloxy définissant R⁷ étant chacun facultativement et indépendamment substitués par un ou plusieurs substituants sélectionnés parmi des groupes halogéno, - (alkyle en C₁-C₁₂), - (alcoxy en C₁-C₄), -NR⁸R⁹, -C(=O) N⁸R⁹, -C(=O) R⁸, -C(=O)OR⁸, -NR⁹C(=O) R⁸, -NR⁹SO₂R⁸, -S(O)₂NR⁸R⁹, -S(O)ₙR⁸ ou -OH ;
chaque R⁸ et R⁹ étant indépendamment sélectionné parmi -H, - (alkyle en C₁-C₁₅), -(alkényle en C₂-C₁₅), - (alkynyle en C₂-C₁₅) , -(alkylène en C_{zéro}-C₄)-(cycloalkyle en C₃-C₁₅), - (alkylène en C_{zéro}-C₄)-(cycloalkényle en C₄-C₈), -(alkylène en C_{zéro}-C₄)-(hétérocycloalkyle ayant de 5 à 15 éléments), - (alkylène en C_{zéro}-C₄)-(aryle en C₆-C₁₅) et - (alkylène en C_{zéro}-C₄)-(hétéroaryle ayant de 5 à 15 éléments), lesdits groupes alkyle, alkényle, alkynyle, cycloalkyle, cycloalkényle, hétérocycloalkyle, aryle et hétéroaryle définissant R⁸ et R⁹ étant chacun facultativement et indépendamment substitués par un ou plusieurs substituants indépendamment sélectionnés parmi -OH, -(alkyle en C₁-C₁₂). - (alkényle en C₂-C₁₂), -(alkynyle en C₂-C₁₂), alcoxy en C₁-C₆, - (alkénoxy en C₂-C₆), -(alkynoxy en C₂-C₆), -(hydroxyalkyle en C₁-C₆), halogéno, -CN, -NO₂, -CF₃, -NH₂, -NH(alkyle en C₁-C₆), -N(alkyle en C₁-C₆)₂, -C(=O)NH₂, -C(=O)NH(alkyle en C₁-C₆), -C(=O)N(alkyle en C₁-C₆)₂, -SO₂NH₂, -SO₂NH(alkyle en C₁-C₆), -SO₂N(alkyle en C₁-C₆)₂, -C(=O)H, -C(=O)OH et -C(=O)O(alkyle en C₁-C₆) ;
n représente 0, 1 ou 2 ; m représente 0, 1, 2, 3 ou 4, et p représente 0, 1, 2 ou 3.

2. Composé selon la revendication 1 ou sel ou solvate pharmaceutiquement acceptable correspondant, dans lequel R² représente un groupe -(hétérocycloalkyle ayant de 5 à 15 éléments).

3. Composé selon la revendication 1 ou sel ou solvate pharmaceutiquement acceptable correspondant, dans lequel R² est un groupe -(hétérocycloalkyle ayant de 5 à 15 éléments) substitué par R⁷ ; R⁷ représentant -C(=O)R⁸, -C(=O)OR⁸ ou -S(O)ₙR⁸ et R⁸ représentant - (alkylène en C_{zéro}-C₆) - (aryle en C₆-C₁₅).

4. Composé selon l'une quelconque des revendications 1 à 3, ou sel ou solvate pharmaceutiquement acceptable correspondant, qui est un composé de Formule I.

5. Composé selon l'une quelconque des revendications 1 à 3, ou sel ou solvate pharmaceutiquement acceptable correspondant, qui est un composé de Formule II.

6. Composition pharmaceutique comprenant une quantité d'un composé selon l'une quelconque des revendications 1 à 5, ou d'un sel ou solvate pharmaceutiquement acceptable correspondant, et un support, véhicule ou diluant pharmaceutiquement acceptable.

7. Composé tel que défini dans l'une quelconque des revendications 1 à 5, ou sel ou solvate pharmaceutiquement acceptable correspondant, destiné à une utilisation en tant que médicament.

8. Composé tel que défini dans l'une quelconque des revendications 1 à 5, ou sel ou solvate pharmaceutiquement acceptable correspondant, permettant de traiter un trouble sélectionné parmi : la maladie d'Alzheimer, le cancer, le diabète, le syndrome X, l'obésité, la perte des cheveux, l'inflammation, les troubles de l'humeur, la mort des cellules neuronales, l'accident vasculaire cérébral, le trouble bipolaire, les états résultant d'une perte de la masse et de la fonction musculaires, la fragilité et la cardioprotection.
